(19) Europäisches Patentamt
European Patent Office
Office européen des brevets

(11) EP 2 578 678 B1

(12) EUROPEAN PATENT SPECIFICATION

(45) Date of publication and mention of the grant of the patent:
**28.10.2015 Bulletin 2015/44**

(21) Application number: **11789954.2**

(22) Date of filing: **31.05.2011**

(51) Int Cl.:
*C12N 5/10* (2006.01) *A01K 67/027* (2006.01)
*A61P 35/00* (2006.01) *C12Q 1/02* (2006.01)
*G01N 33/15* (2006.01) *G01N 33/50* (2006.01)
*C07K 14/435* (2006.01) *C07K 14/82* (2006.01)
*C12N 15/09* (2006.01)

(86) International application number:
**PCT/JP2011/062876**

(87) International publication number:
**WO 2011/152554 (08.12.2011 Gazette 2011/49)**

(54) **METHOD FOR PRODUCING TUMOR CELL**

VERFAHREN ZUR HERSTELLUNG VON TUMORZELLEN

PROCÉDÉ DE PRODUCTION DE CELLULES TUMORALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.05.2010 JP 2010125256**

(43) Date of publication of application:
**10.04.2013 Bulletin 2013/15**

(73) Proprietor: **Eisai R&D Management Co., Ltd.**
**Tokyo 112-8088 (JP)**

(72) Inventors:
- **AKAGI, Tsuyoshi**
  **Kobe-shi**
  **Hyogo 650-0047 (JP)**
- **SASAI, Ken**
  **Kobe-shi**
  **Hyogo 650-0047 (JP)**

(74) Representative: **Woods, Geoffrey Corlett et al**
**J A Kemp**
**14 South Square**
**Gray's Inn**
**London WC1R 5JJ (GB)**

(56) References cited:
**WO-A1-2006/108123** **WO-A2-2006/063182**
**WO-A2-2009/023194**

- **JESSE S BOEHM ET AL: "Immortalized cells as experimental models to study cancer", CYTOTECHNOLOGY, KLUWER ACADEMIC PUBLISHERS, DO, vol. 45, no. 1-2, 1 June 2004 (2004-06-01) , pages 47-59, XP019236836, ISSN: 1573-0778, DOI: 10.1007/S10616-004-5125-1**
- **LUNDBERG A S ET AL.: 'Immortalization and transformation of primary human airway epithelial cells by gene transfer.' ONCOGENE vol. 21, 2002, pages 4577 - 4586, XP008164132**
- **SATO M ET AL.: 'Multiple oncogenic changes (K-RASV12, p53 knockdown, mutant EGFRs, p16 bypass, telomerase) are not sufficient to confer a full malignant phenotype on human bronchial epithelial cells.' CANCER RES. vol. 66, 2006, pages 2116 - 2128, XP008164134**
- **WEN G ET AL.: 'Neoplastic transformation of human small airway epithelial cells induced by arsenic.' MOL. MED. vol. 14, no. 1-2, 2008, pages 2 - 10, XP008164130**
- **OKUDELA K ET AL.: 'PIK3CA mutation and amplification in human lung cancer.' PATHOL. INT. vol. 57, 2007, pages 664 - 671, XP008164144**
- **PIAO C Q ET AL.: 'Immortalization of human small airway epithelial cells by ectopic expression of telomerase.' CARCINOGENESIS vol. 26, no. 4, 2005, pages 725 - 731, XP008164128**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- FRAPPART D S ET AL.: 'Immortalization strategies for epithelial cells in primary culture.' DRUG ABSORPTION STUDIES (BIOTECHNOLOGY: PHARMACEUTICAL ASPECTS, [Online] vol. VII, 2008, NEW YORK, pages 616 - 639, XP008164126 Retrieved from the Internet: <URL:http://dx.doi.org/10.1007/978-0-387-74901-3>
- SASAI K ET AL.: 'Oncogene-mediated human lung epithelial cell transformation produces adenocarcinoma phenotypes in vivo.' CANCER RES. vol. 71, April 2011, pages 2541 - 2549, XP008164131
- BOS J L: "ras oncogenes in human cancer: a review.", CANCER RESEARCH 1 SEP 1989, vol. 49, no. 17, 1 September 1989 (1989-09-01), pages 4682-4689, ISSN: 0008-5472
- HJELLE B ET AL: "Oncogene v-src transforms and establishes embryonic rodent fibroblasts but not diploid human fibroblasts.", PROCEEDINGS OF THE NATIONAL ACADEMY OF SCIENCES OF THE UNITED STATES OF AMERICA JUN 1988, vol. 85, no. 12, June 1988 (1988-06), pages 4355-4359, ISSN: 0027-8424
- RANGARAJAN ANNAPOORNI ET AL: "Species- and cell type-specific requirements for cellular transformation.", CANCER CELL AUG 2004, vol. 6, no. 2, August 2004 (2004-08), pages 171-183, ISSN: 1535-6108

Sequence Listing

SEQUENCE LISTING

<110> EISAI R&D MANAGEMENT C0., LTD.

<120> Method of Producing Tumour Cell

<130> PCT11-0025

<150> JP 2010-125256
<151> 2010-05-31

<160> 26

<170> PatentIn version 3.4

<210> 1
<211> 1581
<212> DNA
<213> Rous sarcoma virus

<220>
<221> CDS
<222> (1)..(1581)

<400> 1

atg ggg agt agc aag agc aag cct aag gac ccc agc cag cgc cgg cgc 48
Met Gly Ser Ser Lys Ser Lys Pro Lys Asp Pro Ser Gln Arg Arg Arg
1 5 10 15

agc ctg gag cca ccc gac agc acc cac cac ggg gga ttc cca gcc tcg 96
Ser Leu Glu Pro Pro Asp Ser Thr His His Gly Gly Phe Pro Ala Ser
20 25 30

cag acc ccc aac aag aca gca gcc ccc gac acg cac cgc acc ccc agc 144
Gln Thr Pro Asn Lys Thr Ala Ala Pro Asp Thr His Arg Thr Pro Ser
35 40 45

cgc tcc ttc ggg acc gtg gcc acc gag ccc aag ctc ttc ggg ggc ttc 192
Arg Ser Phe Gly Thr Val Ala Thr Glu Pro Lys Leu Phe Gly Gly Phe
50 55 60

aac act tct gac acc gtt acg tcg ccg cag cgt gcc ggg gca ctg gct 240
Asn Thr Ser Asp Thr Val Thr Ser Pro Gln Arg Ala Gly Ala Leu Ala
65 70 75 80

ggc ggc gtc acc act ttc gtg gct ctc tac gac tac gag tcc tgg att 288
Gly Gly Val Thr Thr Phe Val Ala Leu Tyr Asp Tyr Glu Ser Trp Ile
85 90 95

gaa acg gac ttg tcc ttc aag aaa gga gaa cgg ctg cag att gtc aac 336
Glu Thr Asp Leu Ser Phe Lys Lys Gly Glu Arg Leu Gln Ile Val Asn
100 105 110

aac acg gaa ggt aac tgg tgg ctg gct cat tcc ctc act aca gga cag 384
Asn Thr Glu Gly Asn Trp Trp Leu Ala His Ser Leu Thr Thr Gly Gln
115 120 125

acg ggc tac atc ccc agt aac tat gtc gcg ccc tca gac tcc atc cag 432
Thr Gly Tyr Ile Pro Ser Asn Tyr Val Ala Pro Ser Asp Ser Ile Gln
130 135 140

gct gaa gag tgg tac ttt ggg aag atc act cgt cgg gag tcc gag cgg 480
Ala Glu Glu Trp Tyr Phe Gly Lys Ile Thr Arg Arg Glu Ser Glu Arg
145 150 155 160

ctg ctg ctc aac ccc gaa aac ccc cgg gga acc ttc ttg gtc cgg gag 528
Leu Leu Leu Asn Pro Glu Asn Pro Arg Gly Thr Phe Leu Val Arg Glu
165 170 175

agc gag acg aca aaa ggt gcc tat tgc ctc tcc gtt tct gac ttt gac 576
Ser Glu Thr Thr Lys Gly Ala Tyr Cys Leu Ser Val Ser Asp Phe Asp
180 185 190

aac gcc aag ggc ctc aat gtg aag cac tac aag atc cgc aag ctg gac 624
Asn Ala Lys Gly Leu Asn Val Lys His Tyr Lys Ile Arg Lys Leu Asp

195 200 205

agc ggc ggc ttc tac atc acc tca cgc aca cag ttc agc agc ctg cag 672
Ser Gly Gly Phe Tyr Ile Thr Ser Arg Thr Gln Phe Ser Ser Leu Gln
210 215 220

cag ctg gtg gcc tac tac tcc aaa cat gct gat ggc ttg tgc cac cgc 720
Gln Leu Val Ala Tyr Tyr Ser Lys His Ala Asp Gly Leu Cys His Arg
225 230 235 240

ctg acc aac gtc tgc ccc acg tcc aag ccc cag acc cag gga ctc gcc 768
Leu Thr Asn Val Cys Pro Thr Ser Lys Pro Gln Thr Gln Gly Leu Ala
245 250 255

aag gac gcg tgg gaa atc ccc cgg gag tcg ctg cgg ctg gag gtg aag 816
Lys Asp Ala Trp Glu Ile Pro Arg Glu Ser Leu Arg Leu Glu Val Lys
260 265 270

ctg ggg cag ggc tgc ttt gga gag gtc tgg atg ggg acc tgg aac ggc 864
Leu Gly Gln Gly Cys Phe Gly Glu Val Trp Met Gly Thr Trp Asn Gly
275 280 285

acc acc aga gtg gcc ata aag act ctg aag ccc ggc acc atg tcc ccg 912
Thr Thr Arg Val Ala Ile Lys Thr Leu Lys Pro Gly Thr Met Ser Pro
290 295 300

gag gcc ttc ctg cag gaa gcc caa gtg atg aag aag ctc cgg cat gag 960
Glu Ala Phe Leu Gln Glu Ala Gln Val Met Lys Lys Leu Arg His Glu
305 310 315 320

aag ctg gtt caa ctg tac gca gtg gtg tcg gaa gag ccc atc tac atc 1008
Lys Leu Val Gln Leu Tyr Ala Val Val Ser Glu Glu Pro Ile Tyr Ile
325 330 335

gtc att gag tac atg agc aag ggg agc ctc ctg gat ttc ctg aag gga 1056
Val Ile Glu Tyr Met Ser Lys Gly Ser Leu Leu Asp Phe Leu Lys Gly
340 345 350

gag atg ggc aag tac ctg cgg ctg cca cag ctc gtt gat atg gct gct 1104
Glu Met Gly Lys Tyr Leu Arg Leu Pro Gln Leu Val Asp Met Ala Ala
355 360 365

cag att gca tcc ggc atg gcc tat gtg gag agg atg aac tac gtg cac 1152
Gln Ile Ala Ser Gly Met Ala Tyr Val Glu Arg Met Asn Tyr Val His
370 375 380

cga gac ctg cgg gcg gcc aac atc ctg gtg ggg gag aac ctg gtg tgc 1200
Arg Asp Leu Arg Ala Ala Asn Ile Leu Val Gly Glu Asn Leu Val Cys
385 390 395 400

aag gtg gct gac ttt ggg ctg gca cgc ctc atc gag gac aac gag tac 1248
Lys Val Ala Asp Phe Gly Leu Ala Arg Leu Ile Glu Asp Asn Glu Tyr
405 410 415

aca gca cgg caa ggt gcc aag ttc ccc atc aag tgg aca gcc ccc gag 1296
Thr Ala Arg Gln Gly Ala Lys Phe Pro Ile Lys Trp Thr Ala Pro Glu
420 425 430

gca gcc ctc tat ggc cgg ttc acc atc aag tcg gat gtc tgg tcc ttc 1344
Ala Ala Leu Tyr Gly Arg Phe Thr Ile Lys Ser Asp Val Trp Ser Phe
435 440 445

ggc atc ctg ctg act gag ctg acc acc aag ggc cgg gtg cca tac cca 1392
Gly Ile Leu Leu Thr Glu Leu Thr Thr Lys Gly Arg Val Pro Tyr Pro
450 455 460

ggg atg ggc aac ggg gag gtg ctg gac cgg gtg gag agg ggc tac cgc 1440
Gly Met Gly Asn Gly Glu Val Leu Asp Arg Val Glu Arg Gly Tyr Arg
465 470 475 480

atg ccc tgc ccg ccc gag tgc ccc gag tcg ctg cat gac ctt atg tgc 1488
Met Pro Cys Pro Pro Glu Cys Pro Glu Ser Leu His Asp Leu Met Cys
485 490 495

cag tgc tgg cgg agg gac cct gag gag cgg ccc act ttc gag tac ctg 1536
Gln Cys Trp Arg Arg Asp Pro Glu Glu Arg Pro Thr Phe Glu Tyr Leu

500 505 510

cag gcc cag ctg ctc cct gct tgt gtg ttg gag gtc gct gag tag 1581
Gln Ala Gln Leu Leu Pro Ala Cys Val Leu Glu Val Ala Glu
515 520 525

<210> 2
<211> 526
<212> PRT
<213> Rous sarcoma virus

<400> 2

Met Gly Ser Ser Lys Ser Lys Pro Lys Asp Pro Ser Gln Arg Arg Arg
1 5 10 15

Ser Leu Glu Pro Pro Asp Ser Thr His His Gly Gly Phe Pro Ala Ser
20 25 30

Gln Thr Pro Asn Lys Thr Ala Ala Pro Asp Thr His Arg Thr Pro Ser
35 40 45

Arg Ser Phe Gly Thr Val Ala Thr Glu Pro Lys Leu Phe Gly Gly Phe
50 55 60

Asn Thr Ser Asp Thr Val Thr Ser Pro Gln Arg Ala Gly Ala Leu Ala
65 70 75 80

Gly Gly Val Thr Thr Phe Val Ala Leu Tyr Asp Tyr Glu Ser Trp Ile
85 90 95

Glu Thr Asp Leu Ser Phe Lys Lys Gly Glu Arg Leu Gln Ile Val Asn
100 105 110

Asn Thr Glu Gly Asn Trp Trp Leu Ala His Ser Leu Thr Thr Gly Gln
115 120 125

Thr Gly Tyr Ile Pro Ser Asn Tyr Val Ala Pro Ser Asp Ser Ile Gln
130 135 140

Ala Glu Glu Trp Tyr Phe Gly Lys Ile Thr Arg Arg Glu Ser Glu Arg
145 150 155 160

Leu Leu Leu Asn Pro Glu Asn Pro Arg Gly Thr Phe Leu Val Arg Glu
165 170 175

Ser Glu Thr Thr Lys Gly Ala Tyr Cys Leu Ser Val Ser Asp Phe Asp
180 185 190

Asn Ala Lys Gly Leu Asn Val Lys His Tyr Lys Ile Arg Lys Leu Asp
195 200 205

Ser Gly Gly Phe Tyr Ile Thr Ser Arg Thr Gln Phe Ser Ser Leu Gln
210 215 220

Gln Leu Val Ala Tyr Tyr Ser Lys His Ala Asp Gly Leu Cys His Arg
225 230 235 240

Leu Thr Asn Val Cys Pro Thr Ser Lys Pro Gln Thr Gln Gly Leu Ala
245 250 255

Lys Asp Ala Trp Glu Ile Pro Arg Glu Ser Leu Arg Leu Glu Val Lys
260 265 270

Leu Gly Gln Gly Cys Phe Gly Glu Val Trp Met Gly Thr Trp Asn Gly
275 280 285

Thr Thr Arg Val Ala Ile Lys Thr Leu Lys Pro Gly Thr Met Ser Pro
290 295 300

Glu Ala Phe Leu Gln Glu Ala Gln Val Met Lys Lys Leu Arg His Glu
305 310 315 320

Lys Leu Val Gln Leu Tyr Ala Val Val Ser Glu Glu Pro Ile Tyr Ile
325 330 335

Val Ile Glu Tyr Met Ser Lys Gly Ser Leu Leu Asp Phe Leu Lys Gly
340 345 350

Glu Met Gly Lys Tyr Leu Arg Leu Pro Gln Leu Val Asp Met Ala Ala
355 360 365

Gln Ile Ala Ser Gly Met Ala Tyr Val Glu Arg Met Asn Tyr Val His
370 375 380

Arg Asp Leu Arg Ala Ala Asn Ile Leu Val Gly Glu Asn Leu Val Cys
385 390 395 400

Lys Val Ala Asp Phe Gly Leu Ala Arg Leu Ile Glu Asp Asn Glu Tyr
405 410 415

Thr Ala Arg Gln Gly Ala Lys Phe Pro Ile Lys Trp Thr Ala Pro Glu
420 425 430

Ala Ala Leu Tyr Gly Arg Phe Thr Ile Lys Ser Asp Val Trp Ser Phe
435 440 445

Gly Ile Leu Leu Thr Glu Leu Thr Thr Lys Gly Arg Val Pro Tyr Pro
450 455 460

Gly Met Gly Asn Gly Glu Val Leu Asp Arg Val Glu Arg Gly Tyr Arg
465 470 475 480

Met Pro Cys Pro Pro Glu Cys Pro Glu Ser Leu His Asp Leu Met Cys
485 490 495

Gln Cys Trp Arg Arg Asp Pro Glu Glu Arg Pro Thr Phe Glu Tyr Leu
500 505 510

Gln Ala Gln Leu Leu Pro Ala Cys Val Leu Glu Val Ala Glu
515 520 525

<210> 3
<211> 1320
<212> DNA
<213> Homo sapiens

<220>
<221> CDS
<222> (1)..(1320)

<400> 3

```
atg ccc ctc aac gtt agc ttc acc aac agg aac tat gac ctc gac tac      48
Met Pro Leu Asn Val Ser Phe Thr Asn Arg Asn Tyr Asp Leu Asp Tyr
1               5                   10                  15

gac tcg gtg cag ccg tat ttc tac tgc gac gag gag gag aac ttc tac      96
Asp Ser Val Gln Pro Tyr Phe Tyr Cys Asp Glu Glu Glu Asn Phe Tyr
            20                  25                  30

cag cag cag cag cag agc gag ctg cag ccc ccg gcg ccc agc gag gat     144
Gln Gln Gln Gln Gln Ser Glu Leu Gln Pro Pro Ala Pro Ser Glu Asp
        35                  40                  45

atc tgg aag aaa ttc gag ctg ctg ccc acc ccg ccc ctg tcc cct agc     192
Ile Trp Lys Lys Phe Glu Leu Leu Pro Thr Pro Pro Leu Ser Pro Ser
    50                  55                  60

cgc cgc tcc ggg ctc tgc tcg ccc tcc tac gtt gcg gtc aca ccc ttc     240
Arg Arg Ser Gly Leu Cys Ser Pro Ser Tyr Val Ala Val Thr Pro Phe
65                  70                  75                  80

tcc ctt cgg gga gac aac gac ggc ggt ggc ggg agc ttc tcc acg gcc     288
Ser Leu Arg Gly Asp Asn Asp Gly Gly Gly Gly Ser Phe Ser Thr Ala
                85                  90                  95

gac cag ctg gag atg gtg acc gag ctg ctg gga gga gac atg gtg aac     336
Asp Gln Leu Glu Met Val Thr Glu Leu Leu Gly Gly Asp Met Val Asn
            100                 105                 110

cag agt ttc atc tgc gac ccg gac gac gag acc ttc atc aaa aac atc     384
Gln Ser Phe Ile Cys Asp Pro Asp Asp Glu Thr Phe Ile Lys Asn Ile
        115                 120                 125

atc atc cag gac tgt atg tgg agc ggc ttc tcg gcc gcc gcc aag ctc     432
```

Ile Ile Gln Asp Cys Met Trp Ser Gly Phe Ser Ala Ala Ala Lys Leu
130 135 140

gtc tca gag aag ctg gcc tcc tac cag gct gcg cgc aaa gac agc ggc 480
Val Ser Glu Lys Leu Ala Ser Tyr Gln Ala Ala Arg Lys Asp Ser Gly
145 150 155 160

agc ccg aac ccc gcc cgc ggc cac agc gtc tgc tcc acc tcc agc ttg 528
Ser Pro Asn Pro Ala Arg Gly His Ser Val Cys Ser Thr Ser Ser Leu
165 170 175

tac ctg cag gat ctg agc gcc gcc gcc tca gag tgc atc gac ccc tcg 576
Tyr Leu Gln Asp Leu Ser Ala Ala Ala Ser Glu Cys Ile Asp Pro Ser
180 185 190

gtg gtc ttc ccc tac cct ctc aac gac agc agc tcg ccc aag tcc tgc 624
Val Val Phe Pro Tyr Pro Leu Asn Asp Ser Ser Ser Pro Lys Ser Cys
195 200 205

gcc tcg caa gac tcc agc gcc ttc tct ccg tcc tcg gat tct ctg ctc 672
Ala Ser Gln Asp Ser Ser Ala Phe Ser Pro Ser Ser Asp Ser Leu Leu
210 215 220

tcc tcg acg gag tcc tcc ccg cag ggc agc ccc gag ccc ctg gtg ctc 720
Ser Ser Thr Glu Ser Ser Pro Gln Gly Ser Pro Glu Pro Leu Val Leu
225 230 235 240

cat gag gag aca ccg ccc acc acc agc agc gac tct gag gag gaa caa 768
His Glu Glu Thr Pro Pro Thr Thr Ser Ser Asp Ser Glu Glu Glu Gln
245 250 255

gaa gat gag gaa gaa atc gat gtt gtt tct gtg gaa aag agg cag gct 816
Glu Asp Glu Glu Glu Ile Asp Val Val Ser Val Glu Lys Arg Gln Ala
260 265 270

cct ggc aaa agg tca gag tct gga tca cct tct gct gga ggc cac agc 864
Pro Gly Lys Arg Ser Glu Ser Gly Ser Pro Ser Ala Gly Gly His Ser
275 280 285

aaa cct cct cac agc cca ctg gtc ctc aag agg tgc cac gtc tcc aca 912
Lys Pro Pro His Ser Pro Leu Val Leu Lys Arg Cys His Val Ser Thr
290 295 300

cat cag cac aac tac gca gcg cct ccc tcc act cgg aag gac tat cct 960
His Gln His Asn Tyr Ala Ala Pro Pro Ser Thr Arg Lys Asp Tyr Pro
305 310 315 320

gct gcc aag agg gtc aag ttg gac agt gtc aga gtc ctg aga cag atc 1008
Ala Ala Lys Arg Val Lys Leu Asp Ser Val Arg Val Leu Arg Gln Ile
325 330 335

agc aac aac cga aaa tgc acc agc ccc agg tcc tcg gac acc gag gag 1056
Ser Asn Asn Arg Lys Cys Thr Ser Pro Arg Ser Ser Asp Thr Glu Glu
340 345 350

aat gtc aag agg cga aca cac aac gtc ttg gag cgc cag agg agg aac 1104
Asn Val Lys Arg Arg Thr His Asn Val Leu Glu Arg Gln Arg Arg Asn
355 360 365

gag cta aaa cgg agc ttt ttt gcc ctg cgt gac cag atc ccg gag ttg 1152
Glu Leu Lys Arg Ser Phe Phe Ala Leu Arg Asp Gln Ile Pro Glu Leu
370 375 380

gaa aac aat gaa aag gcc ccc aag gta gtt atc ctt aaa aaa gcc aca 1200
Glu Asn Asn Glu Lys Ala Pro Lys Val Val Ile Leu Lys Lys Ala Thr
385 390 395 400

gca tac atc ctg tcc gtc caa gca gag gag caa aag ctc att tct gaa 1248
Ala Tyr Ile Leu Ser Val Gln Ala Glu Glu Gln Lys Leu Ile Ser Glu
405 410 415

gag gac ttg ttg cgg aaa cga cga gaa cag ttg aaa cac aaa ctt gaa 1296
Glu Asp Leu Leu Arg Lys Arg Arg Glu Gln Leu Lys His Lys Leu Glu
420 425 430

cag cta cgg aac tct tgt gcg taa 1320

Gln Leu Arg Asn Ser Cys Ala
435

<210> 4
<211> 439
<212> PRT
<213> Homo sapiens

<400> 4

Met Pro Leu Asn Val Ser Phe Thr Asn Arg Asn Tyr Asp Leu Asp Tyr
1 5 10 15

Asp Ser Val Gln Pro Tyr Phe Tyr Cys Asp Glu Glu Glu Asn Phe Tyr
20 25 30

Gln Gln Gln Gln Gln Ser Glu Leu Gln Pro Pro Ala Pro Ser Glu Asp
35 40 45

Ile Trp Lys Lys Phe Glu Leu Leu Pro Thr Pro Pro Leu Ser Pro Ser
50 55 60

Arg Arg Ser Gly Leu Cys Ser Pro Ser Tyr Val Ala Val Thr Pro Phe
65 70 75 80

Ser Leu Arg Gly Asp Asn Asp Gly Gly Gly Gly Ser Phe Ser Thr Ala
85 90 95

Asp Gln Leu Glu Met Val Thr Glu Leu Leu Gly Gly Asp Met Val Asn
100 105 110

Gln Ser Phe Ile Cys Asp Pro Asp Asp Glu Thr Phe Ile Lys Asn Ile
115 120 125

Ile Ile Gln Asp Cys Met Trp Ser Gly Phe Ser Ala Ala Ala Lys Leu
130 135 140

Val Ser Glu Lys Leu Ala Ser Tyr Gln Ala Ala Arg Lys Asp Ser Gly
145 150 155 160

Ser Pro Asn Pro Ala Arg Gly His Ser Val Cys Ser Thr Ser Ser Leu
165 170 175

Tyr Leu Gln Asp Leu Ser Ala Ala Ala Ser Glu Cys Ile Asp Pro Ser
180 185 190

Val Val Phe Pro Tyr Pro Leu Asn Asp Ser Ser Ser Pro Lys Ser Cys
195 200 205

Ala Ser Gln Asp Ser Ser Ala Phe Ser Pro Ser Ser Asp Ser Leu Leu
210 215 220

Ser Ser Thr Glu Ser Ser Pro Gln Gly Ser Pro Glu Pro Leu Val Leu
225 230 235 240

His Glu Glu Thr Pro Pro Thr Thr Ser Ser Asp Ser Glu Glu Glu Gln
245 250 255

Glu Asp Glu Glu Glu Ile Asp Val Val Ser Val Glu Lys Arg Gln Ala

260 265 270

Pro Gly Lys Arg Ser Glu Ser Gly Ser Pro Ser Ala Gly Gly His Ser
275 280 285

Lys Pro Pro His Ser Pro Leu Val Leu Lys Arg Cys His Val Ser Thr
290 295 300

His Gln His Asn Tyr Ala Ala Pro Pro Ser Thr Arg Lys Asp Tyr Pro
305 310 315 320

Ala Ala Lys Arg Val Lys Leu Asp Ser Val Arg Val Leu Arg Gln Ile
325 330 335

Ser Asn Asn Arg Lys Cys Thr Ser Pro Arg Ser Ser Asp Thr Glu Glu
340 345 350

Asn Val Lys Arg Arg Thr His Asn Val Leu Glu Arg Gln Arg Arg Asn
355 360 365

Glu Leu Lys Arg Ser Phe Phe Ala Leu Arg Asp Gln Ile Pro Glu Leu
370 375 380

Glu Asn Asn Glu Lys Ala Pro Lys Val Val Ile Leu Lys Lys Ala Thr
385 390 395 400

Ala Tyr Ile Leu Ser Val Gln Ala Glu Glu Gln Lys Leu Ile Ser Glu
405 410 415

Glu Asp Leu Leu Arg Lys Arg Arg Glu Gln Leu Lys His Lys Leu Glu
420 425 430

Gln Leu Arg Asn Ser Cys Ala
435

<210> 5
<211> 567
<212> DNA
<213> Homo sapiens

<220>
<221> CDS
<222> (1)..(567)

<400> 5

atg act gaa tat aaa ctt gtg gta gtt gga gct gtt ggc gta ggc aag 48
Met Thr Glu Tyr Lys Leu Val Val Val Gly Ala Val Gly Val Gly Lys
1 5 10 15

agt gcc ttg acg ata cag cta att cag aat cat ttt gtg gac gaa tat 96
Ser Ala Leu Thr Ile Gln Leu Ile Gln Asn His Phe Val Asp Glu Tyr
20 25 30

gat cca aca ata gag gat tcc tac agg aag caa gta gta att gat gga 144
Asp Pro Thr Ile Glu Asp Ser Tyr Arg Lys Gln Val Val Ile Asp Gly
35 40 45

gaa acc tgt ctc ttg gat att ctc gac aca gca ggt caa gag gag tac 192
Glu Thr Cys Leu Leu Asp Ile Leu Asp Thr Ala Gly Gln Glu Glu Tyr
50 55 60

agt gca atg agg gac cag tac atg agg act ggg gag ggc ttt ctt tgt 240
Ser Ala Met Arg Asp Gln Tyr Met Arg Thr Gly Glu Gly Phe Leu Cys

65 70 75 80

gta ttt gcc ata aat aat act aaa tca ttt gaa gat att cac cat tat 288
Val Phe Ala Ile Asn Asn Thr Lys Ser Phe Glu Asp Ile His His Tyr
85 90 95

aga gaa caa att aaa aga gtt aag gac tct gaa gat gta cct atg gtc 336
Arg Glu Gln Ile Lys Arg Val Lys Asp Ser Glu Asp Val Pro Met Val
100 105 110

cta gta gga aat aaa tgt gat ttg cct tct aga aca gta gac aca aaa 384
Leu Val Gly Asn Lys Cys Asp Leu Pro Ser Arg Thr Val Asp Thr Lys
115 120 125

cag gct cag gac tta gca aga agt tat gga att cct ttt att gaa aca 432
Gln Ala Gln Asp Leu Ala Arg Ser Tyr Gly Ile Pro Phe Ile Glu Thr
130 135 140

tca gca aag aca aga cag ggt gtt gat gat gcc ttc tat aca tta gtt 480
Ser Ala Lys Thr Arg Gln Gly Val Asp Asp Ala Phe Tyr Thr Leu Val
145 150 155 160

cga gaa att cga aaa cat aaa gaa aag atg agc aaa gat ggt aaa aag 528
Arg Glu Ile Arg Lys His Lys Glu Lys Met Ser Lys Asp Gly Lys Lys
165 170 175

aag aaa aag aag tca aag aca aag tgt gta att atg taa 567
Lys Lys Lys Lys Ser Lys Thr Lys Cys Val Ile Met
180 185

<210> 6
<211> 188
<212> PRT
<213> Homo sapiens

<400> 6

Met Thr Glu Tyr Lys Leu Val Val Val Gly Ala Val Gly Val Gly Lys
1               5                   10                  15

Ser Ala Leu Thr Ile Gln Leu Ile Gln Asn His Phe Val Asp Glu Tyr
            20                  25                  30

Asp Pro Thr Ile Glu Asp Ser Tyr Arg Lys Gln Val Val Ile Asp Gly
        35                  40                  45

Glu Thr Cys Leu Leu Asp Ile Leu Asp Thr Ala Gly Gln Glu Glu Tyr
    50                  55                  60

Ser Ala Met Arg Asp Gln Tyr Met Arg Thr Gly Glu Gly Phe Leu Cys
65                  70                  75                  80

Val Phe Ala Ile Asn Asn Thr Lys Ser Phe Glu Asp Ile His His Tyr
                85                  90                  95

Arg Glu Gln Ile Lys Arg Val Lys Asp Ser Glu Asp Val Pro Met Val
            100                 105                 110

Leu Val Gly Asn Lys Cys Asp Leu Pro Ser Arg Thr Val Asp Thr Lys
        115                 120                 125

Gln Ala Gln Asp Leu Ala Arg Ser Tyr Gly Ile Pro Phe Ile Glu Thr
    130                 135                 140

Ser Ala Lys Thr Arg Gln Gly Val Asp Asp Ala Phe Tyr Thr Leu Val
145                 150                 155                 160

Arg Glu Ile Arg Lys His Lys Glu Lys Met Ser Lys Asp Gly Lys Lys
165 170 175

Lys Lys Lys Lys Ser Lys Thr Lys Cys Val Ile Met
180 185

<210> 7
<211> 720
<212> DNA
<213> Homo sapiens

<220>
<221> CDS
<222> (1)..(720)

<400> 7

atg gcg cac gct ggg aga acg ggg tac gac aac cgg gag ata gtg atg 48
Met Ala His Ala Gly Arg Thr Gly Tyr Asp Asn Arg Glu Ile Val Met
1 5 10 15

aag tac atc cat tat aag ctg tcg cag agg ggc tac gag tgg gat gcg 96
Lys Tyr Ile His Tyr Lys Leu Ser Gln Arg Gly Tyr Glu Trp Asp Ala
20 25 30

gga gat gtg ggc gcc gcg ccc ccg ggg gcc gcc ccc gca ccg ggc atc 144
Gly Asp Val Gly Ala Ala Pro Pro Gly Ala Ala Pro Ala Pro Gly Ile
35 40 45

ttc tcc tcc cag ccc ggg cac acg ccc cat cca gcc gca tcc cgc gac 192
Phe Ser Ser Gln Pro Gly His Thr Pro His Pro Ala Ala Ser Arg Asp
50 55 60

ccg gtc gcc agg acc tcg ccg ctg cag acc ccg gct gcc ccc ggc gcc 240

Pro Val Ala Arg Thr Ser Pro Leu Gln Thr Pro Ala Ala Pro Gly Ala
65 70 75 80

gcc gcg ggg cct gcg ctc agc ccg gtg cca cct gtg gtc cac ctg gcc 288
Ala Ala Gly Pro Ala Leu Ser Pro Val Pro Pro Val Val His Leu Ala
85 90 95

ctc cgc caa gcc ggc gac gac ttc tcc cgc cgc tac cgc ggc gac ttc 336
Leu Arg Gln Ala Gly Asp Asp Phe Ser Arg Arg Tyr Arg Gly Asp Phe
100 105 110

gcc gag atg tcc agc cag ctg cac ctg acg ccc ttc acc gcg cgg gga 384
Ala Glu Met Ser Ser Gln Leu His Leu Thr Pro Phe Thr Ala Arg Gly
115 120 125

cgc ttt gcc acg gtg gtg gag gag ctc ttc agg gac ggg gtg aac tgg 432
Arg Phe Ala Thr Val Val Glu Glu Leu Phe Arg Asp Gly Val Asn Trp
130 135 140

ggg agg att gtg gcc ttc ttt gag ttc ggt ggg gtc atg tgt gtg gag 480
Gly Arg Ile Val Ala Phe Phe Glu Phe Gly Gly Val Met Cys Val Glu
145 150 155 160

agc gtc aac cgg gag atg tcg ccc ctg gtg gac aac atc gcc ctg tgg 528
Ser Val Asn Arg Glu Met Ser Pro Leu Val Asp Asn Ile Ala Leu Trp
165 170 175

atg act gag tac ctg aac cgg cac ctg cac acc tgg atc cag gat aac 576
Met Thr Glu Tyr Leu Asn Arg His Leu His Thr Trp Ile Gln Asp Asn
180 185 190

gga ggc tgg gat gcc ttt gtg gaa ctg tac ggc ccc agc atg cgg cct 624
Gly Gly Trp Asp Ala Phe Val Glu Leu Tyr Gly Pro Ser Met Arg Pro
195 200 205

ctg ttt gat ttc tcc tgg ctg tct ctg aag act ctg ctc agt ttg gcc 672
Leu Phe Asp Phe Ser Trp Leu Ser Leu Lys Thr Leu Leu Ser Leu Ala
210 215 220

ctg gtg gga gct tgc atc acc ctg ggt gcc tat ctg agc cac aag tga 720
Leu Val Gly Ala Cys Ile Thr Leu Gly Ala Tyr Leu Ser His Lys
225 230 235

<210> 8
<211> 239
<212> PRT
<213> Homo sapiens

<400> 8

Met Ala His Ala Gly Arg Thr Gly Tyr Asp Asn Arg Glu Ile Val Met
1 5 10 15

Lys Tyr Ile His Tyr Lys Leu Ser Gln Arg Gly Tyr Glu Trp Asp Ala
20 25 30

Gly Asp Val Gly Ala Ala Pro Pro Gly Ala Ala Pro Ala Pro Gly Ile
35 40 45

Phe Ser Ser Gln Pro Gly His Thr Pro His Pro Ala Ala Ser Arg Asp
50 55 60

Pro Val Ala Arg Thr Ser Pro Leu Gln Thr Pro Ala Ala Pro Gly Ala
65 70 75 80

Ala Ala Gly Pro Ala Leu Ser Pro Val Pro Pro Val Val His Leu Ala
85 90 95

Leu Arg Gln Ala Gly Asp Asp Phe Ser Arg Arg Tyr Arg Gly Asp Phe

100 105 110

Ala Glu Met Ser Ser Gln Leu His Leu Thr Pro Phe Thr Ala Arg Gly
115 120 125

Arg Phe Ala Thr Val Val Glu Glu Leu Phe Arg Asp Gly Val Asn Trp
130 135 140

Gly Arg Ile Val Ala Phe Phe Glu Phe Gly Gly Val Met Cys Val Glu
145 150 155 160

Ser Val Asn Arg Glu Met Ser Pro Leu Val Asp Asn Ile Ala Leu Trp
165 170 175

Met Thr Glu Tyr Leu Asn Arg His Leu His Thr Trp Ile Gln Asp Asn
180 185 190

Gly Gly Trp Asp Ala Phe Val Glu Leu Tyr Gly Pro Ser Met Arg Pro
195 200 205

Leu Phe Asp Phe Ser Trp Leu Ser Leu Lys Thr Leu Leu Ser Leu Ala
210 215 220

Leu Val Gly Ala Cys Ile Thr Leu Gly Ala Tyr Leu Ser His Lys
225 230 235

<210> 9
<211> 3207
<212> DNA
<213> Homo sapiens

<220>
<221> CDS

<222> (1).. (3207)

<400> 9

atg cct cca aga cca tca tca ggt gaa ctg tgg ggc atc cac ttg atg 48
Met Pro Pro Arg Pro Ser Ser Gly Glu Leu Trp Gly Ile His Leu Met
1 5 10 15

ccc cca aga atc cta gta gaa tgt tta cta cca aat ggg atg ata gtg 96
Pro Pro Arg Ile Leu Val Glu Cys Leu Leu Pro Asn Gly Met Ile Val
20 25 30

act tta gaa tgc ctc cgt gag gct acg tta ata acg ata aag cat gaa 144
Thr Leu Glu Cys Leu Arg Glu Ala Thr Leu Ile Thr Ile Lys His Glu
35 40 45

cta ttt aaa gaa gca aga aaa tac cct ctc cat caa ctt ctt caa gat 192
Leu Phe Lys Glu Ala Arg Lys Tyr Pro Leu His Gln Leu Leu Gln Asp
50 55 60

gaa tct tct tac att ttc gta agt gtt acc caa gaa gca gaa agg gaa 240
Glu Ser Ser Tyr Ile Phe Val Ser Val Thr Gln Glu Ala Glu Arg Glu
65 70 75 80

gaa ttt ttt gat gaa aca aga cga ctt tgt gac ctt cgg ctt ttt caa 288
Glu Phe Phe Asp Glu Thr Arg Arg Leu Cys Asp Leu Arg Leu Phe Gln
85 90 95

ccc ttt tta aaa gta att gaa cca gta ggc aac cgt gaa gaa aag atc 336
Pro Phe Leu Lys Val Ile Glu Pro Val Gly Asn Arg Glu Glu Lys Ile
100 105 110

ctc aat cga gaa att ggt ttt gct atc ggc atg cca gtg tgt gaa ttt 384
Leu Asn Arg Glu Ile Gly Phe Ala Ile Gly Met Pro Val Cys Glu Phe

115 120 125

gat atg gtt aaa gat cca gaa gta cag gac ttc cga aga aat att ctg 432
Asp Met Val Lys Asp Pro Glu Val Gln Asp Phe Arg Arg Asn Ile Leu
130 135 140

aac gtt tgt aaa gaa gct gtg gat ctt agg gac ctc aat tca cct cat 480
Asn Val Cys Lys Glu Ala Val Asp Leu Arg Asp Leu Asn Ser Pro His
145 150 155 160

agt aga gca atg tat gtc tat cct cca aat gta gaa tct tca cca gaa 528
Ser Arg Ala Met Tyr Val Tyr Pro Pro Asn Val Glu Ser Ser Pro Glu
165 170 175

ttg cca aag cac ata tat aat aaa tta gat aaa ggg caa ata ata gtg 576
Leu Pro Lys His Ile Tyr Asn Lys Leu Asp Lys Gly Gln Ile Ile Val
180 185 190

gtg atc tgg gta ata gtt tct cca aat aat gac aag cag aag tat act 624
Val Ile Trp Val Ile Val Ser Pro Asn Asn Asp Lys Gln Lys Tyr Thr
195 200 205

ctg aaa atc aac cat gac tgt gta cca gaa caa gta att gct gaa gca 672
Leu Lys Ile Asn His Asp Cys Val Pro Glu Gln Val Ile Ala Glu Ala
210 215 220

atc agg aaa aaa act cga agt atg ttg cta tcc tct gaa caa cta aaa 720
Ile Arg Lys Lys Thr Arg Ser Met Leu Leu Ser Ser Glu Gln Leu Lys
225 230 235 240

ctc tgt gtt tta gaa tat cag ggc aag tat att tta aaa gtg tgt gga 768
Leu Cys Val Leu Glu Tyr Gln Gly Lys Tyr Ile Leu Lys Val Cys Gly
245 250 255

tgt gat gaa tac ttc cta gaa aaa tat cct ctg agt cag tat aag tat 816
Cys Asp Glu Tyr Phe Leu Glu Lys Tyr Pro Leu Ser Gln Tyr Lys Tyr
260 265 270

ata aga agc tgt ata atg ctt ggg agg atg ccc aat ttg atg ttg atg 864
Ile Arg Ser Cys Ile Met Leu Gly Arg Met Pro Asn Leu Met Leu Met
275 280 285

gct aaa gaa agc ctt tat tct caa ctg cca atg gac tgt ttt aca atg 912
Ala Lys Glu Ser Leu Tyr Ser Gln Leu Pro Met Asp Cys Phe Thr Met
290 295 300

cca tct tat tcc aga cgc att tcc aca gct aca cca tat atg aat gga 960
Pro Ser Tyr Ser Arg Arg Ile Ser Thr Ala Thr Pro Tyr Met Asn Gly
305 310 315 320

gaa aca tct aca aaa tcc ctt tgg gtt ata aat agt gca ctc aga ata 1008
Glu Thr Ser Thr Lys Ser Leu Trp Val Ile Asn Ser Ala Leu Arg Ile
325 330 335

aaa att ctt tgt gca acc tac gtg aat gta aat att cga gac att gat 1056
Lys Ile Leu Cys Ala Thr Tyr Val Asn Val Asn Ile Arg Asp Ile Asp
340 345 350

aag atc tat gtt cga aca ggt atc tac cat gga gga gaa ccc tta tgt 1104
Lys Ile Tyr Val Arg Thr Gly Ile Tyr His Gly Gly Glu Pro Leu Cys
355 360 365

gac aat gtg aac act caa aga gta cct tgt tcc aat ccc agg tgg aat 1152
Asp Asn Val Asn Thr Gln Arg Val Pro Cys Ser Asn Pro Arg Trp Asn
370 375 380

gaa tgg ctg aat tat gat ata tac att cct gat ctt cct cgt gct gct 1200
Glu Trp Leu Asn Tyr Asp Ile Tyr Ile Pro Asp Leu Pro Arg Ala Ala
385 390 395 400

cga ctt tgc ctt tcc att tgc tct gtt aaa ggc cga aag ggt gct aaa 1248
Arg Leu Cys Leu Ser Ile Cys Ser Val Lys Gly Arg Lys Gly Ala Lys
405 410 415

gag gaa cac tgt cca ttg gca tgg gga aat ata aac ttg ttt gat tac 1296
Glu Glu His Cys Pro Leu Ala Trp Gly Asn Ile Asn Leu Phe Asp Tyr

420 425 430

aca gac act cta gta tct gga aaa atg gct ttg aat ctt tgg cca gta 1344
Thr Asp Thr Leu Val Ser Gly Lys Met Ala Leu Asn Leu Trp Pro Val
435 440 445

cct cat gga tta gaa gat ttg ctg aac cct att ggt gtt act gga tca 1392
Pro His Gly Leu Glu Asp Leu Leu Asn Pro Ile Gly Val Thr Gly Ser
450 455 460

aat cca aat aaa gaa act cca tgc tta gag ttg gag ttt gac tgg ttc 1440
Asn Pro Asn Lys Glu Thr Pro Cys Leu Glu Leu Glu Phe Asp Trp Phe
465 470 475 480

agc agt gtg gta aag ttc cca gat atg tca gtg att gaa gag cat gcc 1488
Ser Ser Val Val Lys Phe Pro Asp Met Ser Val Ile Glu Glu His Ala
485 490 495

aat tgg tct gta tcc cga gaa gca gga ttt agc tat tcc cac gca gga 1536
Asn Trp Ser Val Ser Arg Glu Ala Gly Phe Ser Tyr Ser His Ala Gly
500 505 510

ctg agt aac aga cta gct aga gac aat gaa tta agg gaa aat gac aaa 1584
Leu Ser Asn Arg Leu Ala Arg Asp Asn Glu Leu Arg Glu Asn Asp Lys
515 520 525

gaa cag ctc aaa gca att tct aca cga gat cct ctc tct gaa atc act 1632
Glu Gln Leu Lys Ala Ile Ser Thr Arg Asp Pro Leu Ser Glu Ile Thr
530 535 540

gag cag gag aaa gat ttt cta tgg agt cac aga cac tat tgt gta act 1680
Glu Gln Glu Lys Asp Phe Leu Trp Ser His Arg His Tyr Cys Val Thr
545 550 555 560

atc ccc gaa att cta ccc aaa ttg ctt ctg tct gtt aaa tgg aat tct 1728
Ile Pro Glu Ile Leu Pro Lys Leu Leu Leu Ser Val Lys Trp Asn Ser
565 570 575

aga gat gaa gta gcc cag atg tat tgc ttg gta aaa gat tgg cct cca 1776
Arg Asp Glu Val Ala Gln Met Tyr Cys Leu Val Lys Asp Trp Pro Pro
580 585 590

atc aaa cct gaa cag gct atg gaa ctt ctg gac tgt aat tac cca gat 1824
Ile Lys Pro Glu Gln Ala Met Glu Leu Leu Asp Cys Asn Tyr Pro Asp
595 600 605

cct atg gtt cga ggt ttt gct gtt cgg tgc ttg gaa aaa tat tta aca 1872
Pro Met Val Arg Gly Phe Ala Val Arg Cys Leu Glu Lys Tyr Leu Thr
610 615 620

gat gac aaa ctt tct cag tat tta att cag cta gta cag gtc cta aaa 1920
Asp Asp Lys Leu Ser Gln Tyr Leu Ile Gln Leu Val Gln Val Leu Lys
625 630 635 640

tat gaa caa tat ttg gat aac ttg ctt gtg aga ttt tta ctg aag aaa 1968
Tyr Glu Gln Tyr Leu Asp Asn Leu Leu Val Arg Phe Leu Leu Lys Lys
645 650 655

gca ttg act aat caa agg att ggg cac ttt ttc ttt tgg cat tta aaa 2016
Ala Leu Thr Asn Gln Arg Ile Gly His Phe Phe Phe Trp His Leu Lys
660 665 670

tct gag atg cac aat aaa aca gtt agc cag agg ttt ggc ctg ctt ttg 2064
Ser Glu Met His Asn Lys Thr Val Ser Gln Arg Phe Gly Leu Leu Leu
675 680 685

gag tcc tat tgt cgt gca tgt ggg atg tat ttg aag cac ctg aat agg 2112
Glu Ser Tyr Cys Arg Ala Cys Gly Met Tyr Leu Lys His Leu Asn Arg
690 695 700

caa gtc gag gca atg gaa aag ctc att aac tta act gac att ctc aaa 2160
Gln Val Glu Ala Met Glu Lys Leu Ile Asn Leu Thr Asp Ile Leu Lys
705 710 715 720

cag gag aag aag gat gaa aca caa aag gta cag atg aag ttt tta gtt 2208
Gln Glu Lys Lys Asp Glu Thr Gln Lys Val Gln Met Lys Phe Leu Val

725 730 735

gag caa atg agg cga cca gat ttc atg gat gct cta cag ggc ttt ctg 2256
Glu Gln Met Arg Arg Pro Asp Phe Met Asp Ala Leu Gln Gly Phe Leu
740 745 750

tct cct cta aac cct gct cat caa cta gga aac ctc agg ctt gaa gag 2304
Ser Pro Leu Asn Pro Ala His Gln Leu Gly Asn Leu Arg Leu Glu Glu
755 760 765

tgt cga att atg tcc tct gca aaa agg cca ctg tgg ttg aat tgg gag 2352
Cys Arg Ile Met Ser Ser Ala Lys Arg Pro Leu Trp Leu Asn Trp Glu
770 775 780

aac cca gac atc atg tca gag tta ctg ttt cag aac aat gag atc atc 2400
Asn Pro Asp Ile Met Ser Glu Leu Leu Phe Gln Asn Asn Glu Ile Ile
785 790 795 800

ttt aaa aat ggg gat gat tta cgg caa gat atg cta aca ctt caa att 2448
Phe Lys Asn Gly Asp Asp Leu Arg Gln Asp Met Leu Thr Leu Gln Ile
805 810 815

att cgt att atg gaa aat atc tgg caa aat caa ggt ctt gat ctt cga 2496
Ile Arg Ile Met Glu Asn Ile Trp Gln Asn Gln Gly Leu Asp Leu Arg
820 825 830

atg tta cct tat ggt tgt ctg tca atc ggt gac tgt gtg gga ctt att 2544
Met Leu Pro Tyr Gly Cys Leu Ser Ile Gly Asp Cys Val Gly Leu Ile
835 840 845

gag gtg gtg cga aat tct cac act att atg caa att cag tgc aaa ggc 2592
Glu Val Val Arg Asn Ser His Thr Ile Met Gln Ile Gln Cys Lys Gly
850 855 860

ggc ttg aaa ggt gca ctg cag ttc aac agc cac aca cta cat cag tgg 2640
Gly Leu Lys Gly Ala Leu Gln Phe Asn Ser His Thr Leu His Gln Trp
865 870 875 880

ctc aaa gac aag aac aaa gga gaa ata tat gat gca gcc att gac ctg 2688
Leu Lys Asp Lys Asn Lys Gly Glu Ile Tyr Asp Ala Ala Ile Asp Leu
885 890 895

ttt aca cgt tca tgt gct gga tac tgt gta gct acc ttc att ttg gga 2736
Phe Thr Arg Ser Cys Ala Gly Tyr Cys Val Ala Thr Phe Ile Leu Gly
900 905 910

att gga gat cgt cac aat agt aac atc atg gtg aaa gac gat gga caa 2784
Ile Gly Asp Arg His Asn Ser Asn Ile Met Val Lys Asp Asp Gly Gln
915 920 925

ctg ttt cat ata gat ttt gga cac ttt ttg gat cac aag aag aaa aaa 2832
Leu Phe His Ile Asp Phe Gly His Phe Leu Asp His Lys Lys Lys Lys
930 935 940

ttt ggt tat aaa cga gaa cgt gtg cca ttt gtt ttg aca cag gat ttc 2880
Phe Gly Tyr Lys Arg Glu Arg Val Pro Phe Val Leu Thr Gln Asp Phe
945 950 955 960

tta ata gtg att agt aaa gga gcc caa gaa tgc aca aag aca aga gaa 2928
Leu Ile Val Ile Ser Lys Gly Ala Gln Glu Cys Thr Lys Thr Arg Glu
965 970 975

ttt gag agg ttt cag gag atg tgt tac aag gct tat cta gct att cga 2976
Phe Glu Arg Phe Gln Glu Met Cys Tyr Lys Ala Tyr Leu Ala Ile Arg
980 985 990

cag cat gcc aat ctc ttc ata aat ctt ttc tca atg atg ctt ggc tct 3024
Gln His Ala Asn Leu Phe Ile Asn Leu Phe Ser Met Met Leu Gly Ser
995 1000 1005

gga atg cca gaa cta caa tct ttt gat gac att gca tac att cga 3069
Gly Met Pro Glu Leu Gln Ser Phe Asp Asp Ile Ala Tyr Ile Arg
1010 1015 1020

aag acc cta gcc tta gat aaa act gag caa gag gct ttg gag tat 3114
Lys Thr Leu Ala Leu Asp Lys Thr Glu Gln Glu Ala Leu Glu Tyr

1025 1030 1035

ttc atg aaa caa atg aat gat gca cgc cat ggt ggc tgg aca aca 3159
Phe Met Lys Gln Met Asn Asp Ala Arg His Gly Gly Trp Thr Thr
1040 1045 1050

aaa atg gat tgg atc ttc cac aca att aag cag cat gct ttg aac 3204
Lys Met Asp Trp Ile Phe His Thr Ile Lys Gln His Ala Leu Asn
1055 1060 1065

tga 3207

<210> 10
<211> 1068
<212> PRT
<213> Homo sapiens

<400> 10

Met Pro Pro Arg Pro Ser Ser Gly Glu Leu Trp Gly Ile His Leu Met
1 5 10 15

Pro Pro Arg Ile Leu Val Glu Cys Leu Leu Pro Asn Gly Met Ile Val
20 25 30

Thr Leu Glu Cys Leu Arg Glu Ala Thr Leu Ile Thr Ile Lys His Glu
35 40 45

Leu Phe Lys Glu Ala Arg Lys Tyr Pro Leu His Gln Leu Leu Gln Asp
50 55 60

Glu Ser Ser Tyr Ile Phe Val Ser Val Thr Gln Glu Ala Glu Arg Glu
65 70 75 80

Glu Phe Phe Asp Glu Thr Arg Arg Leu Cys Asp Leu Arg Leu Phe Gln
85 90 95

Pro Phe Leu Lys Val Ile Glu Pro Val Gly Asn Arg Glu Glu Lys Ile
100 105 110

Leu Asn Arg Glu Ile Gly Phe Ala Ile Gly Met Pro Val Cys Glu Phe
115 120 125

Asp Met Val Lys Asp Pro Glu Val Gln Asp Phe Arg Arg Asn Ile Leu
130 135 140

Asn Val Cys Lys Glu Ala Val Asp Leu Arg Asp Leu Asn Ser Pro His
145 150 155 160

Ser Arg Ala Met Tyr Val Tyr Pro Pro Asn Val Glu Ser Ser Pro Glu
165 170 175

Leu Pro Lys His Ile Tyr Asn Lys Leu Asp Lys Gly Gln Ile Ile Val
180 185 190

Val Ile Trp Val Ile Val Ser Pro Asn Asn Asp Lys Gln Lys Tyr Thr
195 200 205

Leu Lys Ile Asn His Asp Cys Val Pro Glu Gln Val Ile Ala Glu Ala
210 215 220

Ile Arg Lys Lys Thr Arg Ser Met Leu Leu Ser Ser Glu Gln Leu Lys
225 230 235 240

Leu Cys Val Leu Glu Tyr Gln Gly Lys Tyr Ile Leu Lys Val Cys Gly
245 250 255

Cys Asp Glu Tyr Phe Leu Glu Lys Tyr Pro Leu Ser Gln Tyr Lys Tyr
260 265 270

Ile Arg Ser Cys Ile Met Leu Gly Arg Met Pro Asn Leu Met Leu Met
275 280 285

Ala Lys Glu Ser Leu Tyr Ser Gln Leu Pro Met Asp Cys Phe Thr Met
290 295 300

Pro Ser Tyr Ser Arg Arg Ile Ser Thr Ala Thr Pro Tyr Met Asn Gly
305 310 315 320

Glu Thr Ser Thr Lys Ser Leu Trp Val Ile Asn Ser Ala Leu Arg Ile
325 330 335

Lys Ile Leu Cys Ala Thr Tyr Val Asn Val Asn Ile Arg Asp Ile Asp
340 345 350

Lys Ile Tyr Val Arg Thr Gly Ile Tyr His Gly Gly Glu Pro Leu Cys
355 360 365

Asp Asn Val Asn Thr Gln Arg Val Pro Cys Ser Asn Pro Arg Trp Asn
370 375 380

Glu Trp Leu Asn Tyr Asp Ile Tyr Ile Pro Asp Leu Pro Arg Ala Ala
385 390 395 400

Arg Leu Cys Leu Ser Ile Cys Ser Val Lys Gly Arg Lys Gly Ala Lys
405 410 415

Glu Glu His Cys Pro Leu Ala Trp Gly Asn Ile Asn Leu Phe Asp Tyr
420 425 430

Thr Asp Thr Leu Val Ser Gly Lys Met Ala Leu Asn Leu Trp Pro Val
435 440 445

Pro His Gly Leu Glu Asp Leu Leu Asn Pro Ile Gly Val Thr Gly Ser
450 455 460

Asn Pro Asn Lys Glu Thr Pro Cys Leu Glu Leu Glu Phe Asp Trp Phe
465 470 475 480

Ser Ser Val Val Lys Phe Pro Asp Met Ser Val Ile Glu Glu His Ala
485 490 495

Asn Trp Ser Val Ser Arg Glu Ala Gly Phe Ser Tyr Ser His Ala Gly
500 505 510

Leu Ser Asn Arg Leu Ala Arg Asp Asn Glu Leu Arg Glu Asn Asp Lys
515 520 525

Glu Gln Leu Lys Ala Ile Ser Thr Arg Asp Pro Leu Ser Glu Ile Thr
530 535 540

Glu Gln Glu Lys Asp Phe Leu Trp Ser His Arg His Tyr Cys Val Thr
545 550 555 560

Ile Pro Glu Ile Leu Pro Lys Leu Leu Leu Ser Val Lys Trp Asn Ser
565 570 575

Arg Asp Glu Val Ala Gln Met Tyr Cys Leu Val Lys Asp Trp Pro Pro
580 585 590

Ile Lys Pro Glu Gln Ala Met Glu Leu Leu Asp Cys Asn Tyr Pro Asp
595 600 605

Pro Met Val Arg Gly Phe Ala Val Arg Cys Leu Glu Lys Tyr Leu Thr
610 615 620

Asp Asp Lys Leu Ser Gln Tyr Leu Ile Gln Leu Val Gln Val Leu Lys
625 630 635 640

Tyr Glu Gln Tyr Leu Asp Asn Leu Leu Val Arg Phe Leu Leu Lys Lys
645 650 655

Ala Leu Thr Asn Gln Arg Ile Gly His Phe Phe Phe Trp His Leu Lys
660 665 670

Ser Glu Met His Asn Lys Thr Val Ser Gln Arg Phe Gly Leu Leu Leu
675 680 685

Glu Ser Tyr Cys Arg Ala Cys Gly Met Tyr Leu Lys His Leu Asn Arg
690 695 700

Gln Val Glu Ala Met Glu Lys Leu Ile Asn Leu Thr Asp Ile Leu Lys
705 710 715 720

Gln Glu Lys Lys Asp Glu Thr Gln Lys Val Gln Met Lys Phe Leu Val
725 730 735

Glu Gln Met Arg Arg Pro Asp Phe Met Asp Ala Leu Gln Gly Phe Leu
740 745 750

Ser Pro Leu Asn Pro Ala His Gln Leu Gly Asn Leu Arg Leu Glu Glu
755 760 765

Cys Arg Ile Met Ser Ser Ala Lys Arg Pro Leu Trp Leu Asn Trp Glu
770 775 780

Asn Pro Asp Ile Met Ser Glu Leu Leu Phe Gln Asn Asn Glu Ile Ile
785 790 795 800

Phe Lys Asn Gly Asp Asp Leu Arg Gln Asp Met Leu Thr Leu Gln Ile
805 810 815

Ile Arg Ile Met Glu Asn Ile Trp Gln Asn Gln Gly Leu Asp Leu Arg
820 825 830

Met Leu Pro Tyr Gly Cys Leu Ser Ile Gly Asp Cys Val Gly Leu Ile
835 840 845

Glu Val Val Arg Asn Ser His Thr Ile Met Gln Ile Gln Cys Lys Gly
850 855 860

Gly Leu Lys Gly Ala Leu Gln Phe Asn Ser His Thr Leu His Gln Trp
865 870 875 880

Leu Lys Asp Lys Asn Lys Gly Glu Ile Tyr Asp Ala Ala Ile Asp Leu
885 890 895

Phe Thr Arg Ser Cys Ala Gly Tyr Cys Val Ala Thr Phe Ile Leu Gly
900 905 910

Ile Gly Asp Arg His Asn Ser Asn Ile Met Val Lys Asp Asp Gly Gln
915 920 925

Leu Phe His Ile Asp Phe Gly His Phe Leu Asp His Lys Lys Lys Lys
930 935 940

Phe Gly Tyr Lys Arg Glu Arg Val Pro Phe Val Leu Thr Gln Asp Phe
945 950 955 960

Leu Ile Val Ile Ser Lys Gly Ala Gln Glu Cys Thr Lys Thr Arg Glu
965 970 975

Phe Glu Arg Phe Gln Glu Met Cys Tyr Lys Ala Tyr Leu Ala Ile Arg
980 985 990

Gln His Ala Asn Leu Phe Ile Asn Leu Phe Ser Met Met Leu Gly Ser
995 1000 1005

Gly Met Pro Glu Leu Gln Ser Phe Asp Asp Ile Ala Tyr Ile Arg
1010 1015 1020

Lys Thr Leu Ala Leu Asp Lys Thr Glu Gln Glu Ala Leu Glu Tyr
1025 1030 1035

Phe Met Lys Gln Met Asn Asp Ala Arg His Gly Gly Trp Thr Thr
1040 1045 1050

Lys Met Asp Trp Ile Phe His Thr Ile Lys Gln His Ala Leu Asn
1055 1060 1065

<210> 11
<211> 3207
<212> DNA
<213> Bos taurus

<220>
<221> CDS
<222> (1)..(3207)

<400> 11

atg cct cca aga cca tca tca ggt gaa ctg tgg ggc atc cac ttg atg 48
Met Pro Pro Arg Pro Ser Ser Gly Glu Leu Trp Gly Ile His Leu Met
1 5 10 15

ccc cca aga atc cta gta gaa tgt tta cta cca aat ggg atg ata gtg 96

Pro Pro Arg Ile Leu Val Glu Cys Leu Leu Pro Asn Gly Met Ile Val
20 25 30

act tta gaa tgc ctc cgt gag gct acg tta ata acg ata aag cat gaa 144
Thr Leu Glu Cys Leu Arg Glu Ala Thr Leu Ile Thr Ile Lys His Glu
35 40 45

cta ttt aaa gaa gca aga aaa tac cct ctc cat caa ctt ctt caa gat 192
Leu Phe Lys Glu Ala Arg Lys Tyr Pro Leu His Gln Leu Leu Gln Asp
50 55 60

gaa tct tct tac att ttc gta agt gtt acc caa gaa gca gaa agg gaa 240
Glu Ser Ser Tyr Ile Phe Val Ser Val Thr Gln Glu Ala Glu Arg Glu
65 70 75 80

gaa ttt ttt gat gaa aca aga cga ctt tgt gac ctt cgg ctt ttt caa 288
Glu Phe Phe Asp Glu Thr Arg Arg Leu Cys Asp Leu Arg Leu Phe Gln
85 90 95

ccc ttt tta aaa gta att gaa cca gta ggc aac cgt gaa gaa aag atc 336
Pro Phe Leu Lys Val Ile Glu Pro Val Gly Asn Arg Glu Glu Lys Ile
100 105 110

ctc aat cga gaa att ggt ttt gct atc ggc atg cca gtg tgt gaa ttc 384
Leu Asn Arg Glu Ile Gly Phe Ala Ile Gly Met Pro Val Cys Glu Phe
115 120 125

gat atg gtt aaa gat cca gaa gta cag gac ttc cga aga aat att ctc 432
Asp Met Val Lys Asp Pro Glu Val Gln Asp Phe Arg Arg Asn Ile Leu
130 135 140

aat gtt tgt aaa gaa gct gtg gat ctt agg gat ctt aat tca cct cat 480
Asn Val Cys Lys Glu Ala Val Asp Leu Arg Asp Leu Asn Ser Pro His
145 150 155 160

agt aga gca atg tat gtt tat cct cca aat gta gaa tct tca cca gaa 528
Ser Arg Ala Met Tyr Val Tyr Pro Pro Asn Val Glu Ser Ser Pro Glu
165 170 175

ctg cca aag cac ata tat aat aaa ttg gat aaa ggg caa ata ata gtg 576
Leu Pro Lys His Ile Tyr Asn Lys Leu Asp Lys Gly Gln Ile Ile Val
180 185 190

gtg att tgg gta ata gtt tct cca aat aat gac aaa cag aag tat act 624
Val Ile Trp Val Ile Val Ser Pro Asn Asn Asp Lys Gln Lys Tyr Thr
195 200 205

ctg aaa atc aac cat gac tgt gtg cca gaa caa gta att gct gaa gca 672
Leu Lys Ile Asn His Asp Cys Val Pro Glu Gln Val Ile Ala Glu Ala
210 215 220

atc agg aaa aaa act cga agt atg ttg cta tca tct gaa caa cta aaa 720
Ile Arg Lys Lys Thr Arg Ser Met Leu Leu Ser Ser Glu Gln Leu Lys
225 230 235 240

ctc tgt gtt tta gaa tat cag ggc aag tat att tta aaa gtg tgt gga 768
Leu Cys Val Leu Glu Tyr Gln Gly Lys Tyr Ile Leu Lys Val Cys Gly
245 250 255

tgt gat gaa tac ttc cta gaa aaa tat cct ctg agt cag tat aag tat 816
Cys Asp Glu Tyr Phe Leu Glu Lys Tyr Pro Leu Ser Gln Tyr Lys Tyr
260 265 270

ata aga agc tgt ata atg ctt ggg agg atg ccc aat ttg atg ctg atg 864
Ile Arg Ser Cys Ile Met Leu Gly Arg Met Pro Asn Leu Met Leu Met
275 280 285

gct aaa gaa agc ctc tat tct caa ctg cca atg gac tgt ttt aca atg 912
Ala Lys Glu Ser Leu Tyr Ser Gln Leu Pro Met Asp Cys Phe Thr Met
290 295 300

cca tca tat tcc aga cgc atc tcc aca gct acg cca tat atg aat gga 960
Pro Ser Tyr Ser Arg Arg Ile Ser Thr Ala Thr Pro Tyr Met Asn Gly
305 310 315 320

gaa aca tct aca aaa tcc ctt tgg gtt ata aat agt gca ctc aga ata 1008

Glu Thr Ser Thr Lys Ser Leu Trp Val Ile Asn Ser Ala Leu Arg Ile
325 330 335

aaa att ctt tgt gca acc tat gtg aat gta aat att cga gac att gac 1056
Lys Ile Leu Cys Ala Thr Tyr Val Asn Val Asn Ile Arg Asp Ile Asp
340 345 350

aag att tat gtt cga aca ggt atc tac cat gga gga gaa ccc tta tgt 1104
Lys Ile Tyr Val Arg Thr Gly Ile Tyr His Gly Gly Glu Pro Leu Cys
355 360 365

gat aat gtg aac act caa aga gta cct tgt tcc aat ccc agg tgg aat 1152
Asp Asn Val Asn Thr Gln Arg Val Pro Cys Ser Asn Pro Arg Trp Asn
370 375 380

gaa tgg ctg aat tac gat ata tac att cct gat ctt cct cgt gct gct 1200
Glu Trp Leu Asn Tyr Asp Ile Tyr Ile Pro Asp Leu Pro Arg Ala Ala
385 390 395 400

cga ctt tgc ctt tcc att tgt tct gtt aaa ggc cga aag ggt gct aaa 1248
Arg Leu Cys Leu Ser Ile Cys Ser Val Lys Gly Arg Lys Gly Ala Lys
405 410 415

gag gaa cac tgt cca ttg gcc tgg gga aat ata aac ttg ttt gat tac 1296
Glu Glu His Cys Pro Leu Ala Trp Gly Asn Ile Asn Leu Phe Asp Tyr
420 425 430

aca gat act cta gta tct gga aaa atg gct ttg aat ctt tgg cca gta 1344
Thr Asp Thr Leu Val Ser Gly Lys Met Ala Leu Asn Leu Trp Pro Val
435 440 445

cct cat gga cta gaa gat ttg ctg aac cct att ggt gtt act gga tca 1392
Pro His Gly Leu Glu Asp Leu Leu Asn Pro Ile Gly Val Thr Gly Ser
450 455 460

aat cca aat aaa gaa act cca tgt tta gag ttg gag ttt gac tgg ttc 1440
Asn Pro Asn Lys Glu Thr Pro Cys Leu Glu Leu Glu Phe Asp Trp Phe
465 470 475 480

agc agt gtg gta aag ttt cca gat atg tca gtg att gaa gag cat gcc 1488
Ser Ser Val Val Lys Phe Pro Asp Met Ser Val Ile Glu Glu His Ala
485 490 495

aat tgg tct gta tcc cgt gaa gca gga ttt agt tat tcc cat gca gga 1536
Asn Trp Ser Val Ser Arg Glu Ala Gly Phe Ser Tyr Ser His Ala Gly
500 505 510

ctg agt aac aga cta gct aga gac aat gaa tta aga gaa aat gat aaa 1584
Leu Ser Asn Arg Leu Ala Arg Asp Asn Glu Leu Arg Glu Asn Asp Lys
515 520 525

gaa cag ctc cga gca att tgt aca cga gat cct cta tct gaa atc act 1632
Glu Gln Leu Arg Ala Ile Cys Thr Arg Asp Pro Leu Ser Glu Ile Thr
530 535 540

gag caa gag aaa gat ttt ctg tgg agc cac aga cac tat tgt gta act 1680
Glu Gln Glu Lys Asp Phe Leu Trp Ser His Arg His Tyr Cys Val Thr
545 550 555 560

atc ccc gaa att cta ccc aaa ttg ctt ctg tct gtt aaa tgg aac tct 1728
Ile Pro Glu Ile Leu Pro Lys Leu Leu Leu Ser Val Lys Trp Asn Ser
565 570 575

aga gat gaa gta gct cag atg tac tgc ttg gta aaa gat tgg cct cca 1776
Arg Asp Glu Val Ala Gln Met Tyr Cys Leu Val Lys Asp Trp Pro Pro
580 585 590

atc aag cct gaa cag gct atg gag ctt ctg gac tgc aat tac cca gat 1824
Ile Lys Pro Glu Gln Ala Met Glu Leu Leu Asp Cys Asn Tyr Pro Asp
595 600 605

cct atg gtt cga ggt ttt gct gtt cgg tgc tta gaa aaa tat tta aca 1872
Pro Met Val Arg Gly Phe Ala Val Arg Cys Leu Glu Lys Tyr Leu Thr
610 615 620

gat gac aaa ctt tct cag tac cta att cag cta gta cag gta cta aaa 1920

Asp Asp Lys Leu Ser Gln Tyr Leu Ile Gln Leu Val Gln Val Leu Lys
625 630 635 640

tat gaa cag tat ttg gat aac ctg ctt gtg aga ttt tta ctc aaa aaa 1968
Tyr Glu Gln Tyr Leu Asp Asn Leu Leu Val Arg Phe Leu Leu Lys Lys
645 650 655

gcg tta act aat caa agg atc ggt cac ttt ttc ttt tgg cat tta aaa 2016
Ala Leu Thr Asn Gln Arg Ile Gly His Phe Phe Phe Trp His Leu Lys
660 665 670

tct gag atg cac aat aaa aca gtt agt cag agg ttt ggc ctg ctt ttg 2064
Ser Glu Met His Asn Lys Thr Val Ser Gln Arg Phe Gly Leu Leu Leu
675 680 685

gag tcc tat tgc cgt gca tgt ggg atg tat ctg aag cac ctt aat agg 2112
Glu Ser Tyr Cys Arg Ala Cys Gly Met Tyr Leu Lys His Leu Asn Arg
690 695 700

caa gtt gag gct atg gaa aag ctc att aac ttg act gac att ctc aaa 2160
Gln Val Glu Ala Met Glu Lys Leu Ile Asn Leu Thr Asp Ile Leu Lys
705 710 715 720

caa gag aag aag gat gaa aca caa aag gta cag atg aag ttt tta gtt 2208
Gln Glu Lys Lys Asp Glu Thr Gln Lys Val Gln Met Lys Phe Leu Val
725 730 735

gag caa atg cgg cga cca gat ttc atg gat gct ctc cag ggc ttt ctg 2256
Glu Gln Met Arg Arg Pro Asp Phe Met Asp Ala Leu Gln Gly Phe Leu
740 745 750

tct cct cta aac cct gct cat cag ctg gga aat ctc agg ctt gaa gag 2304
Ser Pro Leu Asn Pro Ala His Gln Leu Gly Asn Leu Arg Leu Glu Glu
755 760 765

tgt cga att atg tct tct gca aaa agg cca ctg tgg ttg aat tgg gag 2352
Cys Arg Ile Met Ser Ser Ala Lys Arg Pro Leu Trp Leu Asn Trp Glu
770 775 780

aac cca gac atc atg tca gaa tta ctc ttt cag aac aat gag atc atc 2400
Asn Pro Asp Ile Met Ser Glu Leu Leu Phe Gln Asn Asn Glu Ile Ile
785 790 795 800

ttt aaa aat ggg gat gat tta cgg caa gat atg cta acc ctt cag att 2448
Phe Lys Asn Gly Asp Asp Leu Arg Gln Asp Met Leu Thr Leu Gln Ile
805 810 815

att cgc att atg gaa aat atc tgg caa aat caa ggt ctt gat ctt cga 2496
Ile Arg Ile Met Glu Asn Ile Trp Gln Asn Gln Gly Leu Asp Leu Arg
820 825 830

atg tta cct tat gga tgt ctg tca atc ggt gac tgt gtg gga ctt atc 2544
Met Leu Pro Tyr Gly Cys Leu Ser Ile Gly Asp Cys Val Gly Leu Ile
835 840 845

gag gtg gtg aga aat tct cac act ata atg cag att cag tgt aaa gga 2592
Glu Val Val Arg Asn Ser His Thr Ile Met Gln Ile Gln Cys Lys Gly
850 855 860

ggc ctg aaa ggt gca ctg cag ttt aac agc cac aca ctc cat cag tgg 2640
Gly Leu Lys Gly Ala Leu Gln Phe Asn Ser His Thr Leu His Gln Trp
865 870 875 880

ctc aaa gac aag aac aag ggg gaa ata tat gat gcg gcc atc gat ttg 2688
Leu Lys Asp Lys Asn Lys Gly Glu Ile Tyr Asp Ala Ala Ile Asp Leu
885 890 895

ttt aca cga tca tgt gct gga tat tgt gtt gcc acc ttc att ttg gga 2736
Phe Thr Arg Ser Cys Ala Gly Tyr Cys Val Ala Thr Phe Ile Leu Gly
900 905 910

att gga gat cgt cac aat agt aat atc atg gtt aaa gat gat gga caa 2784
Ile Gly Asp Arg His Asn Ser Asn Ile Met Val Lys Asp Asp Gly Gln
915 920 925

ctg ttt cat ata gat ttt gga cac ttt ttg gat cac aag aag aaa aaa 2832

Leu Phe His Ile Asp Phe Gly His Phe Leu Asp His Lys Lys Lys Lys
930 935 940

ttt ggt tat aaa cga gag cgc gtg ccg ttt gtt ttg aca caa gat ttc 2880
Phe Gly Tyr Lys Arg Glu Arg Val Pro Phe Val Leu Thr Gln Asp Phe
945 950 955 960

tta ata gtg att agt aaa gga gcc caa gaa tgc aca aag aca aga gaa 2928
Leu Ile Val Ile Ser Lys Gly Ala Gln Glu Cys Thr Lys Thr Arg Glu
965 970 975

ttt gag agg ttt cag gag atg tgt tac aag gct tat cta gct att cgg 2976
Phe Glu Arg Phe Gln Glu Met Cys Tyr Lys Ala Tyr Leu Ala Ile Arg
980 985 990

cag cat gcc aat ctc ttc ata aat ctt ttc tca atg atg ctt ggc tct 3024
Gln His Ala Asn Leu Phe Ile Asn Leu Phe Ser Met Met Leu Gly Ser
995 1000 1005

gga atg cca gaa ctg caa tct ttt gat gat att gca tac att cga 3069
Gly Met Pro Glu Leu Gln Ser Phe Asp Asp Ile Ala Tyr Ile Arg
1010 1015 1020

aag acc cta gct tta gat aaa act gag caa gag gct ttg gag tat 3114
Lys Thr Leu Ala Leu Asp Lys Thr Glu Gln Glu Ala Leu Glu Tyr
1025 1030 1035

ttc atg aaa caa atg aat gat gca cac cat ggt ggc tgg aca aca 3159
Phe Met Lys Gln Met Asn Asp Ala His His Gly Gly Trp Thr Thr
1040 1045 1050

aaa atg gat tgg atc ttc cac aca att aag cag cat gct ttg aac 3204
Lys Met Asp Trp Ile Phe His Thr Ile Lys Gln His Ala Leu Asn
1055 1060 1065

tga 3207

<210> 12
<211> 1068
<212> PRT

<213> Bos taurus

<400> 12

Met Pro Pro Arg Pro Ser Ser Gly Glu Leu Trp Gly Ile His Leu Met
1               5                   10                  15

Pro Pro Arg Ile Leu Val Glu Cys Leu Leu Pro Asn Gly Met Ile Val
            20                  25                  30

Thr Leu Glu Cys Leu Arg Glu Ala Thr Leu Ile Thr Ile Lys His Glu
        35                  40                  45

Leu Phe Lys Glu Ala Arg Lys Tyr Pro Leu His Gln Leu Leu Gln Asp
    50                  55                  60

Glu Ser Ser Tyr Ile Phe Val Ser Val Thr Gln Glu Ala Glu Arg Glu
65                  70                  75                  80

Glu Phe Phe Asp Glu Thr Arg Arg Leu Cys Asp Leu Arg Leu Phe Gln
                85                  90                  95

Pro Phe Leu Lys Val Ile Glu Pro Val Gly Asn Arg Glu Glu Lys Ile
            100                 105                 110

Leu Asn Arg Glu Ile Gly Phe Ala Ile Gly Met Pro Val Cys Glu Phe
        115                 120                 125

Asp Met Val Lys Asp Pro Glu Val Gln Asp Phe Arg Arg Asn Ile Leu
130 135 140

Asn Val Cys Lys Glu Ala Val Asp Leu Arg Asp Leu Asn Ser Pro His
145 150 155 160

Ser Arg Ala Met Tyr Val Tyr Pro Pro Asn Val Glu Ser Ser Pro Glu
165 170 175

Leu Pro Lys His Ile Tyr Asn Lys Leu Asp Lys Gly Gln Ile Ile Val
180 185 190

Val Ile Trp Val Ile Val Ser Pro Asn Asn Asp Lys Gln Lys Tyr Thr
195 200 205

Leu Lys Ile Asn His Asp Cys Val Pro Glu Gln Val Ile Ala Glu Ala
210 215 220

Ile Arg Lys Lys Thr Arg Ser Met Leu Leu Ser Ser Glu Gln Leu Lys
225 230 235 240

Leu Cys Val Leu Glu Tyr Gln Gly Lys Tyr Ile Leu Lys Val Cys Gly
245 250 255

Cys Asp Glu Tyr Phe Leu Glu Lys Tyr Pro Leu Ser Gln Tyr Lys Tyr
260 265 270

Ile Arg Ser Cys Ile Met Leu Gly Arg Met Pro Asn Leu Met Leu Met

275 280 285

Ala Lys Glu Ser Leu Tyr Ser Gln Leu Pro Met Asp Cys Phe Thr Met
290 295 300

Pro Ser Tyr Ser Arg Arg Ile Ser Thr Ala Thr Pro Tyr Met Asn Gly
305 310 315 320

Glu Thr Ser Thr Lys Ser Leu Trp Val Ile Asn Ser Ala Leu Arg Ile
325 330 335

Lys Ile Leu Cys Ala Thr Tyr Val Asn Val Asn Ile Arg Asp Ile Asp
340 345 350

Lys Ile Tyr Val Arg Thr Gly Ile Tyr His Gly Gly Glu Pro Leu Cys
355 360 365

Asp Asn Val Asn Thr Gln Arg Val Pro Cys Ser Asn Pro Arg Trp Asn
370 375 380

Glu Trp Leu Asn Tyr Asp Ile Tyr Ile Pro Asp Leu Pro Arg Ala Ala
385 390 395 400

Arg Leu Cys Leu Ser Ile Cys Ser Val Lys Gly Arg Lys Gly Ala Lys
405 410 415

Glu Glu His Cys Pro Leu Ala Trp Gly Asn Ile Asn Leu Phe Asp Tyr
420 425 430

Thr Asp Thr Leu Val Ser Gly Lys Met Ala Leu Asn Leu Trp Pro Val
435 440 445

Pro His Gly Leu Glu Asp Leu Leu Asn Pro Ile Gly Val Thr Gly Ser
450 455 460

Asn Pro Asn Lys Glu Thr Pro Cys Leu Glu Leu Glu Phe Asp Trp Phe
465 470 475 480

Ser Ser Val Val Lys Phe Pro Asp Met Ser Val Ile Glu Glu His Ala
485 490 495

Asn Trp Ser Val Ser Arg Glu Ala Gly Phe Ser Tyr Ser His Ala Gly
500 505 510

Leu Ser Asn Arg Leu Ala Arg Asp Asn Glu Leu Arg Glu Asn Asp Lys
515 520 525

Glu Gln Leu Arg Ala Ile Cys Thr Arg Asp Pro Leu Ser Glu Ile Thr
530 535 540

Glu Gln Glu Lys Asp Phe Leu Trp Ser His Arg His Tyr Cys Val Thr
545 550 555 560

Ile Pro Glu Ile Leu Pro Lys Leu Leu Leu Ser Val Lys Trp Asn Ser
565 570 575

Arg Asp Glu Val Ala Gln Met Tyr Cys Leu Val Lys Asp Trp Pro Pro

580 585 590

Ile Lys Pro Glu Gln Ala Met Glu Leu Leu Asp Cys Asn Tyr Pro Asp
595 600 605

Pro Met Val Arg Gly Phe Ala Val Arg Cys Leu Glu Lys Tyr Leu Thr
610 615 620

Asp Asp Lys Leu Ser Gln Tyr Leu Ile Gln Leu Val Gln Val Leu Lys
625 630 635 640

Tyr Glu Gln Tyr Leu Asp Asn Leu Leu Val Arg Phe Leu Leu Lys Lys
645 650 655

Ala Leu Thr Asn Gln Arg Ile Gly His Phe Phe Phe Trp His Leu Lys
660 665 670

Ser Glu Met His Asn Lys Thr Val Ser Gln Arg Phe Gly Leu Leu Leu
675 680 685

Glu Ser Tyr Cys Arg Ala Cys Gly Met Tyr Leu Lys His Leu Asn Arg
690 695 700

Gln Val Glu Ala Met Glu Lys Leu Ile Asn Leu Thr Asp Ile Leu Lys
705 710 715 720

Gln Glu Lys Lys Asp Glu Thr Gln Lys Val Gln Met Lys Phe Leu Val
725 730 735

Glu Gln Met Arg Arg Pro Asp Phe Met Asp Ala Leu Gln Gly Phe Leu
740 745 750

Ser Pro Leu Asn Pro Ala His Gln Leu Gly Asn Leu Arg Leu Glu Glu
755 760 765

Cys Arg Ile Met Ser Ser Ala Lys Arg Pro Leu Trp Leu Asn Trp Glu
770 775 780

Asn Pro Asp Ile Met Ser Glu Leu Leu Phe Gln Asn Asn Glu Ile Ile
785 790 795 800

Phe Lys Asn Gly Asp Asp Leu Arg Gln Asp Met Leu Thr Leu Gln Ile
805 810 815

Ile Arg Ile Met Glu Asn Ile Trp Gln Asn Gln Gly Leu Asp Leu Arg
820 825 830

Met Leu Pro Tyr Gly Cys Leu Ser Ile Gly Asp Cys Val Gly Leu Ile
835 840 845

Glu Val Val Arg Asn Ser His Thr Ile Met Gln Ile Gln Cys Lys Gly
850 855 860

Gly Leu Lys Gly Ala Leu Gln Phe Asn Ser His Thr Leu His Gln Trp
865 870 875 880

Leu Lys Asp Lys Asn Lys Gly Glu Ile Tyr Asp Ala Ala Ile Asp Leu

885 890 895

Phe Thr Arg Ser Cys Ala Gly Tyr Cys Val Ala Thr Phe Ile Leu Gly
900 905 910

Ile Gly Asp Arg His Asn Ser Asn Ile Met Val Lys Asp Asp Gly Gln
915 920 925

Leu Phe His Ile Asp Phe Gly His Phe Leu Asp His Lys Lys Lys Lys
930 935 940

Phe Gly Tyr Lys Arg Glu Arg Val Pro Phe Val Leu Thr Gln Asp Phe
945 950 955 960

Leu Ile Val Ile Ser Lys Gly Ala Gln Glu Cys Thr Lys Thr Arg Glu
965 970 975

Phe Glu Arg Phe Gln Glu Met Cys Tyr Lys Ala Tyr Leu Ala Ile Arg
980 985 990

Gln His Ala Asn Leu Phe Ile Asn Leu Phe Ser Met Met Leu Gly Ser
995 1000 1005

Gly Met Pro Glu Leu Gln Ser Phe Asp Asp Ile Ala Tyr Ile Arg
1010 1015 1020

Lys Thr Leu Ala Leu Asp Lys Thr Glu Gln Glu Ala Leu Glu Tyr
1025 1030 1035

Phe Met Lys Gln Met Asn Asp Ala His His Gly Gly Trp Thr Thr
1040 1045 1050

Lys Met Asp Trp Ile Phe His Thr Ile Lys Gln His Ala Leu Asn
1055 1060 1065

<210> 13
<211> 888
<212> DNA
<213> Homo sapiens

<220>
<221> CDS
<222> (1)..(888)

<400> 13

atg gaa cac cag ctc ctg tgc tgc gaa gtg gaa acc atc cgc cgc gcg 48
Met Glu His Gln Leu Leu Cys Cys Glu Val Glu Thr Ile Arg Arg Ala
1 5 10 15

tac ccc gat gcc aac ctc ctc aac gac cgg gtg ctg cgg gcc atg ctg 96
Tyr Pro Asp Ala Asn Leu Leu Asn Asp Arg Val Leu Arg Ala Met Leu
20 25 30

aag gcg gag gag acc tgc gcg ccc tcg gtg tcc tac ttc aaa tgt gtg 144
Lys Ala Glu Glu Thr Cys Ala Pro Ser Val Ser Tyr Phe Lys Cys Val
35 40 45

cag aag gag gtc ctg ccg tcc atg cgg aag atc gtc gcc acc tgg atg 192
Gln Lys Glu Val Leu Pro Ser Met Arg Lys Ile Val Ala Thr Trp Met
50 55 60

ctg gag gtc tgc gag gaa cag aag tgc gag gag gag gtc ttc ccg ctg 240
Leu Glu Val Cys Glu Glu Gln Lys Cys Glu Glu Glu Val Phe Pro Leu

65 70 75 80

gcc atg aac tac ctg gac cgc ttc ctg tcg ctg gag ccc gtg aaa aag 288
Ala Met Asn Tyr Leu Asp Arg Phe Leu Ser Leu Glu Pro Val Lys Lys
85 90 95

agc cgc ctg cag ctg ctg ggg gcc act tgc atg ttc gtg gcc tct aag 336
Ser Arg Leu Gln Leu Leu Gly Ala Thr Cys Met Phe Val Ala Ser Lys
100 105 110

atg aag gag acc atc ccc ctg acg gcc gag aag ctg tgc atc tac acc 384
Met Lys Glu Thr Ile Pro Leu Thr Ala Glu Lys Leu Cys Ile Tyr Thr
115 120 125

gac aac tcc atc cgg ccc gag gag ctg ctg caa atg gag ctg ctc ctg 432
Asp Asn Ser Ile Arg Pro Glu Glu Leu Leu Gln Met Glu Leu Leu Leu
130 135 140

gtg aac aag ctc aag tgg aac ctg gcc gca atg acc ccg cac gat ttc 480
Val Asn Lys Leu Lys Trp Asn Leu Ala Ala Met Thr Pro His Asp Phe
145 150 155 160

att gaa cac ttc ctc tcc aaa atg cca gag gcg gag gag aac aaa cag 528
Ile Glu His Phe Leu Ser Lys Met Pro Glu Ala Glu Glu Asn Lys Gln
165 170 175

atc atc cgc aaa cac gcg cag acc ttc gtt gcc ctc tgt gcc aca gat 576
Ile Ile Arg Lys His Ala Gln Thr Phe Val Ala Leu Cys Ala Thr Asp
180 185 190

gtg aag ttc att tcc aat ccg ccc tcc atg gtg gca gcg ggg agc gtg 624
Val Lys Phe Ile Ser Asn Pro Pro Ser Met Val Ala Ala Gly Ser Val
195 200 205

gtg gcc gca gtg caa ggc ctg aac ctg agg agc ccc aac aac ttc ctg 672
Val Ala Ala Val Gln Gly Leu Asn Leu Arg Ser Pro Asn Asn Phe Leu
210 215 220

tcc tac tac cgc ctc aca cgc ttc ctc tcc aga gtg atc aag tgt gac 720
Ser Tyr Tyr Arg Leu Thr Arg Phe Leu Ser Arg Val Ile Lys Cys Asp
225 230 235 240

ccg gac tgc ctc cgg gcc tgc cag gag cag atc gaa gcc ctg ctg gag 768
Pro Asp Cys Leu Arg Ala Cys Gln Glu Gln Ile Glu Ala Leu Leu Glu
245 250 255

tca agc ctg cgc cag gcc cag cag aac atg gac ccc aag gcc gcc gag 816
Ser Ser Leu Arg Gln Ala Gln Gln Asn Met Asp Pro Lys Ala Ala Glu
260 265 270

gag gag gaa gag gag gag gag gag gtg gac ctg gct tgc aca ccc acc 864
Glu Glu Glu Glu Glu Glu Glu Glu Val Asp Leu Ala Cys Thr Pro Thr
275 280 285

gac gtg cgg gac gtg gac atc tga 888
Asp Val Arg Asp Val Asp Ile
290 295

<210> 14
<211> 295
<212> PRT
<213> Homo sapiens

<400> 14

Met Glu His Gln Leu Leu Cys Cys Glu Val Glu Thr Ile Arg Arg Ala
1 5 10 15

Tyr Pro Asp Ala Asn Leu Leu Asn Asp Arg Val Leu Arg Ala Met Leu
20 25 30

Lys Ala Glu Glu Thr Cys Ala Pro Ser Val Ser Tyr Phe Lys Cys Val
35 40 45

Gln Lys Glu Val Leu Pro Ser Met Arg Lys Ile Val Ala Thr Trp Met
50 55 60

Leu Glu Val Cys Glu Glu Gln Lys Cys Glu Glu Glu Val Phe Pro Leu
65 70 75 80

Ala Met Asn Tyr Leu Asp Arg Phe Leu Ser Leu Glu Pro Val Lys Lys
85 90 95

Ser Arg Leu Gln Leu Leu Gly Ala Thr Cys Met Phe Val Ala Ser Lys
100 105 110

Met Lys Glu Thr Ile Pro Leu Thr Ala Glu Lys Leu Cys Ile Tyr Thr
115 120 125

Asp Asn Ser Ile Arg Pro Glu Glu Leu Leu Gln Met Glu Leu Leu Leu
130 135 140

Val Asn Lys Leu Lys Trp Asn Leu Ala Ala Met Thr Pro His Asp Phe
145 150 155 160

Ile Glu His Phe Leu Ser Lys Met Pro Glu Ala Glu Glu Asn Lys Gln
165 170 175

Ile Ile Arg Lys His Ala Gln Thr Phe Val Ala Leu Cys Ala Thr Asp
180 185 190

Val Lys Phe Ile Ser Asn Pro Pro Ser Met Val Ala Ala Gly Ser Val
195 200 205

Val Ala Ala Val Gln Gly Leu Asn Leu Arg Ser Pro Asn Asn Phe Leu
210 215 220

Ser Tyr Tyr Arg Leu Thr Arg Phe Leu Ser Arg Val Ile Lys Cys Asp
225 230 235 240

Pro Asp Cys Leu Arg Ala Cys Gln Glu Gln Ile Glu Ala Leu Leu Glu
245 250 255

Ser Ser Leu Arg Gln Ala Gln Gln Asn Met Asp Pro Lys Ala Ala Glu
260 265 270

Glu Glu Glu Glu Glu Glu Glu Glu Val Asp Leu Ala Cys Thr Pro Thr
275 280 285

Asp Val Arg Asp Val Asp Ile
290 295

<210> 15
<211> 1302
<212> DNA
<213> Homo sapiens

<220>
<221> CDS
<222> (1)..(1302)

<400> 15

atg gag gtg gtg gac ccg cag cag ctg ggc atg ttc acg gag ggc gag 48
Met Glu Val Val Asp Pro Gln Gln Leu Gly Met Phe Thr Glu Gly Glu
1 5 10 15

ctg atg tcg gtg ggt atg gac acg ttc atc cac cgc atc gac tcc acc 96
Leu Met Ser Val Gly Met Asp Thr Phe Ile His Arg Ile Asp Ser Thr
20 25 30

gag gtc atc tac cag ccg cgc cgc aag cgg gcc aag ctc atc ggc aag 144
Glu Val Ile Tyr Gln Pro Arg Arg Lys Arg Ala Lys Leu Ile Gly Lys
35 40 45

tac ctg atg ggg gac ctg ctg ggg gaa ggc tct tac ggc aag gtg aag 192
Tyr Leu Met Gly Asp Leu Leu Gly Glu Gly Ser Tyr Gly Lys Val Lys
50 55 60

gag gtg ctg gac tcg gag acg ctg tgc agg agg gcc gtc aag atc ctc 240
Glu Val Leu Asp Ser Glu Thr Leu Cys Arg Arg Ala Val Lys Ile Leu
65 70 75 80

aag aag aag aag ttg cga agg atc ccc aac ggg gag gcc aac gtg aag 288
Lys Lys Lys Lys Leu Arg Arg Ile Pro Asn Gly Glu Ala Asn Val Lys
85 90 95

aag gaa att caa cta ctg agg agg tta cgg cac aaa aat gtc atc cag 336
Lys Glu Ile Gln Leu Leu Arg Arg Leu Arg His Lys Asn Val Ile Gln
100 105 110

ctg gtg gat gtg tta tac aac gaa gag aag cag aaa atg tat atg gtg 384
Leu Val Asp Val Leu Tyr Asn Glu Glu Lys Gln Lys Met Tyr Met Val
115 120 125

atg gag tac tgc gtg tgt ggc atg cag gaa atg ctg gac agc gtg ccg 432
Met Glu Tyr Cys Val Cys Gly Met Gln Glu Met Leu Asp Ser Val Pro
130 135 140

gag aag cgt ttc cca gtg tgc cag gcc cac ggg tac ttc tgt cag ctg 480

Glu Lys Arg Phe Pro Val Cys Gln Ala His Gly Tyr Phe Cys Gln Leu
145 150 155 160

att gac ggc ctg gag tac ctg cat agc cag ggc att gtg cac aag gac 528
Ile Asp Gly Leu Glu Tyr Leu His Ser Gln Gly Ile Val His Lys Asp
165 170 175

atc aag ccg ggg aac ctg ctg ctc acc acc ggt ggc acc ctc aaa atc 576
Ile Lys Pro Gly Asn Leu Leu Leu Thr Thr Gly Gly Thr Leu Lys Ile
180 185 190

tcc gcc ctg ggc gtg gcc gag gca ctg cac ccg ttc gcg gcg gac gac 624
Ser Ala Leu Gly Val Ala Glu Ala Leu His Pro Phe Ala Ala Asp Asp
195 200 205

acc tgc cgg acc agc cag ggc tcc ccg gct ttc cag ccg ccc gag att 672
Thr Cys Arg Thr Ser Gln Gly Ser Pro Ala Phe Gln Pro Pro Glu Ile
210 215 220

gcc aac ggc ctg gac acc ttc tcc ggc ttc aag gtg gac atc tgg tcg 720
Ala Asn Gly Leu Asp Thr Phe Ser Gly Phe Lys Val Asp Ile Trp Ser
225 230 235 240

gct ggg gtc acc ctc tac aac atc acc acg ggt ctg tac ccc ttc gaa 768
Ala Gly Val Thr Leu Tyr Asn Ile Thr Thr Gly Leu Tyr Pro Phe Glu
245 250 255

ggg gac aac atc tac aag ttg ttt gag aac atc ggg aag ggg agc tac 816
Gly Asp Asn Ile Tyr Lys Leu Phe Glu Asn Ile Gly Lys Gly Ser Tyr
260 265 270

gcc atc ccg ggc gac tgt ggc ccc ccg ctc tct gac ctg ctg aaa ggg 864
Ala Ile Pro Gly Asp Cys Gly Pro Pro Leu Ser Asp Leu Leu Lys Gly
275 280 285

atg ctt gag tac gaa ccg gcc aag agg ttc tcc atc cgg cag atc cgg 912
Met Leu Glu Tyr Glu Pro Ala Lys Arg Phe Ser Ile Arg Gln Ile Arg
290 295 300

cag cac agc tgg ttc cgg aag aaa cat cct ccg gct gaa gca cca gtg 960
Gln His Ser Trp Phe Arg Lys Lys His Pro Pro Ala Glu Ala Pro Val
305 310 315 320

ccc atc cca ccg agc cca gac acc aag gac cgg tgg cgc agc atg act 1008
Pro Ile Pro Pro Ser Pro Asp Thr Lys Asp Arg Trp Arg Ser Met Thr
325 330 335

gtg gtg ccg tac ttg gag gac ctg cac ggc gcg gac gag gac gag gac 1056
Val Val Pro Tyr Leu Glu Asp Leu His Gly Ala Asp Glu Asp Glu Asp
340 345 350

ctc ttc gac atc gag gat gac atc atc tac act cag gac ttc acg gtg 1104
Leu Phe Asp Ile Glu Asp Asp Ile Ile Tyr Thr Gln Asp Phe Thr Val
355 360 365

ccc gga cag gtc cca gaa gag gag gcc agt cac aat gga cag cgc cgg 1152
Pro Gly Gln Val Pro Glu Glu Glu Ala Ser His Asn Gly Gln Arg Arg
370 375 380

ggc ctc ccc aag gcc gtg tgt atg aac ggc aca gag gcg gcg cag ctg 1200
Gly Leu Pro Lys Ala Val Cys Met Asn Gly Thr Glu Ala Ala Gln Leu
385 390 395 400

agc acc aaa tcc agg gcg gag ggc cgg gcc ccc aac cct gcc cgc aag 1248
Ser Thr Lys Ser Arg Ala Glu Gly Arg Ala Pro Asn Pro Ala Arg Lys
405 410 415

gcc tgc tcc gcc agc agc aag atc cgc cgg ctg tcg gcc tgc aag cag 1296
Ala Cys Ser Ala Ser Ser Lys Ile Arg Arg Leu Ser Ala Cys Lys Gln
420 425 430

cag tga 1302
Gln

<210> 16
<211> 433
<212> PRT
<213> Homo sapiens

<400> 16

Met Glu Val Val Asp Pro Gln Gln Leu Gly Met Phe Thr Glu Gly Glu
1 5 10 15

Leu Met Ser Val Gly Met Asp Thr Phe Ile His Arg Ile Asp Ser Thr
20 25 30

Glu Val Ile Tyr Gln Pro Arg Arg Lys Arg Ala Lys Leu Ile Gly Lys
35 40 45

Tyr Leu Met Gly Asp Leu Leu Gly Glu Gly Ser Tyr Gly Lys Val Lys
50 55 60

Glu Val Leu Asp Ser Glu Thr Leu Cys Arg Arg Ala Val Lys Ile Leu
65 70 75 80

Lys Lys Lys Lys Leu Arg Arg Ile Pro Asn Gly Glu Ala Asn Val Lys
85 90 95

Lys Glu Ile Gln Leu Leu Arg Arg Leu Arg His Lys Asn Val Ile Gln
100 105 110

Leu Val Asp Val Leu Tyr Asn Glu Glu Lys Gln Lys Met Tyr Met Val
115 120 125

Met Glu Tyr Cys Val Cys Gly Met Gln Glu Met Leu Asp Ser Val Pro
130 135 140

Glu Lys Arg Phe Pro Val Cys Gln Ala His Gly Tyr Phe Cys Gln Leu
145 150 155 160

Ile Asp Gly Leu Glu Tyr Leu His Ser Gln Gly Ile Val His Lys Asp
165 170 175

Ile Lys Pro Gly Asn Leu Leu Leu Thr Thr Gly Gly Thr Leu Lys Ile
180 185 190

Ser Ala Leu Gly Val Ala Glu Ala Leu His Pro Phe Ala Ala Asp Asp
195 200 205

Thr Cys Arg Thr Ser Gln Gly Ser Pro Ala Phe Gln Pro Pro Glu Ile
210 215 220

Ala Asn Gly Leu Asp Thr Phe Ser Gly Phe Lys Val Asp Ile Trp Ser
225 230 235 240

Ala Gly Val Thr Leu Tyr Asn Ile Thr Thr Gly Leu Tyr Pro Phe Glu
245 250 255

Gly Asp Asn Ile Tyr Lys Leu Phe Glu Asn Ile Gly Lys Gly Ser Tyr
260 265 270

Ala Ile Pro Gly Asp Cys Gly Pro Pro Leu Ser Asp Leu Leu Lys Gly

275 280 285

Met Leu Glu Tyr Glu Pro Ala Lys Arg Phe Ser Ile Arg Gln Ile Arg
290 295 300

Gln His Ser Trp Phe Arg Lys Lys His Pro Pro Ala Glu Ala Pro Val
305 310 315 320

Pro Ile Pro Pro Ser Pro Asp Thr Lys Asp Arg Trp Arg Ser Met Thr
325 330 335

Val Val Pro Tyr Leu Glu Asp Leu His Gly Ala Asp Glu Asp Glu Asp
340 345 350

Leu Phe Asp Ile Glu Asp Asp Ile Ile Tyr Thr Gln Asp Phe Thr Val
355 360 365

Pro Gly Gln Val Pro Glu Glu Glu Ala Ser His Asn Gly Gln Arg Arg
370 375 380

Gly Leu Pro Lys Ala Val Cys Met Asn Gly Thr Glu Ala Ala Gln Leu
385 390 395 400

Ser Thr Lys Ser Arg Ala Glu Gly Arg Ala Pro Asn Pro Ala Arg Lys
405 410 415

Ala Cys Ser Ala Ser Ser Lys Ile Arg Arg Leu Ser Ala Cys Lys Gln
420 425 430

Gln

<210> 17
<211> 3633
<212> DNA
<213> Homo sapiens

<220>
<221> CDS
<222> (1)..(3633)

<400> 17

```
atg cga ccc tcc ggg acg gcc ggg gca gcg ctc ctg gcg ctg ctg gct      48
Met Arg Pro Ser Gly Thr Ala Gly Ala Ala Leu Leu Ala Leu Leu Ala
1               5                   10                  15

gcg ctc tgc ccg gcg agt cgg gct ctg gag gaa aag aaa gtt tgc caa      96
Ala Leu Cys Pro Ala Ser Arg Ala Leu Glu Glu Lys Lys Val Cys Gln
            20                  25                  30

ggc acg agt aac aag ctc acg cag ttg ggc act ttt gaa gat cat ttt     144
Gly Thr Ser Asn Lys Leu Thr Gln Leu Gly Thr Phe Glu Asp His Phe
        35                  40                  45

ctc agc ctc cag agg atg ttc aat aac tgt gag gtg gtc ctt ggg aat     192
Leu Ser Leu Gln Arg Met Phe Asn Asn Cys Glu Val Val Leu Gly Asn
    50                  55                  60

ttg gaa att acc tat gtg cag agg aat tat gat ctt tcc ttc tta aag     240
Leu Glu Ile Thr Tyr Val Gln Arg Asn Tyr Asp Leu Ser Phe Leu Lys
65                  70                  75                  80

acc atc cag gag gtg gct ggt tat gtc ctc att gcc ctc aac aca gtg     288
Thr Ile Gln Glu Val Ala Gly Tyr Val Leu Ile Ala Leu Asn Thr Val
```

85 90 95

gag cga att cct ttg gaa aac ctg cag atc atc aga gga aat atg tac 336
Glu Arg Ile Pro Leu Glu Asn Leu Gln Ile Ile Arg Gly Asn Met Tyr
100 105 110

tac gaa aat tcc tat gcc tta gca gtc tta tct aac tat gat gca aat 384
Tyr Glu Asn Ser Tyr Ala Leu Ala Val Leu Ser Asn Tyr Asp Ala Asn
115 120 125

aaa acc gga ctg aag gag ctg ccc atg aga aat tta cag gaa atc ctg 432
Lys Thr Gly Leu Lys Glu Leu Pro Met Arg Asn Leu Gln Glu Ile Leu
130 135 140

cat ggc gcc gtg cgg ttc agc aac aac cct gcc ctg tgc aac gtg gag 480
His Gly Ala Val Arg Phe Ser Asn Asn Pro Ala Leu Cys Asn Val Glu
145 150 155 160

agc atc cag tgg cgg gac ata gtc agc agt gac ttt ctc agc aac atg 528
Ser Ile Gln Trp Arg Asp Ile Val Ser Ser Asp Phe Leu Ser Asn Met
165 170 175

tcg atg gac ttc cag aac cac ctg ggc agc tgc caa aag tgt gat cca 576
Ser Met Asp Phe Gln Asn His Leu Gly Ser Cys Gln Lys Cys Asp Pro
180 185 190

agc tgt ccc aat ggg agc tgc tgg ggt gca gga gag gag aac tgc cag 624
Ser Cys Pro Asn Gly Ser Cys Trp Gly Ala Gly Glu Glu Asn Cys Gln
195 200 205

aaa ctg acc aaa atc atc tgt gcc cag cag tgc tcc ggg cgc tgc cgt 672
Lys Leu Thr Lys Ile Ile Cys Ala Gln Gln Cys Ser Gly Arg Cys Arg
210 215 220

ggc aag tcc ccc agt gac tgc tgc cac aac cag tgt gct gca ggc tgc 720
Gly Lys Ser Pro Ser Asp Cys Cys His Asn Gln Cys Ala Ala Gly Cys
225 230 235 240

aca ggc ccc cgg gag agc gac tgc ctg gtc tgc cgc aaa ttc cga gac 768
Thr Gly Pro Arg Glu Ser Asp Cys Leu Val Cys Arg Lys Phe Arg Asp
245 250 255

gaa gcc acg tgc aag gac acc tgc ccc cca ctc atg ctc tac aac ccc 816
Glu Ala Thr Cys Lys Asp Thr Cys Pro Pro Leu Met Leu Tyr Asn Pro
260 265 270

acc acg tac cag atg gat gtg aac ccc gag ggc aaa tac agc ttt ggt 864
Thr Thr Tyr Gln Met Asp Val Asn Pro Glu Gly Lys Tyr Ser Phe Gly
275 280 285

gcc acc tgc gtg aag aag tgt ccc cgt aat tat gtg gtg aca gat cac 912
Ala Thr Cys Val Lys Lys Cys Pro Arg Asn Tyr Val Val Thr Asp His
290 295 300

ggc tcg tgc gtc cga gcc tgt ggg gcc gac agc tat gag atg gag gaa 960
Gly Ser Cys Val Arg Ala Cys Gly Ala Asp Ser Tyr Glu Met Glu Glu
305 310 315 320

gac ggc gtc cgc aag tgt aag aag tgc gaa ggg cct tgc cgc aaa gtg 1008
Asp Gly Val Arg Lys Cys Lys Lys Cys Glu Gly Pro Cys Arg Lys Val
325 330 335

tgt aac gga ata ggt att ggt gaa ttt aaa gac tca ctc tcc ata aat 1056
Cys Asn Gly Ile Gly Ile Gly Glu Phe Lys Asp Ser Leu Ser Ile Asn
340 345 350

gct acg aat att aaa cac ttc aaa aac tgc acc tcc atc agt ggc gat 1104
Ala Thr Asn Ile Lys His Phe Lys Asn Cys Thr Ser Ile Ser Gly Asp
355 360 365

ctc cac atc ctg ccg gtg gca ttt agg ggt gac tcc ttc aca cat act 1152
Leu His Ile Leu Pro Val Ala Phe Arg Gly Asp Ser Phe Thr His Thr
370 375 380

cct cct ctg gat cca cag gaa ctg gat att ctg aaa acc gta aag gaa 1200
Pro Pro Leu Asp Pro Gln Glu Leu Asp Ile Leu Lys Thr Val Lys Glu

385 390 395 400

atc aca ggg ttt ttg ctg att cag gct tgg cct gaa aac agg acg gac 1248
Ile Thr Gly Phe Leu Leu Ile Gln Ala Trp Pro Glu Asn Arg Thr Asp
405 410 415

ctc cat gcc ttt gag aac cta gaa atc ata cgc ggc agg acc aag caa 1296
Leu His Ala Phe Glu Asn Leu Glu Ile Ile Arg Gly Arg Thr Lys Gln
420 425 430

cat ggt cag ttt tct ctt gca gtc gtc agc ctg aac ata aca tcc ttg 1344
His Gly Gln Phe Ser Leu Ala Val Val Ser Leu Asn Ile Thr Ser Leu
435 440 445

gga tta cgc tcc ctc aag gag ata agt gat gga gat gtg ata att tca 1392
Gly Leu Arg Ser Leu Lys Glu Ile Ser Asp Gly Asp Val Ile Ile Ser
450 455 460

gga aac aaa aat ttg tgc tat gca aat aca ata aac tgg aaa aaa ctg 1440
Gly Asn Lys Asn Leu Cys Tyr Ala Asn Thr Ile Asn Trp Lys Lys Leu
465 470 475 480

ttt ggg acc tcc ggt cag aaa acc aaa att ata agc aac aga ggt gaa 1488
Phe Gly Thr Ser Gly Gln Lys Thr Lys Ile Ile Ser Asn Arg Gly Glu
485 490 495

aac agc tgc aag gcc aca ggc cag gtc tgc cat gcc ttg tgc tcc ccc 1536
Asn Ser Cys Lys Ala Thr Gly Gln Val Cys His Ala Leu Cys Ser Pro
500 505 510

gag ggc tgc tgg ggc ccg gag ccc agg gac tgc gtc tct tgc cgg aat 1584
Glu Gly Cys Trp Gly Pro Glu Pro Arg Asp Cys Val Ser Cys Arg Asn
515 520 525

gtc agc cga ggc agg gaa tgc gtg gac aag tgc aac ctt ctg gag ggt 1632
Val Ser Arg Gly Arg Glu Cys Val Asp Lys Cys Asn Leu Leu Glu Gly
530 535 540

gag cca agg gag ttt gtg gag aac tct gag tgc ata cag tgc cac cca 1680
Glu Pro Arg Glu Phe Val Glu Asn Ser Glu Cys Ile Gln Cys His Pro
545 550 555 560

gag tgc ctg cct cag gcc atg aac atc acc tgc aca gga cgg gga cca 1728
Glu Cys Leu Pro Gln Ala Met Asn Ile Thr Cys Thr Gly Arg Gly Pro
565 570 575

gac aac tgt atc cag tgt gcc cac tac att gac ggc ccc cac tgc gtc 1776
Asp Asn Cys Ile Gln Cys Ala His Tyr Ile Asp Gly Pro His Cys Val
580 585 590

aag acc tgc ccg gca gga gtc atg gga gaa aac aac acc ctg gtc tgg 1824
Lys Thr Cys Pro Ala Gly Val Met Gly Glu Asn Asn Thr Leu Val Trp
595 600 605

aag tac gca gac gcc ggc cat gtg tgc cac ctg tgc cat cca aac tgc 1872
Lys Tyr Ala Asp Ala Gly His Val Cys His Leu Cys His Pro Asn Cys
610 615 620

acc tac gga tgc act ggg cca ggt ctt gaa ggc tgt cca acg aat ggg 1920
Thr Tyr Gly Cys Thr Gly Pro Gly Leu Glu Gly Cys Pro Thr Asn Gly
625 630 635 640

cct aag atc ccg tcc atc gcc act ggg atg gtg ggg gcc ctc ctc ttg 1968
Pro Lys Ile Pro Ser Ile Ala Thr Gly Met Val Gly Ala Leu Leu Leu
645 650 655

ctg ctg gtg gtg gcc ctg ggg atc ggc ctc ttc atg cga agg cgc cac 2016
Leu Leu Val Val Ala Leu Gly Ile Gly Leu Phe Met Arg Arg Arg His
660 665 670

atc gtt cgg aag cgc acg ctg cgg agg ctg ctg cag gag agg gag ctt 2064
Ile Val Arg Lys Arg Thr Leu Arg Arg Leu Leu Gln Glu Arg Glu Leu
675 680 685

gtg gag cct ctt aca ccc agt gga gaa gct ccc aac caa gct ctc ttg 2112
Val Glu Pro Leu Thr Pro Ser Gly Glu Ala Pro Asn Gln Ala Leu Leu

690 695 700

agg atc ttg aag gaa act gaa ttc aaa aag atc aaa gtg ctg ggc tcc 2160
Arg Ile Leu Lys Glu Thr Glu Phe Lys Lys Ile Lys Val Leu Gly Ser
705 710 715 720

ggt gcg ttc ggc acg gtg tat aag gga ctc tgg atc cca gaa ggt gag 2208
Gly Ala Phe Gly Thr Val Tyr Lys Gly Leu Trp Ile Pro Glu Gly Glu
725 730 735

aaa gtt aaa att ccc gtc gct atc aag gaa tta aga gaa gca aca tct 2256
Lys Val Lys Ile Pro Val Ala Ile Lys Glu Leu Arg Glu Ala Thr Ser
740 745 750

ccg aaa gcc aac aag gaa atc ctc gat gaa gcc tac gtg atg gcc agc 2304
Pro Lys Ala Asn Lys Glu Ile Leu Asp Glu Ala Tyr Val Met Ala Ser
755 760 765

gtg gac aac ccc cac gtg tgc cgc ctg ctg ggc atc tgc ctc acc tcc 2352
Val Asp Asn Pro His Val Cys Arg Leu Leu Gly Ile Cys Leu Thr Ser
770 775 780

acc gtg cag ctc atc atg cag ctc atg ccc ttc ggc tgc ctc ctg gac 2400
Thr Val Gln Leu Ile Met Gln Leu Met Pro Phe Gly Cys Leu Leu Asp
785 790 795 800

tat gtc cgg gaa cac aaa gac aat att ggc tcc cag tac ctg ctc aac 2448
Tyr Val Arg Glu His Lys Asp Asn Ile Gly Ser Gln Tyr Leu Leu Asn
805 810 815

tgg tgt gtg cag atc gca aag ggc atg aac tac ttg gag gac cgt cgc 2496
Trp Cys Val Gln Ile Ala Lys Gly Met Asn Tyr Leu Glu Asp Arg Arg
820 825 830

ttg gtg cac cgc gac ctg gca gcc agg aac gta ctg gtg aaa aca ccg 2544
Leu Val His Arg Asp Leu Ala Ala Arg Asn Val Leu Val Lys Thr Pro
835 840 845

cag cat gtc aag atc aca gat ttt ggg cgg gcc aaa ctg ctg ggt gcg 2592
Gln His Val Lys Ile Thr Asp Phe Gly Arg Ala Lys Leu Leu Gly Ala
850 855 860

gaa gag aaa gaa tac cat gca gaa gga ggc aaa gtg cct atc aag tgg 2640
Glu Glu Lys Glu Tyr His Ala Glu Gly Gly Lys Val Pro Ile Lys Trp
865 870 875 880

atg gca ttg gaa tca att tta cac aga atc tat acc cac cag agt gat 2688
Met Ala Leu Glu Ser Ile Leu His Arg Ile Tyr Thr His Gln Ser Asp
885 890 895

gtc tgg agc tac ggg gtg acc gtt tgg gag ttg atg acc ttt gga tcc 2736
Val Trp Ser Tyr Gly Val Thr Val Trp Glu Leu Met Thr Phe Gly Ser
900 905 910

aag cca tat gac gga atc cct gcc agc gag atc tcc tcc atc ctg gag 2784
Lys Pro Tyr Asp Gly Ile Pro Ala Ser Glu Ile Ser Ser Ile Leu Glu
915 920 925

aaa gga gaa cgc ctc cct cag cca ccc ata tgt acc atc gat gtc tac 2832
Lys Gly Glu Arg Leu Pro Gln Pro Pro Ile Cys Thr Ile Asp Val Tyr
930 935 940

atg atc atg gtc aag tgc tgg atg ata gac gca gat agt cgc cca aag 2880
Met Ile Met Val Lys Cys Trp Met Ile Asp Ala Asp Ser Arg Pro Lys
945 950 955 960

ttc cgt gag ttg atc atc gaa ttc tcc aaa atg gcc cga gac ccc cag 2928
Phe Arg Glu Leu Ile Ile Glu Phe Ser Lys Met Ala Arg Asp Pro Gln
965 970 975

cgc tac ctt gtc att cag ggg gat gaa aga atg cat ttg cca agt cct 2976
Arg Tyr Leu Val Ile Gln Gly Asp Glu Arg Met His Leu Pro Ser Pro
980 985 990

aca gac tcc aac ttc tac cgt gcc ctg atg gat gaa gaa gac atg gac 3024
Thr Asp Ser Asn Phe Tyr Arg Ala Leu Met Asp Glu Glu Asp Met Asp

995 1000 1005

gac gtg gtg gat gcc gac gag tac ctc atc cca cag cag ggc ttc 3069
Asp Val Val Asp Ala Asp Glu Tyr Leu Ile Pro Gln Gln Gly Phe
1010 1015 1020

ttc agc agc ccc tcc acg tca cgg act ccc ctc ctg agc tct ctg 3114
Phe Ser Ser Pro Ser Thr Ser Arg Thr Pro Leu Leu Ser Ser Leu
1025 1030 1035

agt gca acc agc aac aat tcc acc gtg gct tgc att gat aga aat 3159
Ser Ala Thr Ser Asn Asn Ser Thr Val Ala Cys Ile Asp Arg Asn
1040 1045 1050

ggg ctg caa agc tgt ccc atc aag gaa gac agc ttc ttg cag cga 3204
Gly Leu Gln Ser Cys Pro Ile Lys Glu Asp Ser Phe Leu Gln Arg
1055 1060 1065

tac agc tca gac ccc aca ggc gcc ttg act gag gac agc ata gac 3249
Tyr Ser Ser Asp Pro Thr Gly Ala Leu Thr Glu Asp Ser Ile Asp
1070 1075 1080

gac acc ttc ctc cca gtg cct gaa tac ata aac cag tcc gtt ccc 3294
Asp Thr Phe Leu Pro Val Pro Glu Tyr Ile Asn Gln Ser Val Pro
1085 1090 1095

aaa agg ccc gct ggc tct gtg cag aat cct gtc tat cac aat cag 3339
Lys Arg Pro Ala Gly Ser Val Gln Asn Pro Val Tyr His Asn Gln
1100 1105 1110

cct ctg aac ccc gcg ccc agc aga gac cca cac tac cag gac ccc 3384
Pro Leu Asn Pro Ala Pro Ser Arg Asp Pro His Tyr Gln Asp Pro
1115 1120 1125

cac agc act gca gtg ggc aac ccc gag tat ctc aac act gtc cag 3429
His Ser Thr Ala Val Gly Asn Pro Glu Tyr Leu Asn Thr Val Gln
1130 1135 1140

ccc acc tgt gtc aac agc aca ttc gac agc cct gcc cac tgg gcc 3474
Pro Thr Cys Val Asn Ser Thr Phe Asp Ser Pro Ala His Trp Ala
1145 1150 1155

cag aaa ggc agc cac caa att agc ctg gac aac cct gac tac cag 3519
Gln Lys Gly Ser His Gln Ile Ser Leu Asp Asn Pro Asp Tyr Gln
1160 1165 1170

cag gac ttc ttt ccc aag gaa gcc aag cca aat ggc atc ttt aag 3564
Gln Asp Phe Phe Pro Lys Glu Ala Lys Pro Asn Gly Ile Phe Lys
1175 1180 1185

ggc tcc aca gct gaa aat gca gaa tac cta agg gtc gcg cca caa 3609
Gly Ser Thr Ala Glu Asn Ala Glu Tyr Leu Arg Val Ala Pro Gln
1190 1195 1200

agc agt gaa ttt att gga gca tga 3633
Ser Ser Glu Phe Ile Gly Ala
1205 1210

<210> 18
<211> 1210
<212> PRT
<213> Homo sapiens

<400> 18

Met Arg Pro Ser Gly Thr Ala Gly Ala Ala Leu Leu Ala Leu Leu Ala
1 5 10 15

Ala Leu Cys Pro Ala Ser Arg Ala Leu Glu Glu Lys Lys Val Cys Gln
20 25 30

Gly Thr Ser Asn Lys Leu Thr Gln Leu Gly Thr Phe Glu Asp His Phe
35 40 45

Leu Ser Leu Gln Arg Met Phe Asn Asn Cys Glu Val Val Leu Gly Asn
50 55 60

Leu Glu Ile Thr Tyr Val Gln Arg Asn Tyr Asp Leu Ser Phe Leu Lys
65 70 75 80

Thr Ile Gln Glu Val Ala Gly Tyr Val Leu Ile Ala Leu Asn Thr Val
85 90 95

Glu Arg Ile Pro Leu Glu Asn Leu Gln Ile Ile Arg Gly Asn Met Tyr
100 105 110

Tyr Glu Asn Ser Tyr Ala Leu Ala Val Leu Ser Asn Tyr Asp Ala Asn
115 120 125

Lys Thr Gly Leu Lys Glu Leu Pro Met Arg Asn Leu Gln Glu Ile Leu
130 135 140

His Gly Ala Val Arg Phe Ser Asn Asn Pro Ala Leu Cys Asn Val Glu
145 150 155 160

Ser Ile Gln Trp Arg Asp Ile Val Ser Ser Asp Phe Leu Ser Asn Met
165 170 175

Ser Met Asp Phe Gln Asn His Leu Gly Ser Cys Gln Lys Cys Asp Pro
180 185 190

Ser Cys Pro Asn Gly Ser Cys Trp Gly Ala Gly Glu Glu Asn Cys Gln
195 200 205

Lys Leu Thr Lys Ile Ile Cys Ala Gln Gln Cys Ser Gly Arg Cys Arg
210 215 220

Gly Lys Ser Pro Ser Asp Cys Cys His Asn Gln Cys Ala Ala Gly Cys
225 230 235 240

Thr Gly Pro Arg Glu Ser Asp Cys Leu Val Cys Arg Lys Phe Arg Asp
245 250 255

Glu Ala Thr Cys Lys Asp Thr Cys Pro Pro Leu Met Leu Tyr Asn Pro
260 265 270

Thr Thr Tyr Gln Met Asp Val Asn Pro Glu Gly Lys Tyr Ser Phe Gly
275 280 285

Ala Thr Cys Val Lys Lys Cys Pro Arg Asn Tyr Val Val Thr Asp His
290 295 300

Gly Ser Cys Val Arg Ala Cys Gly Ala Asp Ser Tyr Glu Met Glu Glu
305 310 315 320

Asp Gly Val Arg Lys Cys Lys Lys Cys Glu Gly Pro Cys Arg Lys Val
325 330 335

Cys Asn Gly Ile Gly Ile Gly Glu Phe Lys Asp Ser Leu Ser Ile Asn
340 345 350

Ala Thr Asn Ile Lys His Phe Lys Asn Cys Thr Ser Ile Ser Gly Asp
355 360 365

Leu His Ile Leu Pro Val Ala Phe Arg Gly Asp Ser Phe Thr His Thr
370 375 380

Pro Pro Leu Asp Pro Gln Glu Leu Asp Ile Leu Lys Thr Val Lys Glu
385 390 395 400

Ile Thr Gly Phe Leu Leu Ile Gln Ala Trp Pro Glu Asn Arg Thr Asp
405 410 415

Leu His Ala Phe Glu Asn Leu Glu Ile Ile Arg Gly Arg Thr Lys Gln
420 425 430

His Gly Gln Phe Ser Leu Ala Val Val Ser Leu Asn Ile Thr Ser Leu
435 440 445

Gly Leu Arg Ser Leu Lys Glu Ile Ser Asp Gly Asp Val Ile Ile Ser
450 455 460

Gly Asn Lys Asn Leu Cys Tyr Ala Asn Thr Ile Asn Trp Lys Lys Leu
465 470 475 480

Phe Gly Thr Ser Gly Gln Lys Thr Lys Ile Ile Ser Asn Arg Gly Glu
485 490 495

Asn Ser Cys Lys Ala Thr Gly Gln Val Cys His Ala Leu Cys Ser Pro
500 505 510

Glu Gly Cys Trp Gly Pro Glu Pro Arg Asp Cys Val Ser Cys Arg Asn
515 520 525

Val Ser Arg Gly Arg Glu Cys Val Asp Lys Cys Asn Leu Leu Glu Gly
530 535 540

Glu Pro Arg Glu Phe Val Glu Asn Ser Glu Cys Ile Gln Cys His Pro
545 550 555 560

Glu Cys Leu Pro Gln Ala Met Asn Ile Thr Cys Thr Gly Arg Gly Pro
565 570 575

Asp Asn Cys Ile Gln Cys Ala His Tyr Ile Asp Gly Pro His Cys Val
580 585 590

Lys Thr Cys Pro Ala Gly Val Met Gly Glu Asn Asn Thr Leu Val Trp
595 600 605

Lys Tyr Ala Asp Ala Gly His Val Cys His Leu Cys His Pro Asn Cys
610 615 620

Thr Tyr Gly Cys Thr Gly Pro Gly Leu Glu Gly Cys Pro Thr Asn Gly
625 630 635 640

Pro Lys Ile Pro Ser Ile Ala Thr Gly Met Val Gly Ala Leu Leu Leu
645 650 655

Leu Leu Val Val Ala Leu Gly Ile Gly Leu Phe Met Arg Arg Arg His
660 665 670

Ile Val Arg Lys Arg Thr Leu Arg Arg Leu Leu Gln Glu Arg Glu Leu
675 680 685

Val Glu Pro Leu Thr Pro Ser Gly Glu Ala Pro Asn Gln Ala Leu Leu
690 695 700

Arg Ile Leu Lys Glu Thr Glu Phe Lys Lys Ile Lys Val Leu Gly Ser
705 710 715 720

Gly Ala Phe Gly Thr Val Tyr Lys Gly Leu Trp Ile Pro Glu Gly Glu
725 730 735

Lys Val Lys Ile Pro Val Ala Ile Lys Glu Leu Arg Glu Ala Thr Ser
740 745 750

Pro Lys Ala Asn Lys Glu Ile Leu Asp Glu Ala Tyr Val Met Ala Ser
755 760 765

Val Asp Asn Pro His Val Cys Arg Leu Leu Gly Ile Cys Leu Thr Ser
770 775 780

Thr Val Gln Leu Ile Met Gln Leu Met Pro Phe Gly Cys Leu Leu Asp
785 790 795 800

Tyr Val Arg Glu His Lys Asp Asn Ile Gly Ser Gln Tyr Leu Leu Asn
805 810 815

Trp Cys Val Gln Ile Ala Lys Gly Met Asn Tyr Leu Glu Asp Arg Arg
820 825 830

Leu Val His Arg Asp Leu Ala Ala Arg Asn Val Leu Val Lys Thr Pro
835 840 845

Gln His Val Lys Ile Thr Asp Phe Gly Arg Ala Lys Leu Leu Gly Ala
850 855 860

Glu Glu Lys Glu Tyr His Ala Glu Gly Gly Lys Val Pro Ile Lys Trp
865 870 875 880

Met Ala Leu Glu Ser Ile Leu His Arg Ile Tyr Thr His Gln Ser Asp
885 890 895

Val Trp Ser Tyr Gly Val Thr Val Trp Glu Leu Met Thr Phe Gly Ser
900 905 910

Lys Pro Tyr Asp Gly Ile Pro Ala Ser Glu Ile Ser Ser Ile Leu Glu
915 920 925

Lys Gly Glu Arg Leu Pro Gln Pro Pro Ile Cys Thr Ile Asp Val Tyr
930 935 940

Met Ile Met Val Lys Cys Trp Met Ile Asp Ala Asp Ser Arg Pro Lys
945 950 955 960

Phe Arg Glu Leu Ile Ile Glu Phe Ser Lys Met Ala Arg Asp Pro Gln
965 970 975

Arg Tyr Leu Val Ile Gln Gly Asp Glu Arg Met His Leu Pro Ser Pro
980 985 990

Thr Asp Ser Asn Phe Tyr Arg Ala Leu Met Asp Glu Glu Asp Met Asp
995 1000 1005

Asp Val Val Asp Ala Asp Glu Tyr Leu Ile Pro Gln Gln Gly Phe
1010 1015 1020

Phe Ser Ser Pro Ser Thr Ser Arg Thr Pro Leu Leu Ser Ser Leu
1025 1030 1035

Ser Ala Thr Ser Asn Asn Ser Thr Val Ala Cys Ile Asp Arg Asn
1040 1045 1050

Gly Leu Gln Ser Cys Pro Ile Lys Glu Asp Ser Phe Leu Gln Arg
1055 1060 1065

Tyr Ser Ser Asp Pro Thr Gly Ala Leu Thr Glu Asp Ser Ile Asp
1070 1075 1080

Asp Thr Phe Leu Pro Val Pro Glu Tyr Ile Asn Gln Ser Val Pro
1085 1090 1095

Lys Arg Pro Ala Gly Ser Val Gln Asn Pro Val Tyr His Asn Gln
1100 1105 1110

Pro Leu Asn Pro Ala Pro Ser Arg Asp Pro His Tyr Gln Asp Pro
1115 1120 1125

His Ser Thr Ala Val Gly Asn Pro Glu Tyr Leu Asn Thr Val Gln
1130 1135 1140

Pro Thr Cys Val Asn Ser Thr Phe Asp Ser Pro Ala His Trp Ala
1145 1150 1155

Gln Lys Gly Ser His Gln Ile Ser Leu Asp Asn Pro Asp Tyr Gln
1160 1165 1170

Gln Asp Phe Phe Pro Lys Glu Ala Lys Pro Asn Gly Ile Phe Lys
1175 1180 1185

Gly Ser Thr Ala Glu Asn Ala Glu Tyr Leu Arg Val Ala Pro Gln
1190 1195 1200

Ser Ser Glu Phe Ile Gly Ala
1205 1210

<210> 19
<211> 1761
<212> DNA
<213> Homo sapiens

<220>
<221> CDS
<222> (1)..(1761)

<400> 19

atg ttg tac ctg gaa aac aat gcc cag act caa ttt agt gag cca cag 48
Met Leu Tyr Leu Glu Asn Asn Ala Gln Thr Gln Phe Ser Glu Pro Gln
1 5 10 15

tac acg aac ctg ggg ctc ctg aac agc atg gac cag cag att cag aac 96
Tyr Thr Asn Leu Gly Leu Leu Asn Ser Met Asp Gln Gln Ile Gln Asn
20 25 30

ggc tcc tcg tcc acc agt ccc tat aac aca gac cac gcg cag aac agc 144
Gly Ser Ser Ser Thr Ser Pro Tyr Asn Thr Asp His Ala Gln Asn Ser
35 40 45

gtc acg gcg ccc tcg ccc tac gca cag ccc agc tcc acc ttc gat gct 192
Val Thr Ala Pro Ser Pro Tyr Ala Gln Pro Ser Ser Thr Phe Asp Ala
50 55 60

ctc tct cca tca ccc gcc atc ccc tcc aac acc gac tac cca ggc ccg 240
Leu Ser Pro Ser Pro Ala Ile Pro Ser Asn Thr Asp Tyr Pro Gly Pro
65 70 75 80

cac agt ttc gac gtg tcc ttc cag cag tcg agc acc gcc aag tcg gcc 288
His Ser Phe Asp Val Ser Phe Gln Gln Ser Ser Thr Ala Lys Ser Ala
85 90 95

acc tgg acg tat tcc act gaa ctg aag aaa ctc tac tgc caa att gca 336
Thr Trp Thr Tyr Ser Thr Glu Leu Lys Lys Leu Tyr Cys Gln Ile Ala
100 105 110

aag aca tgc ccc atc cag atc aag gtg atg acc cca cct cct cag gga 384
Lys Thr Cys Pro Ile Gln Ile Lys Val Met Thr Pro Pro Pro Gln Gly
115 120 125

gct gtt atc cgc gcc atg cct gtc tac aaa aaa gct gag cac gtc acg 432

Ala Val Ile Arg Ala Met Pro Val Tyr Lys Lys Ala Glu His Val Thr
130 135 140

gag gtg gtg aag cgg tgc ccc aac cat gag ctg agc cgt gaa ttc aac 480
Glu Val Val Lys Arg Cys Pro Asn His Glu Leu Ser Arg Glu Phe Asn
145 150 155 160

gag gga cag att gcc cct cct agt cat ttg att cga gta gag ggg aac 528
Glu Gly Gln Ile Ala Pro Pro Ser His Leu Ile Arg Val Glu Gly Asn
165 170 175

agc cat gcc cag tat gta gaa gat ccc atc aca gga aga cag agt gtg 576
Ser His Ala Gln Tyr Val Glu Asp Pro Ile Thr Gly Arg Gln Ser Val
180 185 190

ctg gta cct tat gag cca ccc cag gtt ggc act gaa ttc acg aca gtc 624
Leu Val Pro Tyr Glu Pro Pro Gln Val Gly Thr Glu Phe Thr Thr Val
195 200 205

ttg tac aat ttc atg tgt aac agc agt tgt gtt gga ggg atg aac cgc 672
Leu Tyr Asn Phe Met Cys Asn Ser Ser Cys Val Gly Gly Met Asn Arg
210 215 220

cgt cca att tta atc att gtt act ctg gaa acc aga gat ggg caa gtc 720
Arg Pro Ile Leu Ile Ile Val Thr Leu Glu Thr Arg Asp Gly Gln Val
225 230 235 240

ctg ggc cga cgc tgc ttt gag gcc cgg atc tgt gct tgc cca gga aga 768
Leu Gly Arg Arg Cys Phe Glu Ala Arg Ile Cys Ala Cys Pro Gly Arg
245 250 255

gac agg aag gcg gat gaa gat agc atc aga aag cag caa gtt tcg gac 816
Asp Arg Lys Ala Asp Glu Asp Ser Ile Arg Lys Gln Gln Val Ser Asp
260 265 270

agt aca aag aac ggt gat ggt acg aag cgc ccg ttt cgt cag aac aca 864
Ser Thr Lys Asn Gly Asp Gly Thr Lys Arg Pro Phe Arg Gln Asn Thr
275 280 285

cat ggt atc cag atg aca tcc atc aag aaa cga aga tcc cca gat gat 912
His Gly Ile Gln Met Thr Ser Ile Lys Lys Arg Arg Ser Pro Asp Asp
290 295 300

gaa ctg tta tac tta cca gtg agg ggc cgt gag act tat gaa atg ctg 960
Glu Leu Leu Tyr Leu Pro Val Arg Gly Arg Glu Thr Tyr Glu Met Leu
305 310 315 320

ttg aag atc aaa gag tcc ctg gaa ctc atg cag tac ctt cct cag cac 1008
Leu Lys Ile Lys Glu Ser Leu Glu Leu Met Gln Tyr Leu Pro Gln His
325 330 335

aca att gaa acg tac agg caa cag caa cag cag cag cac cag cac tta 1056
Thr Ile Glu Thr Tyr Arg Gln Gln Gln Gln Gln Gln His Gln His Leu
340 345 350

ctt cag aaa cag acc tca ata cag tct cca tct tca tat ggt aac agc 1104
Leu Gln Lys Gln Thr Ser Ile Gln Ser Pro Ser Ser Tyr Gly Asn Ser
355 360 365

tcc cca cct ctg aac aaa atg aac agc atg aac aag ctg cct tct gtg 1152
Ser Pro Pro Leu Asn Lys Met Asn Ser Met Asn Lys Leu Pro Ser Val
370 375 380

agc cag ctt atc aac cct cag cag cgc aac gcc ctc act cct aca acc 1200
Ser Gln Leu Ile Asn Pro Gln Gln Arg Asn Ala Leu Thr Pro Thr Thr
385 390 395 400

att cct gat ggc atg gga gcc aac att ccc atg atg ggc acc cac atg 1248
Ile Pro Asp Gly Met Gly Ala Asn Ile Pro Met Met Gly Thr His Met
405 410 415

cca atg gct gga gac atg aat gga ctc agc ccc acc cag gca ctc cct 1296
Pro Met Ala Gly Asp Met Asn Gly Leu Ser Pro Thr Gln Ala Leu Pro
420 425 430

ccc cca ctc tcc atg cca tcc acc tcc cac tgc aca ccc cca cct ccg 1344

Pro Pro Leu Ser Met Pro Ser Thr Ser His Cys Thr Pro Pro Pro Pro
435 440 445

tat ccc aca gat tgc agc att gtc agt ttc tta gcg agg ttg ggc tgt 1392
Tyr Pro Thr Asp Cys Ser Ile Val Ser Phe Leu Ala Arg Leu Gly Cys
450 455 460

tca tca tgt ctg gac tat ttc acg acc cag ggg ctg acc acc atc tat 1440
Ser Ser Cys Leu Asp Tyr Phe Thr Thr Gln Gly Leu Thr Thr Ile Tyr
465 470 475 480

cag att gag cat tac tcc atg gat gat ctg gca agt ctg aaa atc cct 1488
Gln Ile Glu His Tyr Ser Met Asp Asp Leu Ala Ser Leu Lys Ile Pro
485 490 495

gag caa ttt cga cat gcg atc tgg aag ggc atc ctg gac cac cgg cag 1536
Glu Gln Phe Arg His Ala Ile Trp Lys Gly Ile Leu Asp His Arg Gln
500 505 510

ctc cac gaa ttc tcc tcc cct tct cat ctc ctg cgg acc cca agc agt 1584
Leu His Glu Phe Ser Ser Pro Ser His Leu Leu Arg Thr Pro Ser Ser
515 520 525

gcc tct aca gtc agt gtg ggc tcc agt gag acc cgg ggt gag cgt gtt 1632
Ala Ser Thr Val Ser Val Gly Ser Ser Glu Thr Arg Gly Glu Arg Val
530 535 540

att gat gct gtg cga ttc acc ctc cgc cag acc atc tct ttc cca ccc 1680
Ile Asp Ala Val Arg Phe Thr Leu Arg Gln Thr Ile Ser Phe Pro Pro
545 550 555 560

cga gat gag tgg aat gac ttc aac ttt gac atg gat gct cgc cgc aat 1728
Arg Asp Glu Trp Asn Asp Phe Asn Phe Asp Met Asp Ala Arg Arg Asn
565 570 575

aag caa cag cgc atc aaa gag gag ggg gag tga 1761
Lys Gln Gln Arg Ile Lys Glu Glu Gly Glu
580 585

<210> 20
<211> 586
<212> PRT
<213> Homo sapiens

<400> 20

Met Leu Tyr Leu Glu Asn Asn Ala Gln Thr Gln Phe Ser Glu Pro Gln
1 5 10 15

Tyr Thr Asn Leu Gly Leu Leu Asn Ser Met Asp Gln Gln Ile Gln Asn
20 25 30

Gly Ser Ser Ser Thr Ser Pro Tyr Asn Thr Asp His Ala Gln Asn Ser
35 40 45

Val Thr Ala Pro Ser Pro Tyr Ala Gln Pro Ser Ser Thr Phe Asp Ala
50 55 60

Leu Ser Pro Ser Pro Ala Ile Pro Ser Asn Thr Asp Tyr Pro Gly Pro
65 70 75 80

His Ser Phe Asp Val Ser Phe Gln Gln Ser Ser Thr Ala Lys Ser Ala
85 90 95

Thr Trp Thr Tyr Ser Thr Glu Leu Lys Lys Leu Tyr Cys Gln Ile Ala
100 105 110

Lys Thr Cys Pro Ile Gln Ile Lys Val Met Thr Pro Pro Pro Gln Gly

115 120 125

Ala Val Ile Arg Ala Met Pro Val Tyr Lys Lys Ala Glu His Val Thr
130 135 140

Glu Val Val Lys Arg Cys Pro Asn His Glu Leu Ser Arg Glu Phe Asn
145 150 155 160

Glu Gly Gln Ile Ala Pro Pro Ser His Leu Ile Arg Val Glu Gly Asn
165 170 175

Ser His Ala Gln Tyr Val Glu Asp Pro Ile Thr Gly Arg Gln Ser Val
180 185 190

Leu Val Pro Tyr Glu Pro Pro Gln Val Gly Thr Glu Phe Thr Thr Val
195 200 205

Leu Tyr Asn Phe Met Cys Asn Ser Ser Cys Val Gly Gly Met Asn Arg
210 215 220

Arg Pro Ile Leu Ile Ile Val Thr Leu Glu Thr Arg Asp Gly Gln Val
225 230 235 240

Leu Gly Arg Arg Cys Phe Glu Ala Arg Ile Cys Ala Cys Pro Gly Arg
245 250 255

Asp Arg Lys Ala Asp Glu Asp Ser Ile Arg Lys Gln Gln Val Ser Asp
260 265 270

Ser Thr Lys Asn Gly Asp Gly Thr Lys Arg Pro Phe Arg Gln Asn Thr
275 280 285

His Gly Ile Gln Met Thr Ser Ile Lys Lys Arg Arg Ser Pro Asp Asp
290 295 300

Glu Leu Leu Tyr Leu Pro Val Arg Gly Arg Glu Thr Tyr Glu Met Leu
305 310 315 320

Leu Lys Ile Lys Glu Ser Leu Glu Leu Met Gln Tyr Leu Pro Gln His
325 330 335

Thr Ile Glu Thr Tyr Arg Gln Gln Gln Gln Gln Gln His Gln His Leu
340 345 350

Leu Gln Lys Gln Thr Ser Ile Gln Ser Pro Ser Ser Tyr Gly Asn Ser
355 360 365

Ser Pro Pro Leu Asn Lys Met Asn Ser Met Asn Lys Leu Pro Ser Val
370 375 380

Ser Gln Leu Ile Asn Pro Gln Gln Arg Asn Ala Leu Thr Pro Thr Thr
385 390 395 400

Ile Pro Asp Gly Met Gly Ala Asn Ile Pro Met Met Gly Thr His Met
405 410 415

Pro Met Ala Gly Asp Met Asn Gly Leu Ser Pro Thr Gln Ala Leu Pro

420 425 430

Pro Pro Leu Ser Met Pro Ser Thr Ser His Cys Thr Pro Pro Pro Pro
435 440 445

Tyr Pro Thr Asp Cys Ser Ile Val Ser Phe Leu Ala Arg Leu Gly Cys
450 455 460

Ser Ser Cys Leu Asp Tyr Phe Thr Thr Gln Gly Leu Thr Thr Ile Tyr
465 470 475 480

Gln Ile Glu His Tyr Ser Met Asp Asp Leu Ala Ser Leu Lys Ile Pro
485 490 495

Glu Gln Phe Arg His Ala Ile Trp Lys Gly Ile Leu Asp His Arg Gln
500 505 510

Leu His Glu Phe Ser Ser Pro Ser His Leu Leu Arg Thr Pro Ser Ser
515 520 525

Ala Ser Thr Val Ser Val Gly Ser Ser Glu Thr Arg Gly Glu Arg Val
530 535 540

Ile Asp Ala Val Arg Phe Thr Leu Arg Gln Thr Ile Ser Phe Pro Pro
545 550 555 560

Arg Asp Glu Trp Asn Asp Phe Asn Phe Asp Met Asp Ala Arg Arg Asn
565 570 575

Lys Gln Gln Arg Ile Lys Glu Glu Gly Glu
580 585

<210> 21
<211> 3399
<212> DNA
<213> Homo sapiens

<220>
<221> CDS
<222> (1)..(3399)

<400> 21

atg ccg cgc gct ccc cgc tgc cga gcc gtg cgc tcc ctg ctg cgc agc 48
Met Pro Arg Ala Pro Arg Cys Arg Ala Val Arg Ser Leu Leu Arg Ser
1 5 10 15

cac tac cgc gag gtg ctg ccg ctg gcc acg ttc gtg cgg cgc ctg ggg 96
His Tyr Arg Glu Val Leu Pro Leu Ala Thr Phe Val Arg Arg Leu Gly
20 25 30

ccc cag ggc tgg cgg ctg gtg cag cgc ggg gac ccg gcg gct ttc cgc 144
Pro Gln Gly Trp Arg Leu Val Gln Arg Gly Asp Pro Ala Ala Phe Arg
35 40 45

gcg ctg gtg gcc cag tgc ctg gtg tgc gtg ccc tgg gac gca cgg ccg 192
Ala Leu Val Ala Gln Cys Leu Val Cys Val Pro Trp Asp Ala Arg Pro
50 55 60

ccc ccc gcc gcc ccc tcc ttc cgc cag gtg tcc tgc ctg aag gag ctg 240
Pro Pro Ala Ala Pro Ser Phe Arg Gln Val Ser Cys Leu Lys Glu Leu
65 70 75 80

gtg gcc cga gtg ctg cag agg ctg tgc gag cgc ggc gcg aag aac gtg 288
Val Ala Arg Val Leu Gln Arg Leu Cys Glu Arg Gly Ala Lys Asn Val

85 90 95

ctg gcc ttc ggc ttc gcg ctg ctg gac ggg gcc cgc ggg ggc ccc ccc 336
Leu Ala Phe Gly Phe Ala Leu Leu Asp Gly Ala Arg Gly Gly Pro Pro
100 105 110

gag gcc ttc acc acc agc gtg cgc agc tac ctg ccc aac acg gtg acc 384
Glu Ala Phe Thr Thr Ser Val Arg Ser Tyr Leu Pro Asn Thr Val Thr
115 120 125

gac gca ctg cgg ggg agc ggg gcg tgg ggg ctg ctg ttg cgc cgc gtg 432
Asp Ala Leu Arg Gly Ser Gly Ala Trp Gly Leu Leu Leu Arg Arg Val
130 135 140

ggc gac gac gtg ctg gtt cac ctg ctg gca cgc tgc gcg ctc ttt gtg 480
Gly Asp Asp Val Leu Val His Leu Leu Ala Arg Cys Ala Leu Phe Val
145 150 155 160

ctg gtg gct ccc agc tgc gcc tac cag gtg tgc ggg ccg ccg ctg tac 528
Leu Val Ala Pro Ser Cys Ala Tyr Gln Val Cys Gly Pro Pro Leu Tyr
165 170 175

cag ctc ggc gct gcc act cag gcc cgg ccc ccg cca cac gct agt gga 576
Gln Leu Gly Ala Ala Thr Gln Ala Arg Pro Pro Pro His Ala Ser Gly
180 185 190

ccc cga agg cgt ctg gga tgc gaa cgg gcc tgg aac cat agc gtc agg 624
Pro Arg Arg Arg Leu Gly Cys Glu Arg Ala Trp Asn His Ser Val Arg
195 200 205

gag gcc ggg gtc ccc ctg ggc ctg cca gcc ccg ggt gcg agg agg cgc 672
Glu Ala Gly Val Pro Leu Gly Leu Pro Ala Pro Gly Ala Arg Arg Arg
210 215 220

ggg ggc agt gcc agc cga agt ctg ccg ttg ccc aag agg ccc agg cgt 720
Gly Gly Ser Ala Ser Arg Ser Leu Pro Leu Pro Lys Arg Pro Arg Arg
225 230 235 240

ggc gct gcc cct gag ccg gag cgg acg ccc gtt ggg cag ggg tcc tgg 768
Gly Ala Ala Pro Glu Pro Glu Arg Thr Pro Val Gly Gln Gly Ser Trp
245 250 255

gcc cac ccg ggc agg acg cgt gga ccg agt gac cgt ggt ttc tgt gtg 816
Ala His Pro Gly Arg Thr Arg Gly Pro Ser Asp Arg Gly Phe Cys Val
260 265 270

gtg tca cct gcc aga ccc gcc gaa gaa gcc acc tct ttg gag ggt gcg 864
Val Ser Pro Ala Arg Pro Ala Glu Glu Ala Thr Ser Leu Glu Gly Ala
275 280 285

ctc tct ggc acg cgc cac tcc cac cca tcc gtg ggc cgc cag cac cac 912
Leu Ser Gly Thr Arg His Ser His Pro Ser Val Gly Arg Gln His His
290 295 300

gcg ggc ccc cca tcc aca tcg cgg cca cca cgt ccc tgg gac acg cct 960
Ala Gly Pro Pro Ser Thr Ser Arg Pro Pro Arg Pro Trp Asp Thr Pro
305 310 315 320

tgt ccc ccg gtg tac gcc gag acc aag cac ttc ctc tac tcc tca ggc 1008
Cys Pro Pro Val Tyr Ala Glu Thr Lys His Phe Leu Tyr Ser Ser Gly
325 330 335

gac aag gag cag ctg cgg ccc tcc ttc cta ctc agc tct ctg agg ccc 1056
Asp Lys Glu Gln Leu Arg Pro Ser Phe Leu Leu Ser Ser Leu Arg Pro
340 345 350

agc ctg act ggc gct cgg agg ctc gtg gag acc atc ttt ctg ggt tcc 1104
Ser Leu Thr Gly Ala Arg Arg Leu Val Glu Thr Ile Phe Leu Gly Ser
355 360 365

agg ccc tgg atg cca ggg act ccc cgc agg ttg ccc cgc ctg ccc cag 1152
Arg Pro Trp Met Pro Gly Thr Pro Arg Arg Leu Pro Arg Leu Pro Gln
370 375 380

cgc tac tgg caa atg cgg ccc ctg ttt ctg gag ctg ctt ggg aac cac 1200
Arg Tyr Trp Gln Met Arg Pro Leu Phe Leu Glu Leu Leu Gly Asn His

385 390 395 400

gcg cag tgc ccc tac ggg gtg ctc ctc aag acg cac tgc ccg ctg cga 1248
Ala Gln Cys Pro Tyr Gly Val Leu Leu Lys Thr His Cys Pro Leu Arg
405 410 415

gct gcg gtc acc cca gca gcc ggt gtc tgt gcc cgg gag aag ccc cag 1296
Ala Ala Val Thr Pro Ala Ala Gly Val Cys Ala Arg Glu Lys Pro Gln
420 425 430

ggc tct gtg gcg gcc ccc gag gag gag gac aca gac ccc cgt cgc ctg 1344
Gly Ser Val Ala Ala Pro Glu Glu Glu Asp Thr Asp Pro Arg Arg Leu
435 440 445

gtg cag ctg ctc cgc cag cac agc agc ccc tgg cag gtg tac ggc ttc 1392
Val Gln Leu Leu Arg Gln His Ser Ser Pro Trp Gln Val Tyr Gly Phe
450 455 460

gtg cgg gcc tgc ctg cgc cgg ctg gtg ccc cca ggc ctc tgg ggc tcc 1440
Val Arg Ala Cys Leu Arg Arg Leu Val Pro Pro Gly Leu Trp Gly Ser
465 470 475 480

agg cac aac gaa cgc cgc ttc ctc agg aac acc aag aag ttc atc tcc 1488
Arg His Asn Glu Arg Arg Phe Leu Arg Asn Thr Lys Lys Phe Ile Ser
485 490 495

ctg ggg aag cat gcc aag ctc tcg ctg cag gag ctg acg tgg aag atg 1536
Leu Gly Lys His Ala Lys Leu Ser Leu Gln Glu Leu Thr Trp Lys Met
500 505 510

agc gtg cgg ggc tgc gct tgg ctg cgc agg agc cca ggg gtt ggc tgt 1584
Ser Val Arg Gly Cys Ala Trp Leu Arg Arg Ser Pro Gly Val Gly Cys
515 520 525

gtt ccg gcc gca gag cac cgt ctg cgt gag gag atc ctg gcc aag ttc 1632
Val Pro Ala Ala Glu His Arg Leu Arg Glu Glu Ile Leu Ala Lys Phe
530 535 540

ctg cac tgg ctg atg agt gtg tac gtc gtc gag ctg ctc agg tct ttc 1680
Leu His Trp Leu Met Ser Val Tyr Val Val Glu Leu Leu Arg Ser Phe
545 550 555 560

ttt tat gtc acg gag acc acg ttt caa aag aac agg ctc ttt ttc tac 1728
Phe Tyr Val Thr Glu Thr Thr Phe Gln Lys Asn Arg Leu Phe Phe Tyr
565 570 575

cgg aag agt gtc tgg agc aag ttg caa agc att gga atc aga cag cac 1776
Arg Lys Ser Val Trp Ser Lys Leu Gln Ser Ile Gly Ile Arg Gln His
580 585 590

ttg aag agg gtg cag ctg cgg gag ctg tcg gaa gca gag gtc agg cag 1824
Leu Lys Arg Val Gln Leu Arg Glu Leu Ser Glu Ala Glu Val Arg Gln
595 600 605

cat cgg gaa gcc agg ccc gcc ctg ctg acg tcc aga ctc cgc ttc atc 1872
His Arg Glu Ala Arg Pro Ala Leu Leu Thr Ser Arg Leu Arg Phe Ile
610 615 620

ccc aag cct gac ggg ctg cgg ccg att gtg aac atg gac tac gtc gtg 1920
Pro Lys Pro Asp Gly Leu Arg Pro Ile Val Asn Met Asp Tyr Val Val
625 630 635 640

gga gcc aga acg ttc cgc aga gaa aag agg gcc gag cgt ctc acc tcg 1968
Gly Ala Arg Thr Phe Arg Arg Glu Lys Arg Ala Glu Arg Leu Thr Ser
645 650 655

agg gtg aag gca ctg ttc agc gtg ctc aac tac gag cgg gcg cgg cgc 2016
Arg Val Lys Ala Leu Phe Ser Val Leu Asn Tyr Glu Arg Ala Arg Arg
660 665 670

ccc ggc ctc ctg ggc gcc tct gtg ctg ggc ctg gac gat atc cac agg 2064
Pro Gly Leu Leu Gly Ala Ser Val Leu Gly Leu Asp Asp Ile His Arg
675 680 685

gcc tgg cgc acc ttc gtg ctg cgt gtg cgg gcc cag gac ccg ccg cct 2112
Ala Trp Arg Thr Phe Val Leu Arg Val Arg Ala Gln Asp Pro Pro Pro

690 695 700

gag ctg tac ttt gtc aag gtg gat gtg acg ggc gcg tac gac acc atc 2160
Glu Leu Tyr Phe Val Lys Val Asp Val Thr Gly Ala Tyr Asp Thr Ile
705 710 715 720

ccc cag gac agg ctc acg gag gtc atc gcc agc atc atc aaa ccc cag 2208
Pro Gln Asp Arg Leu Thr Glu Val Ile Ala Ser Ile Ile Lys Pro Gln
725 730 735

aac acg tac tgc gtg cgt cgg tat gcc gtg gtc cag aag gcc gcc cat 2256
Asn Thr Tyr Cys Val Arg Arg Tyr Ala Val Val Gln Lys Ala Ala His
740 745 750

ggg cac gtc cgc aag gcc ttc aag agc cac gtc tct acc ttg aca gac 2304
Gly His Val Arg Lys Ala Phe Lys Ser His Val Ser Thr Leu Thr Asp
755 760 765

ctc cag ccg tac atg cga cag ttc gtg gct cac ctg cag gag acc agc 2352
Leu Gln Pro Tyr Met Arg Gln Phe Val Ala His Leu Gln Glu Thr Ser
770 775 780

ccg ctg agg gat gcc gtc gtc atc gag cag agc tcc tcc ctg aat gag 2400
Pro Leu Arg Asp Ala Val Val Ile Glu Gln Ser Ser Ser Leu Asn Glu
785 790 795 800

gcc agc agt ggc ctc ttc gac gtc ttc cta cgc ttc atg tgc cac cac 2448
Ala Ser Ser Gly Leu Phe Asp Val Phe Leu Arg Phe Met Cys His His
805 810 815

gcc gtg cgc atc agg ggc aag tcc tac gtc cag tgc cag ggg atc ccg 2496
Ala Val Arg Ile Arg Gly Lys Ser Tyr Val Gln Cys Gln Gly Ile Pro
820 825 830

cag ggc tcc atc ctc tcc acg ctg ctc tgc agc ctg tgc tac ggc gac 2544
Gln Gly Ser Ile Leu Ser Thr Leu Leu Cys Ser Leu Cys Tyr Gly Asp
835 840 845

```
atg gag aac aag ctg ttt gcg ggg att cgg cgg gac ggg ctg ctc ctg      2592
Met Glu Asn Lys Leu Phe Ala Gly Ile Arg Arg Asp Gly Leu Leu Leu
    850                 855                 860

cgt ttg gtg gat gat ttc ttg ttg gtg aca cct cac ctc acc cac gcg      2640
Arg Leu Val Asp Asp Phe Leu Leu Val Thr Pro His Leu Thr His Ala
865                 870                 875                 880

aaa acc ttc ctc agg acc ctg gtc cga ggt gtc cct gag tat ggc tgc      2688
Lys Thr Phe Leu Arg Thr Leu Val Arg Gly Val Pro Glu Tyr Gly Cys
                885                 890                 895

gtg gtg aac ttg cgg aag aca gtg gtg aac ttc cct gta gaa gac gag      2736
Val Val Asn Leu Arg Lys Thr Val Val Asn Phe Pro Val Glu Asp Glu
            900                 905                 910

gcc ctg ggt ggc acg gct ttt gtt cag atg ccg gcc cac ggc cta ttc      2784
Ala Leu Gly Gly Thr Ala Phe Val Gln Met Pro Ala His Gly Leu Phe
        915                 920                 925

ccc tgg tgc ggc ctg ctg ctg gat acc cgg acc ctg gag gtg cag agc      2832
Pro Trp Cys Gly Leu Leu Leu Asp Thr Arg Thr Leu Glu Val Gln Ser
    930                 935                 940

gac tac tcc agc tat gcc cgg acc tcc atc aga gcc agt ctc acc ttc      2880
Asp Tyr Ser Ser Tyr Ala Arg Thr Ser Ile Arg Ala Ser Leu Thr Phe
945                 950                 955                 960

aac cgc ggc ttc aag gct ggg agg aac atg cgt cgc aaa ctc ttt ggg      2928
Asn Arg Gly Phe Lys Ala Gly Arg Asn Met Arg Arg Lys Leu Phe Gly
                965                 970                 975

gtc ttg cgg ctg aag tgt cac agc ctg ttt ctg gat ttg cag gtg aac      2976
Val Leu Arg Leu Lys Cys His Ser Leu Phe Leu Asp Leu Gln Val Asn
            980                 985                 990

agc ctc cag acg gtg tgc acc aac  atc tac aag atc ctc  ctg ctg cag    3024
Ser Leu Gln Thr Val Cys Thr Asn  Ile Tyr Lys Ile Leu  Leu Leu Gln
```

995 1000 1005

gcg tac agg ttt cac gca tgt gtg ctg cag ctc cca ttt cat cag 3069
Ala Tyr Arg Phe His Ala Cys Val Leu Gln Leu Pro Phe His Gln
1010 1015 1020

caa gtt tgg aag aac ccc aca ttt ttc ctg cgc gtc atc tct gac 3114
Gln Val Trp Lys Asn Pro Thr Phe Phe Leu Arg Val Ile Ser Asp
1025 1030 1035

acg gcc tcc ctc tgc tac tcc atc ctg aaa gcc aag aac gca ggg 3159
Thr Ala Ser Leu Cys Tyr Ser Ile Leu Lys Ala Lys Asn Ala Gly
1040 1045 1050

atg tcg ctg ggg gcc aag ggc gcc gcc ggc cct ctg ccc tcc gag 3204
Met Ser Leu Gly Ala Lys Gly Ala Ala Gly Pro Leu Pro Ser Glu
1055 1060 1065

gcc gtg cag tgg ctg tgc cac caa gca ttc ctg ctc aag ctg act 3249
Ala Val Gln Trp Leu Cys His Gln Ala Phe Leu Leu Lys Leu Thr
1070 1075 1080

cga cac cgt gtc acc tac gtg cca ctc ctg ggg tca ctc agg aca 3294
Arg His Arg Val Thr Tyr Val Pro Leu Leu Gly Ser Leu Arg Thr
1085 1090 1095

gcc cag acg cag ctg agt cgg aag ctc ccg ggg acg acg ctg act 3339
Ala Gln Thr Gln Leu Ser Arg Lys Leu Pro Gly Thr Thr Leu Thr
1100 1105 1110

gcc ctg gag gcc gca gcc aac ccg gca ctg ccc tca gac ttc aag 3384
Ala Leu Glu Ala Ala Ala Asn Pro Ala Leu Pro Ser Asp Phe Lys
1115 1120 1125

acc atc ctg gac taa 3399
Thr Ile Leu Asp
1130

<210> 22

<211> 1132
<212> PRT
<213> Homo sapiens

<400> 22

Met Pro Arg Ala Pro Arg Cys Arg Ala Val Arg Ser Leu Leu Arg Ser
1 5 10 15

His Tyr Arg Glu Val Leu Pro Leu Ala Thr Phe Val Arg Arg Leu Gly
20 25 30

Pro Gln Gly Trp Arg Leu Val Gln Arg Gly Asp Pro Ala Ala Phe Arg
35 40 45

Ala Leu Val Ala Gln Cys Leu Val Cys Val Pro Trp Asp Ala Arg Pro
50 55 60

Pro Pro Ala Ala Pro Ser Phe Arg Gln Val Ser Cys Leu Lys Glu Leu
65 70 75 80

Val Ala Arg Val Leu Gln Arg Leu Cys Glu Arg Gly Ala Lys Asn Val
85 90 95

Leu Ala Phe Gly Phe Ala Leu Leu Asp Gly Ala Arg Gly Gly Pro Pro
100 105 110

Glu Ala Phe Thr Thr Ser Val Arg Ser Tyr Leu Pro Asn Thr Val Thr
115 120 125

Asp Ala Leu Arg Gly Ser Gly Ala Trp Gly Leu Leu Leu Arg Arg Val
130 135 140

Gly Asp Asp Val Leu Val His Leu Leu Ala Arg Cys Ala Leu Phe Val
145 150 155 160

Leu Val Ala Pro Ser Cys Ala Tyr Gln Val Cys Gly Pro Pro Leu Tyr
165 170 175

Gln Leu Gly Ala Ala Thr Gln Ala Arg Pro Pro Pro His Ala Ser Gly
180 185 190

Pro Arg Arg Arg Leu Gly Cys Glu Arg Ala Trp Asn His Ser Val Arg
195 200 205

Glu Ala Gly Val Pro Leu Gly Leu Pro Ala Pro Gly Ala Arg Arg Arg
210 215 220

Gly Gly Ser Ala Ser Arg Ser Leu Pro Leu Pro Lys Arg Pro Arg Arg
225 230 235 240

Gly Ala Ala Pro Glu Pro Glu Arg Thr Pro Val Gly Gln Gly Ser Trp
245 250 255

Ala His Pro Gly Arg Thr Arg Gly Pro Ser Asp Arg Gly Phe Cys Val
260 265 270

Val Ser Pro Ala Arg Pro Ala Glu Glu Ala Thr Ser Leu Glu Gly Ala
275 280 285

Leu Ser Gly Thr Arg His Ser His Pro Ser Val Gly Arg Gln His His
290 295 300

Ala Gly Pro Pro Ser Thr Ser Arg Pro Pro Arg Pro Trp Asp Thr Pro
305 310 315 320

Cys Pro Pro Val Tyr Ala Glu Thr Lys His Phe Leu Tyr Ser Ser Gly
325 330 335

Asp Lys Glu Gln Leu Arg Pro Ser Phe Leu Leu Ser Ser Leu Arg Pro
340 345 350

Ser Leu Thr Gly Ala Arg Arg Leu Val Glu Thr Ile Phe Leu Gly Ser
355 360 365

Arg Pro Trp Met Pro Gly Thr Pro Arg Arg Leu Pro Arg Leu Pro Gln
370 375 380

Arg Tyr Trp Gln Met Arg Pro Leu Phe Leu Glu Leu Leu Gly Asn His
385 390 395 400

Ala Gln Cys Pro Tyr Gly Val Leu Leu Lys Thr His Cys Pro Leu Arg
405 410 415

Ala Ala Val Thr Pro Ala Ala Gly Val Cys Ala Arg Glu Lys Pro Gln
420 425 430

Gly Ser Val Ala Ala Pro Glu Glu Glu Asp Thr Asp Pro Arg Arg Leu
435 440 445

Val Gln Leu Leu Arg Gln His Ser Ser Pro Trp Gln Val Tyr Gly Phe
450 455 460

Val Arg Ala Cys Leu Arg Arg Leu Val Pro Pro Gly Leu Trp Gly Ser
465 470 475 480

Arg His Asn Glu Arg Arg Phe Leu Arg Asn Thr Lys Lys Phe Ile Ser
485 490 495

Leu Gly Lys His Ala Lys Leu Ser Leu Gln Glu Leu Thr Trp Lys Met
500 505 510

Ser Val Arg Gly Cys Ala Trp Leu Arg Arg Ser Pro Gly Val Gly Cys
515 520 525

Val Pro Ala Ala Glu His Arg Leu Arg Glu Glu Ile Leu Ala Lys Phe
530 535 540

Leu His Trp Leu Met Ser Val Tyr Val Val Glu Leu Leu Arg Ser Phe
545 550 555 560

Phe Tyr Val Thr Glu Thr Thr Phe Gln Lys Asn Arg Leu Phe Phe Tyr
565 570 575

Arg Lys Ser Val Trp Ser Lys Leu Gln Ser Ile Gly Ile Arg Gln His
580 585 590

Leu Lys Arg Val Gln Leu Arg Glu Leu Ser Glu Ala Glu Val Arg Gln
595 600 605

His Arg Glu Ala Arg Pro Ala Leu Leu Thr Ser Arg Leu Arg Phe Ile
610 615 620

Pro Lys Pro Asp Gly Leu Arg Pro Ile Val Asn Met Asp Tyr Val Val
625 630 635 640

Gly Ala Arg Thr Phe Arg Arg Glu Lys Arg Ala Glu Arg Leu Thr Ser
645 650 655

Arg Val Lys Ala Leu Phe Ser Val Leu Asn Tyr Glu Arg Ala Arg Arg
660 665 670

Pro Gly Leu Leu Gly Ala Ser Val Leu Gly Leu Asp Asp Ile His Arg
675 680 685

Ala Trp Arg Thr Phe Val Leu Arg Val Arg Ala Gln Asp Pro Pro Pro
690 695 700

Glu Leu Tyr Phe Val Lys Val Asp Val Thr Gly Ala Tyr Asp Thr Ile
705 710 715 720

Pro Gln Asp Arg Leu Thr Glu Val Ile Ala Ser Ile Ile Lys Pro Gln
725 730 735

Asn Thr Tyr Cys Val Arg Arg Tyr Ala Val Val Gln Lys Ala Ala His
740 745 750

Gly His Val Arg Lys Ala Phe Lys Ser His Val Ser Thr Leu Thr Asp
755 760 765

Leu Gln Pro Tyr Met Arg Gln Phe Val Ala His Leu Gln Glu Thr Ser
770 775 780

Pro Leu Arg Asp Ala Val Val Ile Glu Gln Ser Ser Ser Leu Asn Glu
785 790 795 800

Ala Ser Ser Gly Leu Phe Asp Val Phe Leu Arg Phe Met Cys His His
805 810 815

Ala Val Arg Ile Arg Gly Lys Ser Tyr Val Gln Cys Gln Gly Ile Pro
820 825 830

Gln Gly Ser Ile Leu Ser Thr Leu Leu Cys Ser Leu Cys Tyr Gly Asp
835 840 845

Met Glu Asn Lys Leu Phe Ala Gly Ile Arg Arg Asp Gly Leu Leu Leu
850 855 860

Arg Leu Val Asp Asp Phe Leu Leu Val Thr Pro His Leu Thr His Ala
865 870 875 880

Lys Thr Phe Leu Arg Thr Leu Val Arg Gly Val Pro Glu Tyr Gly Cys
885 890 895

Val Val Asn Leu Arg Lys Thr Val Val Asn Phe Pro Val Glu Asp Glu
900 905 910

Ala Leu Gly Gly Thr Ala Phe Val Gln Met Pro Ala His Gly Leu Phe
915 920 925

Pro Trp Cys Gly Leu Leu Leu Asp Thr Arg Thr Leu Glu Val Gln Ser
930 935 940

Asp Tyr Ser Ser Tyr Ala Arg Thr Ser Ile Arg Ala Ser Leu Thr Phe
945 950 955 960

Asn Arg Gly Phe Lys Ala Gly Arg Asn Met Arg Arg Lys Leu Phe Gly
965 970 975

Val Leu Arg Leu Lys Cys His Ser Leu Phe Leu Asp Leu Gln Val Asn
980 985 990

Ser Leu Gln Thr Val Cys Thr Asn Ile Tyr Lys Ile Leu Leu Leu Gln
995 1000 1005

Ala Tyr Arg Phe His Ala Cys Val Leu Gln Leu Pro Phe His Gln
1010 1015 1020

Gln Val Trp Lys Asn Pro Thr Phe Phe Leu Arg Val Ile Ser Asp
1025 1030 1035

Thr Ala Ser Leu Cys Tyr Ser Ile Leu Lys Ala Lys Asn Ala Gly
1040 1045 1050

Met Ser Leu Gly Ala Lys Gly Ala Ala Gly Pro Leu Pro Ser Glu
1055 1060 1065

Ala Val Gln Trp Leu Cys His Gln Ala Phe Leu Leu Lys Leu Thr
1070 1075 1080

Arg His Arg Val Thr Tyr Val Pro Leu Leu Gly Ser Leu Arg Thr
1085 1090 1095

Ala Gln Thr Gln Leu Ser Arg Lys Leu Pro Gly Thr Thr Leu Thr
1100 1105 1110

Ala Leu Glu Ala Ala Ala Asn Pro Ala Leu Pro Ser Asp Phe Lys
1115 1120 1125

Thr Ile Leu Asp
1130

<210> 23
<211> 912
<212> DNA
<213> Homo sapiens

<220>
<221> CDS
<222> (1)..(912)

<400> 23

atg gct acc tct cga tat gag cca gtg gct gaa att ggt gtc ggt gcc 48
Met Ala Thr Ser Arg Tyr Glu Pro Val Ala Glu Ile Gly Val Gly Ala
1 5 10 15

tat ggg aca gtg tac aag gcc cgt gat ccc cac agt ggc cac ttt gtg 96
Tyr Gly Thr Val Tyr Lys Ala Arg Asp Pro His Ser Gly His Phe Val
20 25 30

gcc ctc aag agt gtg aga gtc ccc aat gga gga gga ggt gga gga ggc 144
Ala Leu Lys Ser Val Arg Val Pro Asn Gly Gly Gly Gly Gly Gly Gly
35 40 45

ctt ccc atc agc aca gtt cgt gag gtg gct tta ctg agg cga ctg gag 192
Leu Pro Ile Ser Thr Val Arg Glu Val Ala Leu Leu Arg Arg Leu Glu
50 55 60

gct ttt gag cat ccc aat gtt gtc cgg ctg atg gac gtc tgt gcc aca 240
Ala Phe Glu His Pro Asn Val Val Arg Leu Met Asp Val Cys Ala Thr
65 70 75 80

tcc cga act gac cgg gag atc aag gta acc ctg gtg ttt gag cat gta 288
Ser Arg Thr Asp Arg Glu Ile Lys Val Thr Leu Val Phe Glu His Val
85 90 95

gac cag gac cta agg aca tat ctg gac aag gca ccc cca cca ggc ttg 336
Asp Gln Asp Leu Arg Thr Tyr Leu Asp Lys Ala Pro Pro Pro Gly Leu
100 105 110

cca gcc gaa acg atc aag gat ctg atg cgc cag ttt cta aga ggc cta 384
Pro Ala Glu Thr Ile Lys Asp Leu Met Arg Gln Phe Leu Arg Gly Leu
115 120 125

gat ttc ctt cat gcc aat tgc atc gtt cac cga gat ctg aag cca gag 432
Asp Phe Leu His Ala Asn Cys Ile Val His Arg Asp Leu Lys Pro Glu
130 135 140

```
aac att ctg gtg aca agt ggt gga aca gtc aag ctg gct gac ttt ggc      480
Asn Ile Leu Val Thr Ser Gly Gly Thr Val Lys Leu Ala Asp Phe Gly
145                 150                 155                 160

ctg gcc aga atc tac agc tac cag atg gca ctt aca ccc gtg gtt gtt      528
Leu Ala Arg Ile Tyr Ser Tyr Gln Met Ala Leu Thr Pro Val Val Val
                165                 170                 175

aca ctc tgg tac cga gct ccc gaa gtt ctt ctg cag tcc aca tat gca      576
Thr Leu Trp Tyr Arg Ala Pro Glu Val Leu Leu Gln Ser Thr Tyr Ala
            180                 185                 190

aca cct gtg gac atg tgg agt gtt ggc tgt atc ttt gca gag atg ttt      624
Thr Pro Val Asp Met Trp Ser Val Gly Cys Ile Phe Ala Glu Met Phe
        195                 200                 205

cgt cga aag cct ctc ttc tgt gga aac tct gaa gcc gac cag ttg ggc      672
Arg Arg Lys Pro Leu Phe Cys Gly Asn Ser Glu Ala Asp Gln Leu Gly
    210                 215                 220

aaa atc ttt gac ctg att ggg ctg cct cca gag gat gac tgg cct cga      720
Lys Ile Phe Asp Leu Ile Gly Leu Pro Pro Glu Asp Asp Trp Pro Arg
225                 230                 235                 240

gat gta tcc ctg ccc cgt gga gcc ttt ccc ccc aga ggg ccc cgc cca      768
Asp Val Ser Leu Pro Arg Gly Ala Phe Pro Pro Arg Gly Pro Arg Pro
                245                 250                 255

gtg cag tcg gtg gta cct gag atg gag gag tcg gga gca cag ctg ctg      816
Val Gln Ser Val Val Pro Glu Met Glu Glu Ser Gly Ala Gln Leu Leu
            260                 265                 270

ctg gaa atg ctg act ttt aac cca cac aag cga atc tct gcc ttt cga      864
Leu Glu Met Leu Thr Phe Asn Pro His Lys Arg Ile Ser Ala Phe Arg
        275                 280                 285

gct ctg cag cac tct tat cta cat aag gat gaa ggt aat ccg gag tga      912
```

Ala Leu Gln His Ser Tyr Leu His Lys Asp Glu Gly Asn Pro Glu
290 295 300

<210> 24
<211> 303
<212> PRT
<213> Homo sapiens

<400> 24

Met Ala Thr Ser Arg Tyr Glu Pro Val Ala Glu Ile Gly Val Gly Ala
1 5 10 15

Tyr Gly Thr Val Tyr Lys Ala Arg Asp Pro His Ser Gly His Phe Val
20 25 30

Ala Leu Lys Ser Val Arg Val Pro Asn Gly Gly Gly Gly Gly Gly Gly
35 40 45

Leu Pro Ile Ser Thr Val Arg Glu Val Ala Leu Leu Arg Arg Leu Glu
50 55 60

Ala Phe Glu His Pro Asn Val Val Arg Leu Met Asp Val Cys Ala Thr
65 70 75 80

Ser Arg Thr Asp Arg Glu Ile Lys Val Thr Leu Val Phe Glu His Val
85 90 95

Asp Gln Asp Leu Arg Thr Tyr Leu Asp Lys Ala Pro Pro Pro Gly Leu
100 105 110

Pro Ala Glu Thr Ile Lys Asp Leu Met Arg Gln Phe Leu Arg Gly Leu
115 120 125

Asp Phe Leu His Ala Asn Cys Ile Val His Arg Asp Leu Lys Pro Glu
130 135 140

Asn Ile Leu Val Thr Ser Gly Gly Thr Val Lys Leu Ala Asp Phe Gly
145 150 155 160

Leu Ala Arg Ile Tyr Ser Tyr Gln Met Ala Leu Thr Pro Val Val Val
165 170 175

Thr Leu Trp Tyr Arg Ala Pro Glu Val Leu Leu Gln Ser Thr Tyr Ala
180 185 190

Thr Pro Val Asp Met Trp Ser Val Gly Cys Ile Phe Ala Glu Met Phe
195 200 205

Arg Arg Lys Pro Leu Phe Cys Gly Asn Ser Glu Ala Asp Gln Leu Gly
210 215 220

Lys Ile Phe Asp Leu Ile Gly Leu Pro Pro Glu Asp Asp Trp Pro Arg
225 230 235 240

Asp Val Ser Leu Pro Arg Gly Ala Phe Pro Pro Arg Gly Pro Arg Pro
245 250 255

Val Gln Ser Val Val Pro Glu Met Glu Glu Ser Gly Ala Gln Leu Leu

260 265 270

Leu Glu Met Leu Thr Phe Asn Pro His Lys Arg Ile Ser Ala Phe Arg
275 280 285

Ala Leu Gln His Ser Tyr Leu His Lys Asp Glu Gly Asn Pro Glu
290 295 300

<210> 25
<211> 312
<212> DNA
<213> Homo sapiens

<220>
<221> CDS
<222> (1).. (312)

<400> 25

atg tac cca tac gat gtt cca gat tac gct cca ggg agc act aag cga 48
Met Tyr Pro Tyr Asp Val Pro Asp Tyr Ala Pro Gly Ser Thr Lys Arg
1 5 10 15

gca ctg ccc aac aac acc agc tcc tct ccc cag cca aag aag aaa cca 96
Ala Leu Pro Asn Asn Thr Ser Ser Ser Pro Gln Pro Lys Lys Lys Pro
20 25 30

ctg gat gga gaa tat ttc acc ctt cag atc cgt ggg cgt gag cgc ttc 144
Leu Asp Gly Glu Tyr Phe Thr Leu Gln Ile Arg Gly Arg Glu Arg Phe
35 40 45

gag atg ttc cga gag ctg aat gag gcc ttg gaa ctc aag gat gcc cag 192
Glu Met Phe Arg Glu Leu Asn Glu Ala Leu Glu Leu Lys Asp Ala Gln
50 55 60

gct ggg aag gag cca ggg ggg agc agg gct cac tcc agc cac ctg aag 240
Ala Gly Lys Glu Pro Gly Gly Ser Arg Ala His Ser Ser His Leu Lys
65 70 75 80

tcc aaa aag ggt cag tct acc tcc cgc cat aaa aaa ctc atg ttc aag 288
Ser Lys Lys Gly Gln Ser Thr Ser Arg His Lys Lys Leu Met Phe Lys
85 90 95

aca gaa ggg cct gac tca gac tga 312
Thr Glu Gly Pro Asp Ser Asp
100

<210> 26
<211> 103
<212> PRT
<213> Homo sapiens

<400> 26

Met Tyr Pro Tyr Asp Val Pro Asp Tyr Ala Pro Gly Ser Thr Lys Arg
1 5 10 15

Ala Leu Pro Asn Asn Thr Ser Ser Ser Pro Gln Pro Lys Lys Lys Pro
20 25 30

Leu Asp Gly Glu Tyr Phe Thr Leu Gln Ile Arg Gly Arg Glu Arg Phe
35 40 45

Glu Met Phe Arg Glu Leu Asn Glu Ala Leu Glu Leu Lys Asp Ala Gln
50 55 60

Ala Gly Lys Glu Pro Gly Gly Ser Arg Ala His Ser Ser His Leu Lys
65 70 75 80

Ser Lys Lys Gly Gln Ser Thr Ser Arg His Lys Lys Leu Met Phe Lys
85 90 95

Thr Glu Gly Pro Asp Ser Asp
100

**Description**

TECHNICAL FIELD

The present invention relates to a method for producing tumor cells by gene transfer based on small airway epithelial cells.

BACKGROUND ART

Each year, more than one million people die as a result of lung cancer. Indeed, lung cancer can be regarded as the most common type of cancer in both men and women. Non-small cell lung cancer accounts for 80% of all types of lung cancer with associated adenocarcinoma. In most cases, diagnosis takes place at an advanced stage of the disease and, despite the progress that has been made in methods of early detection and treatment, a good prognosis is difficult to come by (Non-Patent Documents 1 and 2). Although molecular analysis of the state and progression of non-small cell lung cancer is expected to lead to improvements in methods of treatment and prevention, at present, effective methods of treatment and prevention have yet to be established. Identifying the minimal and most important changes associated with the tumorigenic transformation of cells is essential to finding the most suitable targets for initial detection and therapeutic intervention. In order to identify such changes, *in-vitro* cancer model systems obtained by the genetic manipulation of normal human cells are being developed. Because existing cell lines established from tumor tissue contain unknown genetic aberrations, they have not been suitable for research on tumorigenic transformation through genetic change. However, some of *in-vitro* cancer model systems are reported to be useful in directly elucidating the influence of specific genetic changes on tumorigenic transformation (see Non-Patent Documents 3 and 4).

A number of research groups are conducting studies on the immortalization and oncogenic transformation of human lung epithelial cells (Non-Patent Documents 5 to 7). In these studies, three types of genetic changes observed in most human non-small cell lung cancers, namely, telomerase reverse transcriptase gene introduction and inactivation of the pRB and p53 pathways, are being tested by genetic manipulation using retroviruses. The high expression of telomerase is observed in substantially all lung cancers (Non-Patent Document 8), p53 loss of function is observed in about 50% of non-small cell lung cancers, and an inability to express p 16 protein due to promoter region methylation or homozygous deletion is observed in about 70% of non-small cell lung cancer (Non-Patent Document 9).

However, in spite of the advances being made in such research, the literature to date is substantially devoid of reports on the successful artificial production of lung cancer cells which are pathologically very similar to lung cancer tissue isolated from actual lung cancer patients.

At present, cancer cell lines derived from cancer patients are being used in cancer cell research and in the development of cancer drugs. However, because substantially most cancer cell lines derived from patients have unspecified genetic damage, there exists some variability between cell lines in the state of the genetic aberrations. Accordingly, when such cells are used in research on specific molecules or signal pathways, the experimental results obtained cannot be applied to different cell lines. Moreover, because such cells have unspecified genetic damage, they are unsuitable for use in experiments to evaluate the contributions of individual genes to oncogenic transformation.

Non-Patent Document 1: Meuwissen, R., et al., Genes Dev. 19, 643-664 (2005)
Non-Patent Document 2: Herbst, R.S. et al., "Lung cancer," N. Engl. J. Med. 359, 1367-1380 (2008)
Non-Patent Document 3: Akagi, T., Trends Mol. Med. 10, 542-548 (2004)
Non-Patent Document 4: Zhao, J.J., et al., Trends Mol. Med. 10, 344-350 (2004)
Non-Patent Document 5: Lundberg, A.S., et al., Oncogen 21, 4577-4586 (2002)
Non-Patent Document 6: Ramirez, R.D., et al., Cancer Res. 64, 9027-9034 (2004)
Non-Patent Document 7: Sato, M., et al., Cancer Res. 66, 2116-2128 (2006)
Non-Patent Document 8: Sekido, Y. et al., Annu. Rev. Med. 54, 73-87 (2003)
Non-Patent Document 2: Herbst, R.S. et al., "Lung cancer," N. Engl. J. Med. 359, 1367-1380 (2008)

DISCLOSURE OF THE INVENTION

In light of the above, the development of cancer model cells having a defined genetic state has been awaited.

The inventors have successfully achieved the complete tumorigenic transformation of small airway epithelial cells by genetic manipulation. Because the tumorigenic transformed cells thus obtained have been transformed by the genetic manipulation of normal cells, the genetic state has been identified. The inventors, on closely observing these cells, have found that the cells exhibit the histological characteristics of differentiated or undifferentiated human lung cancer cells depending on the combination of genetic elements that have been introduced. In addition, the inventors have succeeded in producing cells having the nature of cancer stem cells for undifferentiated and differentiated human lung cancer.

Accordingly, the invention relates to the following.

[1] A method for producing a tumor cell by transferring a combination of cancer-associated gene(s) into immortalized primate small airway epithelial cells, wherein

- the immortalized primate small airway epithelial cells are produced by subjecting normal small airway epithelial cells to following treatments (1) to (3):

  (1) forced expression of telomere reverse transcriptase gene;
  (2) forced expression of cyclin-dependent kinase 4 gene; and
  (3) induction of p53 loss of function, and

- the combination of cancer-associated genes is:

  (a) c-Myc gene and v-Src gene,
  (b) c-Myc gene, KRAS mutated gene and BCL2 gene,
  (c) KRAS mutated gene and Cyclin D1 gene,
  (d) KRAS mutated gene, Cyclin D1 gene and TP63 gene,
  (e) KRAS mutated gene and PIK3CA mutated gene,
  (f) KRAS mutated gene and LKB1 mutated gene, or
  (g) EGFR mutated gene and Cyclin D1 gene.

[2] The method according to [1], wherein the immortalized primate small airway epithelial cells are human small airway epithelial cells.

[3] The method according [1] or [2], wherein the combination of cancer-associated genes is c-Myc gene and v-Src gene or c-Myc gene, KRAS mutated gene and BCL2 gene.

[4] The method according to [3], wherein the tumor cells are undifferentiated cancer cells.

[5] The method according to [4], wherein the tumor cells are cancer stem cells and wherein the combination of cancer-associated genes is c-Myc gene and v-Src gene.

[6] The method according to [2] wherein the combination of cancer-associated genes is c-Myc gene and v-Src gene, wherein the c-Myc gene is inductively expressed in the immortalized human small airway epithelial cells.

[7] The method according to [6], wherein the tumor cells are poorly differentiated lung cancer cells.

[8] The method according to [1] or [2], wherein the combination of cancer-associated genes is KRAS mutated gene and Cyclin D1 gene.

[9] The method according to [1] or [2], wherein the combination of cancer-associated genes is KRAS mutated gene, Cyclin D1 gene and TP63 gene; KRAS mutated gene and PIK3CA mutated gene; KRAS mutated gene and LKB1 mutated gene or EGFR mutated gene and Cyclin D1 gene.

[10] The method according to [8] or [9], wherein the tumor cells are differentiated lung cancer cells.

[11] The method according to [10], wherein the tumor cells are cancer stem cells of differentiated lung cancers.

[12] The method of [10], wherein the combination of cancer-associated genes is (a) KRAS mutated gene and Cyclin D1 gene, (b) EGFR mutated gene and Cyclin D1 gene or (c) KRAS mutated gene and PIK3CA mutated gene.

Because the tumor cells produced by the method of the invention are cells which have been tumorigenically transformed by genetic manipulation on normal cells, the genetic state thereof has been identified. Therefore, a model system which is highly useful for evaluating the contributions of individual genes in the development of lung cancer is provided. Of the tumor cells produced by the method of the invention, those cells produced by the transfer of only cancer-associated genes are cells which precisely express the properties of cancer cells, and are thus expected to be highly effective in such applications as the development of cancer drugs.

BRIEF DESCRIPTION OF THE DRAWINGS

FIG. 1A is a diagram illustrating the tumorigenicity of cells (referred to below as "4T53MS cells") produced by transferring the c-Myc and v-Src genes into HSAE/4T53 cells, FIG. 1B is a graph showing the tumor growth potential of 4T53MS cells, and FIG.1C shows images of the results from the hematoxylin-eosin (H&E) staining of 4T53MS cell-derived tumor tissue and the results from immunostaining the tumor tissue.

FIG. 2A is a table showing the results obtained, according to the number of cells implanted, in a study of the tumorigenicity of 4T53MS cells *in vivo,* and FIG. 2B is a diagram showing tumor formation seven weeks after ten 4T53MS cells were implanted in an NOD-SCID mouse.

FIG. 3A is a diagram showing, in an *in-vitro* experimental system, that c-Myc expression is induced by doxycycline (DOX) treatment in cells (indicated below as "4T53mS cells," wherein 'm' is a lower case letter) produced by transferring the inducible c-Myc gene and the v-Src gene into HSAE/4T53 cells, FIG. 3B is a schematic view depicting a method of producing a poorly differentiated lung cancer model. 4T53mS cells are implanted into nude mice and the mice continue to be administered with doxycycline (DOX). Once tumor formation has been observed, doxycycline administration is stopped. FIG. 3C is a diagram showing the results from immunostaining (cytokeratin staining) 4T53mS cell-derived tumor tissue.

FIG. 4 is a table showing the tumorigenicity of cell lines established by transferring one or more of various cancer-associated genes (M: c-Myc; R: $KRAS^{V12}$; B: BCL2; P: $PIK3CA^{H1047R}$; D: Cyclin-D1; L: $LKB1^{D194A}$; E: $EGFR^{T790M\ L858R}$) into HSAE/4T53 cells.

FIG. 5 presents diagrams showing the results obtained when the indicated genes (M: c-Myc; R: $KRAS^{V12}$; B: BCL2) were transferred into HSAE/4T53 cells to produce 4T53RM cells (transduced with $KRAS^{V12}$ and c-Myc) and 4T53RMB cells (transduced with $KRAS^{V12}$, c-Myc and BCL2), the cells thus produced were transplanted into nude mice, which were then subjected to H&E staining (top row), anti-cytokeratin staining (AE1/AE3: middle row), and anti-Vimentin staining (bottom row).

FIG. 6 presents diagrams showing the results obtained from the H&E staining (top row), anti-cytokeratin staining (AE1/AE3: middle row) and anti-Vimentin staining (bottom row) of sections of xenografts derived from 4T53RP cells (left column), 4T53RD cells (center column) and 4T53RL cells (right column). "4T53RP cells" are cells produced by transferring the $KRAS^{V12}$ gene and the PIK3CA mutated gene into HSAE/4T53 cells. "4T53RD cells" are cells produced by transferring the $KRAS^{V12}$ and Cyclin D1 genes into HSAE/4T53 cells. "4T53RL cells" are cells produced by transferring the $KRAS^{V12}$ gene and the LKB1 mutated gene into HSAE/4T53 cells.

FIG. 7 is a schematic diagram showing that various types of lung cancer models can be produced depending on the combination of genes transferred into 4T53 cells.

FIG. 8A shows the results from the anti-p63 staining (left panel), anti-TTF1 staining (center panel) and Alcian Blue staining (right panel) of sections of a xenograft obtained by subcutaneously implanting cell clones from a single 4T53RD cell, and FIG. 8 B is a schematic diagram of the airway epithelium in a mouse.

FIG. 9 is a table showing the results of *in vivo* tumorigenicity study of cell clones from a single 4T53RD cell, according to the number of cells implanted.

FIG. 10A and FIG. 10B show the results of H&E staining of sections of xenografts derived from 4T53RD cells and 4T53RD Δ Np63 cells respectively.

FIG. 11A, FIG. 11B, and FIG. 11C show the results of H&E staining, Alcian Blue staining, and anti-cytokeratin staining (AE1/AE3) sections of xenografts derived from 4T53ED cells respectively.

BEST MODE FOR CARRYING OUT THE INVENTION

The invention is described in detail below. The following embodiments are given to illustrate the invention, and are not to be construed as limiting the invention. The invention may be practiced in various forms without departing from the gist thereof.

1. Method for Producing Tumor Cells

This invention provides a method for producing tumor cells by transferring a combination of cancer-associated genes into immortalized primate small airway epithelial cells.

The method of the invention is characterized in that the immortalized primate small airway epithelial cells are produced by subjecting normal primate small airway epithelial cells to treatments (1) to (3) below:

(1) forced expression of telomere reverse transcriptase gene;
(2) forced expression of cyclin-dependent kinase 4 gene; and
(3) induction of p53 loss of function,
wherein the combination cancer-associated genes is:

(a) c-Myc gene and v-Src gene,
(b) c-Myc gene, KRAS mutated gene and BCL2 gene,
(c) KRAS mutated gene and Cyclin D1 gene,
(d) KRAS mutated gene, Cyclin D1 gene and TP63 gene,
(e) KRAS mutated gene and PIK3CA mutated gene,
(f) KRAS mutated gene and LKB1 mutated gene, or
(g) EGFR mutated gene and Cyclin D1 gene.

Cancer-Associated Gene

The "cancer-associated gene" used in the method of the invention refers to a gene which, when expressed within a normal cell, has the ability to tumorigenically or oncogenically transform the cell, or to a gene associated with control of the expression of a gene which, when expressed within a normal cell, has the ability to tumorigenically or oncogenically transform the cell. Examples of cancer-associated genes include the myelocytomatosis oncogene (c-Myc), the v-Src gene, the RAS gene, the B-cell leukemia/lymphoma 2 (BCL2) gene, the phosphatidyl inositol 3-kinase catalytic subunit (PIK3CA) gene, the Cyclin D1 gene, the Liver Kinase B1 (LKB1) gene, the c-Abl gene, the c-Sis gene, the epidermal growth factor receptor (EGFR) gene, the platelet-derived growth factor receptor (PDGFR) gene, the vascular endothelial growth factor receptor (VEGFR) gene, the HER2/neu gene, the Raf kinase gene, the cyclin-dependent kinase gene, the Tumor protein p63 (TP63) gene, and mutated genes thereof

Preferred use may be made of the c-Myc gene, the v-Src gene, the RAS gene, the BCL2 gene, the PIK3CA gene, the Cyclin D1 gene, the LKB1 gene, the EGFR gene, the TP63 gene, and mutated genes and splicing isoforms thereof.

In the invention, the type of cancer-associated gene used is of no particular concern, although a mammalian cancer-associate gene is preferred, a primate cancer-associated gene is more preferred, and a human cancer-associated gene is even more preferred.

The "c-Myr gene" is a gene which encodes a DNA-binding factor associated with regulation of the expression of various genes and DNA replication, *i.e.,* a transcription factor.

It has been suggested that an association exists between the impaired expression of the c-Myc gene and various cancers (Dominguez-Sola, D., et al., Nature 448, 445-451 (2007)).

The "v-Src gene" is a cancer-associated gene from the Rous sarcoma virus, which is a type of retrovirus. The sequence for this gene has been described by Mayer B.J., et al., J. Virol. 60 (3), 858-67 (1986).

The "RAS gene" is a gene which encodes a small GTPase that transmits signals which take part in cell differentiation and growth. Cells are known to become cancerous from anomalies in such signal transmission (Goodsell, D.S., Oncologist 4 (3), 263-264 (1999)). RAS gene mutations have been reported in 20 to 30% of non-small cell lung cancers (Aviel-Ronenv, S., et al., Clin. Lung Cancer 1, 30-38 (2006)). In this invention, the RAS gene is a KRAS mutated gene. Here, the KRAS mutated gene is a KRAS gene resulting from the mutation of a wild-type KRAS gene. One example is the gene coding for mutant $KRAS^{V12}$ (SEQ ID NO:6) in which the glycine residue at the 12 position has been replaced with a valine residue. Details on the gene coding for $KRAS^{V12}$ are given in Sato, M. et al, Cancer Res. 66 (4), 2116-2128 (2006).

The "BCL2 gene" is a cancer-associated gene having an apoptosis-suppressing activity, and has been implicated as being associated with melanoma, prostate cancer, breast cancer and lung cancer (Chao, D.T., Korsmeyer, S.J., Annu. Rev. Immunol. 16, 395-419 (1998)).

The "PIK3CA gene" is a gene which encodes a class I PI 3-kinase catalytic subunit, and has been reported to be associated with breast cancer, colorectal cancer and lung cancer (Nature Reviews Cancer 5, 921-929 (2005); Sci. Transl. Med. 2, 26-25 (2010)). In addition, it has been suggested that the PIK3CA gene functions as a cancer-associated gene in the development of uterocervical cancer (Ma, Y.Y., et al., Oncogene 19 (23), 2739-2744 (2000)). In the present invention, the PIK3CA gene is a PIK3CA mutated gene. The PIK3CA mutated gene is a PIK3CA gene that has mutated against its wild type gene, an illustrative example being a gene coding for a $PIK3CA^{H1047R}$ mutated protein (SEQ ID NO:10) in which the histidine residue at the 1047 position has been replaced with arginine.

The "Cyclin D1 gene" is a gene which encodes a cell cycle regulating factor. Overexpression of the Cyclin D1 gene plays a part in the development of parathyroid adenoma, breast cancer, prostate cancer, colorectal cancer, lymphoma, melanoma and lung cancer (Morgan, D.O., Cell 135, 764-794 (2008); Lung Cancer 55, 1-14 (2007)).

The "LKB 1 gene" is a gene which codes for Liver Kinase B1, and its overexpression has been implicated in the development of lung cancer, uterocervical cancer, breast cancer, testicular cancer, pancreatic cancer and skin cancer (Katajisto, P., et al., Biochem. Biophys. Acta 1775 (1), 63-75 (2007)). In this invention, the LKB1 gene is a LKB1 mutated gene. The LKB1 mutated gene is a LKB1 gene that has mutations against its wild type gene. For example, preferred use may be made of a gene coding for a dominant negative-type mutant of LKB1 (LKB1-DN). An example of a dominant negative type LKB1 mutant is $LKB1^{D194A}$ (SEQ ID NO: 16) in which the aspartic acid at the 194 position has been replaced with alanine.

The "EGFR gene" is a gene coding for an epithelial growth factor receptor. The elevation of the expression of the EGFR gene has been observed in many types of cancer, including breast cancer, colorectal cancer, stomach cancer and brain tumors. EGFR gene mutations are known to be found at a high incidence in adenocarcinoma of the lung (Sharma, S.V., et al., Nat. Rev. Cancer 7 (3), 169-81 (2007)). In the present invention, the EGFR gene is an EGFR mutated gene. The EGFR mutated gene is a EGFR gene that has been mutated against its wild type gene, an illustrative example being the gene coding for the $EGFR^{T790M\ L858R}$ mutated protein (SEQ ID NO: 18) in which the threonine residue at the 790 position has been replaced with a methionine residue and the leucine residue at the 858 position has been replaced with an arginine residue.

The "TP63 gene" is a homolog of the TP53 cancer suppressor gene; cases in which it suppresses cancer and cases in which it promotes cancer are both known. The elevation of the expression of the TP63 gene has been reported in squamous cell cancer of the head and neck and of various organs, including the lungs and esophagus (Deyoung, M.P., et al., Oncogene 26, 5169-5183 (2007)). The TP63 gene in this invention is preferably a gene which encodes the splicing isoform of TP63. An example of a gene which codes for the splicing isoform of TP63 is the $TP63^{\Delta N}$ gene which codes for the TP63 splicing isoform which lacks an N-terminal transactivation domain (SEQ ID NO:20).

The respective nucleotide sequences of the human c-Myc, BCL2 and Cyclin D1 genes are available in the NCBI database under the accession numbers indicated below. Details on the respective nucleotide sequences for the $KRAS^{V12}$, $PIK3CA^{H1047R}$ and $LKB1^{D194A}$ genes can be obtained by referring to the wild-type gene sequences available in the NCBI database under the accession numbers indicated below, as well as to McCoy, M.S. et al., Mol. Cell. Biol. 4, 1577-1582 (1984), Samuels, Y., et al., Science 304, 554 (2004), and Scott, K.D. et al., Cancer Res. 67, 5622-5627 (2007).

Table 1

| Gene name | NCBI Accession No. | Species | SEQ ID NO (gene) | SEQ ID NO (protein) |
|---|---|---|---|---|
| v-src gene | -- | Rous sarcoma virus | 1 | 2 |
| c-Myc gene | V00568 | Homo sapiens | 3 | 4 |
| KRAS gene | NM_004985 | Homo sapiens | -- | -- |
| $KRAS^{V12}$ gene | -- | Homo sapiens | 5 | 6 |
| BCL2 gene | NM_000633 | Homo sapiens | 7 | 8 |
| PIK3CA gene | NM_006218.2 | Homo sapiens | -- | -- |
| PIK3CA gene | NM_174574.1 | Bos taurus | -- | -- |
| $PIK3CA^{H1047R}$ gene | -- | Homo sapiens | 9 | 10 |
| $PIK3CA^{H1047R}$ gene | -- | Bos taurus | 11 | 12 |
| Cyclin D1 gene | NM_053056.2 | Homo sapiens | 13 | 14 |
| LKB gene | NM_000455.4 | Homo sapiens | -- | -- |
| $LKB1^{D194A}$ gene | -- | Homo sapiens | 15 | 16 |
| EGFR gene | NM_005228.3 | Homo sapiens | -- | -- |
| $EGFR^{T790M\ L858R}$ gene | -- | Homo sapiens | 17 | 18 |
| TP63 gene | NM_003722.4 | Homo sapiens | -- | -- |
| $TP63^{\Delta N}$ gene | NM_001114980.1 | Homo sapiens | 19 | 20 |

<u>Immortalized Small Airway Epithelial Cells</u>

In the invention, the "immortalized small airway epithelial cells" are immortalized cells from primate small airway epithelial cells, preferably immortalized cells from human small airway epithelial cells. Of such immortalized cells from small airway epithelial cells, immortalized cells from normal small airway epithelial cells are still more preferred. Here, "normal" refers to a healthy state; that is, a state free of any detectable disease or anomaly.

Immortalized Human Small Airway Epithelial Cells

In the invention, "immortalized human small airway epithelial cells" are not subject to any particular limitation, provided they are immortalized cells from a human small airway epithelial cells. However, immortalized cells from normal human small airway epithelial cells are preferred. "Normal human small airway epithelial cells" refer to human small airway epithelial cells from a healthy person, such as small airway epithelial cells of a person in a state free of any detectable disease or anomaly.

The above-described immortalized small airway epithelial cells or immortalized human small airway epithelial cells can be produced by subjecting normal small airway epithelial cells or normal human small airway epithelial cells to any one or combination of treatments (1) to (3) below, although carrying out all of treatments (1) to (3) is preferred. The order in which treatments (1) to (3) are carried out is of no particular concern:

(1) forced expression of a telomerase gene;
(2) forced expression of a cyclin-dependent kinase gene;
(3) induction of loss of function of an apoptosis-inducing protein.

Concerning Treatment (1), telomerase is an enzyme which extends specific repeat sequences at the ends of eukaryotic chromosomes. Telomerase is composed of subunits such as telomere reverse transcriptase, telomere RNA component (TERC), dyskerin and telomerase-associated protein 1 (TEP1), each of which is encoded at a different gene loci. The telomerase gene which is forcibly expressed in Treatment (1) is exemplified by the various genes coding for telomere reverse transcriptase, TERC, dyskerin and TEP1. However, in this invention, preferred use can be made of the telomerase reverse transcriptase gene (referred to below as the "TERT gene") in particular.

Concerning Treatment (2), cyclin-dependent kinases (CDK) take part in turnover of the cell cycle. This family of kinases is composed of CDK1 to 13. The cyclin-dependent kinase (Cdk) gene which is forcibly expressed in Treatment (2) is a gene coding for CDK4 (the Cdk4 gene).
CDK4 is the binding partner of Cyclin D1, and mutations of the Cdk4 gene have been detected in various types of tumors (Zuo, L., et al., Nature Genet. 12, 97-99 (1996)).

Concerning Treatment (3), examples of apoptosis-inducing proteins include p53, caspase-3, caspase-8 to caspase-10, and caspase-12. The protein whose loss of function is induced in Treatment (3) is p53. Examples of methods for inducing p53 loss of function include adding a p53 protein-neutralizing antibody, transferring a gene coding for this antibody into the cell, knockout of the TP53 gene, which is the gene coding for p53, knockdown by RNA interference, and forced expression of a dominant negative-type TP53 gene.

An example of a published study on knockdown of the TP53 gene by RNA interference is that by Sato, M., et al. in Cancer Res. 66, 2116-2128 (2006)). Sato, M., et al. have immortalized human airway epithelial cells by carrying out knockdown of the TP53 gene by RNA interference.

In above Treatments (1) to (3), the species of the genes that are forcibly expressed are of no particular concern, although mammalian genes or proteins are preferred, primate genes or proteins are more preferred, and human genes or proteins are even more preferred.

In the invention, immortalized small airway epithelial cells that are especially preferable for use are cells in which immortality has been acquired by forcibly expressing the TERT gene and the Cdk4 gene and inducing p53 loss of function in normal small airway epithelial cells (referred to below as "SAE cells"). Even more preferred examples are cells in which immortality has been acquired by transferring the TERT gene, the Cdl4 gene and the dominant negative-type TP53 gene to a SAE cell, and forcibly expressing these genes.

Immortalized human airway epithelial cells that may be more preferably used are cells in which immortality has been acquired by forced expressed of the hTERT gene and the Cdk4 gene in normal human small airway epithelial cells (referred to below as "HSAE cells"), and by the induction of p53 loss of function. Even more preferred examples are cells (referred to below as "HSAE/4T53 cells") in which immortality has been acquired by transferring the hTERT gene, the Cdk4 gene and the dominant negative-type TP53 gene to a HSAE cell, and forcibly expressing these genes. The nucleotide sequences of the hTERT gene, the Cdk4 gene and the TP53 gene are available in the NCBI database under the following accession numbers. For the nucleotide sequences of the dominant negative-type TP53 gene, reference may be made to Shaulian, E., et al., Mol. Cell. Biol: 12, 5581 (1992).

Table 2

| Gene name | NCBI Accession No. | Species | SEQ ID NO (gene) | SEQ ID NO (protein) |
|---|---|---|---|---|
| hTERT gene | NM_198253.2 | Homo sapiens | 21 | 22 |
| Cdk4 gene | NM_000075.2 | Homo sapiens | 23 | 24 |
| TP53 gene | NM_000546.4 | Homo sapiens | -- | -- |
| Dominant negative-type TP53 gene | -- | Homo sapiens | 25 | 26 |

The SAE cells used may be ones collected from a primate. Alternatively, cells that are commercially available (from CELLnTEC, in Bern, Switzerland) may be used.

The HSAE cells used may be ones collected from a human. Alternatively, cells that are commercially available (from Lonza, in Walkersville, MD, USA) may be used.

The method of transferring genes to normal small airway epithelial cells or to normal human small airway epithelial cells is similar to the method of transferring genes to immortalized human small airway epithelial cells that is described below.

Gene Transfer Method

In the invention, when transferring a cancer-associated gene into an immortalized human small airway epithelial cell, typically the cancer-associated gene is inserted into an expression vector as a suitable expression cassette, and the immortalized human small airway epithelial cell is transformed with the vector. A suitable expression cassette includes at least (i) to (iii) below as constituent elements:

(i) a promoter which can be transcribed in an immortalized human small airway epithelial cell;
(ii) a cancer-associated gene bound to the promoter; and
(iii) a sequence which encodes an RNA molecule transcription termination and polyadenylation signal.

Examples of promoters which can transcribed in immortalized human small airway epithelial cells include, but are not limited to, CMV, CAG, LTR, EF-1a and SV40 promoters.

The expression vector may have, other than the above expression cassette, a selective marker expression cassette for selection of the transformed immortalized human small airway epithelial cell. Examples of selective markers include, but are not limited to, positive selection markers such as neomycin-resistant genes and the hygromycin B phosphotransferase gene; expression reporters such as the LacZ, GFP (Green Fluorescence Protein) and luciferase genes; and negative selection markers such as the herpes simplex virus thymidine kinase gene (HSV-TK) and the diphtheria toxin A fragment (DTA).

Transformed immortalized human small airway epithelial cells that have been transformed can easily be selected by means of the above markers. For example, in the case of cells to which a neomycin-resistant gene has been transferred as a marker, primary selection can be carried out by culturing the cells in a medium to which G418 has been added. Alternatively, in cases where the targeting vector includes a gene for a fluorescent protein such as GFP as the marker, in addition to selection by drug resistance, sorting of the fluorescent protein expressing cells can be carried out using a fluorescence activated cell sorter (FACS).

Expression vectors which may be used for transferring a cancer-associated gene into immortalized human small airway epithelial cells are exemplified by known expression vectors capable of gene transfer into cells, including expression vectors of this type that are commercially available. Examples of such expression vectors include pEGFP-C1™ (Clontech), pCMV-HA™ (Clontech), pMSCVpuro™ (Clontech), pEF-DEST51™ (*Invitrogen*), pCEP4™ (*Invitrogen*), and ViraPower II Lentiviral Gateway System™ (*Invitrogen*). The expression vector may be transferred into immortalized human small airway epithelial cells by a known gene transfer method such as electroporation, microinjection, the calcium phosphate method, lipofection or virus infection. For further details on gene transfer methods, reference may be made to Sambrook & Russell, Molecular Cloning: A Laboratory Manual, Vol. 3 (Cold Spring Harbor, Laboratory Press, 2001).

2. Tumor Cells

In the invention, "tumor cells" refer to cells which hyperproliferate autonomously *in vivo.* Examples of tumor cells include cells included in (1) sarcomas such as osteosarcoma and soft tissue sarcoma, (2) carcinomas such as carcinoma of the breast, carcinoma of the lung, carcinoma of the bladder, carcinoma of the thyroid gland, carcinoma of the prostate, carcinoma of the colon, colorectal carcinoma, carcinoma of the pancreas, carcinoma of the stomach, carcinoma of the liver, carcinoma of the uterus, carcinoma of the cervix and carcinoma of the ovary, (3) lymphomas such as Hodgkin lymphoma and non-Hodgkin lymphoma, (4) neuroblastomas, (5) melanomas, (6) myelomas, (7) Wilms tumors, (8) leukemias such as acute myelocytic leukemia (AML), chronic myelocytic leukemia (CML), acute lymphocytic leukemia (ALL) and chronic lymphocytic leukemia (CLL), (9) gliomas, and (10) retinoblastomas.

The "tumor cells" may also be exemplified by, in terms of the degree of differentiation, differentiated cancer cells and undifferentiated cancer cells. In some embodiments of the invention, preferred forms of the tumor cell include undifferentiated cancer cells and differentiated lung cancer cells. Differentiated lung cancer cells may be further classified into poorly differentiated lung cancer cells and differentiated lung cancer cells. Example of poorly differentiated lung cancer cells include large cell cancer-like cells. Examples of differentiated lung cancer cells include squamous cancer cells and adenocarcinoma cells.

Confirmation of the tumorigenic transformation of cells may be carried out by transferring a cancer-associated gene into immortalized human small airway epithelial cells or immortalized human small airway epithelial cells, culturing these cells for several days, then subcutaneously injecting the cultured cells into a suitable model animal and observing tumor mass formation.

The model animal is preferably a mammal other than human, and more preferably an immunosuppressed mammal. Examples of immunosuppressed mammals include, but are not limited to, nude rats, nude mice and SCID mice.

Another exemplary method of confirming the tumorigenic transformation of cells involves culturing on soft agar immortalized human small airway epithelial cells into which a cancer-associated gene has been transferred, and observing colony formation by the cells (soft agar colony forming assay). Specifically, this is a method in which immortalized human small airway epithelial cells are prepared with a certain cell concentration, seeded onto a soft agar medium, and the rate of cell proliferation is observed. For details on soft agar colony forming assays, reference may be made to Tanaka, S., et al., Proc. Natl. Acad. Sci. USA 94, 2356-2361 (1997).

Types of Tumor Cells

In the method of the invention, undifferentiated cancer cells, poorly differentiated lung cancer cells or differentiated lung cancer cells may be selectively produced depending on the combination of cancer-associated genes that are transferred.

Undifferentiated Cancer Cells:

In the method of the invention, undifferentiated cancer cells can be produced by transferring the combination of genes shown in (A) or (B) below to immortalized human small airway epithelial cells:

(A) c-Myc gene and v-Src gene;
(B) c-Myc gene, KRAS mutated gene and BCL2 gene.

In this method, an N-Myc gene or an L-Myc gene may be used in place of the c-Myc gene, an HRAS gene or an NRAS gene may be used in place of the KRAS gene, or a BCL-X gene may be used in place of the BCL2 gene.

With regard to the undifferentiated cancer cells produced by the above combinations of genes (which cells are referred to below as the "undifferentiated cancer cells of the invention"), in immunohistochemical analysis, most of the cells are negative or weakly positive for the epithelial cell marker cytokeratin (antibody clone name, AE1/AE3), and positive for the mesenchymal marker VIMENTIN (FIGS. 1C and 5). However, the presence of some cells which are positive for AE1/AE3 and negative for VIMENTIN is acceptable (FIG. 1C).

The undifferentiated cancer cells of the invention are characterized with a high tumorigenicity (FIGS. 2 and 4).

Undifferentiated cancer cells produced by the combination of genes in (A) above have, as shown in FIG. 1B, a tumorigenicity about twice as large that of NCI-H460 human non-small cell lung cancer cells (ATCC HTB-177), and are capable of forming tumor tissue even when only ten of the cells have been inoculated subcutaneously in immunodeficient mice (FIG. 2A). As a result, undifferentiated cancer cells produced by the combination of genes in (A) according to the invention have a very strong tumor-initiating ability, which can be regarded as one characteristic of a cancer stem cell.

Undifferentiated cancer cells produced by the combination of genes in (B) above are able to form tumors in nude mice at a high incidence of 80%, and thus have a high tumor-forming ability (FIG. 4).

Poorly Differentiated Lung Cancer Cells:

In the method of the invention, by transferring the combination of genes shown below in (C) to immortalized human small airway epithelial cells and inductively expressing the c-Myc gene, poorly differentiated lung cancer cells can be produced:

(C) Inducible c-Myc Gene and v-Src Gene.

Here, "inducible c-Myc gene" refers to a c-Myc gene which is capable of inductive expression under an external stimulus, or to an expression system thereof. "Inducible c-Myc genes" are encompassed by the above-mentioned "c-Myc genes." Also, "inductive expression" refers to the control of gene expression by an expression-inducing system. Inductive expression should be started after transfer of the genes indicated in (C) to the immortalized human small airway epithelial cells, and is preferably carried out in the period following transfer to the immortalized human small airway epithelial cells and up to confirmation of tumor formation.

The expression-inducing system is not subject to any particular limitation, provided expression of the target gene (c-Myc gene) can be artificially controlled. Illustrative examples include an expression-inducing system which uses dexamethasone and a mouse mammary tumor virus (MMTV) promoter, an expression-inducing system (Tet-on system) which uses tetracycline, doxycycline or the like and a tetracycline-responsive promoter, an expression-inducing system which uses metal ions such as nickel, cobalt, manganese or iron and a metallothionein promoter, and an expression-inducing system which uses a heat shock protein promoter.

Alternatively, once the c-Myc gene has been forcibly induced and tumor formation has been confirmed, the c-Myc function may be destroyed using a system which suppresses expression of the c-Myc gene (e.g., a Tet-off system) or a system which degrades the c-Myc protein (Auxin-based degron system: Nishimura, K. et al., Nature Methods, Vol. 6, No. 12 (December 2009)). Advantageous use can be made of a Tet-on system, with the addition of doxycycline being preferred from the standpoint of sensitivity.

Also, in the present invention, an N-Myc gene or an L-Myc gene may be used instead of the c-Myc gene.

The poorly differentiated lung cancer cells produced with the above combinations of genes (which cells are referred to below as "poorly differentiated lung cancer cells of the invention") exhibit the large cell cancer-like morphological feature of being positive for the epithelial cell marker cytokeratin (antibody clone name, AE1/AE3) (FIG. 3C).

Differentiated Lung Cancer Cells:

In the method of the invention, differentiated lung cancer cells can be produced by transferring the combination of genes shown in any of (D) to (H) below to immortalized human small airway epithelial cells:

(D) KRAS mutated gene and PIK3CA mutated gene;
(E) KRAS mutated gene and Cyclin D 1 gene;
(F) KRAS mutated gene and LKB 1 mutated gene;
(G) KRAS mutated gene, Cyclin D1 gene and TP63 gene;
(H) Cyclin D1 gene and EGFR mutated gene.

In the invention, an HRAS gene or an NRAS gene may be used instead of the KRAS gene, or a Cyclin D2 gene or a Cyclin D3 gene may be used instead of the Cyclin D1 gene.

Differentiated lung cancer cells produced with the above gene combinations (which cells are referred to below as "differential lung cancer cells of the invention") are all characterized by having a very high tumorigenicity (FIG. 4).

Of the differentiated lung cancer cells of the invention, those cells produced with combination (D) or (H) exhibit a morphology in which squamous epithelial-like cells and adenocarcinoma-like cells are intermixed, whereas those cells produce with combination (E) or (F) each have the morphology of adenocarcinoma-like cells. Cells produced with combination (G) have the morphology of squamous epithelial-like cells.

In immunohistochemical analysis, differentiated lung cancer cells produced with any one of combinations (D), (E) and (F) are positive for AE1/AE3 and p63 (FIG. 6). Differentiated lung cancer cells produced with combination (H) are positive for AE1/AE3 (FIG. 11C) and positive for Alcian Blue staining (FIG. 11B).

The tumor cells of the invention all have a high tumor incidence and ability to proliferate. When these cells are implanted in model animals and induced to form tumors, the tumors thus formed show very similar pathology to tumor tissue which forms in the animal species to which the small airway epithelial cells prior to transfer of the cancer-associated gene belong. For example, when a human small airway epithelial cell is used, thus formed tumor shows very similar pathology to that of a cancer tissue, especially lung cancer tissue, isolated from human cancer patients. Therefore, the tumor cells of the invention are very useful in a wide variety of lung cancer research, such as research on the reprogramming, differentiation and biological mechanisms of lung cancer cells, the identification of target molecules for lung cancer treatment, and the screening of cancer drugs.

3. Tumor-Bearing Animal Model

A "tumor-bearing animal model" refers to a non-human animal model in which the above-described tumor cells have been implanted and a tumor mass has been formed.

The model animal is preferably a mammal other than human, examples of which include, but are not limited to, mouse, rat, pig, dog, monkey, hamster and rabbit. Of these model animals, an immunosuppressed mammal is especially preferred. Immunosuppressed mammals can be created by administering an immunosuppressant such as cyclosporine to an ordinary mammal, although a mammal in which immunity has been congenitally suppressed on account of the genetic background is preferred. Examples of congenitally immunosuppressed mammals include, but are not limited to, nude rats, nude mice and SCID mice.

No particular limit is imposed on the method of transferring the above-described tumor cells to the model animal. A method that is conventionally used in the art may be adequately selected according to the model animal in which implantation is to be carried out. As an instance of implantation in an animal other than mice, reference may be made to, for example, "Genetic induction oftumorigenesis in swine," Oncogen 26, 1038-1045 (September 11, 2006). Also, insofar as the tumor cells used at the time of implantation form tumor masses when implanted in the model animals, the model animals may be of the same species or of different species.

From the standpoint of the ease of re-extracting the implanted tumor cells, implantation by subcutaneous injection or intraperitoneal injection is preferred. On the other hand, from the anatomical standpoint, orthotopic implantation is preferred.

4. Method of Screening for Cancer Drugs

A method for screening cancer drugs includes the steps of:

(a) contacting a sample derived from the tumor cells produced by the method of the invention with a candidate substance; and
(b) detecting tumor cell growth inhibiting effects.

"A sample derived from the tumor cells" refers to tumor cells or tumor tissue, or to a sample that has been suitably prepared so as to be easy to use in the screening method steps.

"Contacting a sample derived from the tumor cells with a candidate substance" means that the candidate substance approaches to a degree such as to give rise to interactions with molecules at the surface of the tumor cells, binds with such molecules, or is taken up into the tumor cells. In cases where the tumor cells are cultured cells, the cells and the candidate substance can be brought into contact by adding the candidate substance to at least a given concentration to the culture medium in contact with the cells. On the other hand, in cases where the tumor cells have been implanted into the body of an animal, the tumor cells and the candidate substance can be brought into contact by administering a given amount of the candidate substance to the animal. In such a case, the route of administration is not particularly limited, provided it is a route commonly used for administering the candidate substance. Illustrative examples of suitable routes of administration include oral, sublingual, nasal, pulmonary, gastrointestinal and percutaneous administration, ocular instillation, intravenous injection, subcutaneous injection, intramuscular injection, intraperitoneal injection, localized injection, and surgical implantation. Preferred routes are oral administration, intraperitoneal injection and intravenous injection.

Exemplary candidate substances include drugs which have already been confirmed to have antitumor effects, and compounds, polypeptides, nucleic acids, antibodies and low-molecular-weight compounds having latent antitumor effects. Illustrative examples of such candidate substances include, but are not limited to, antimetabolites (e.g., 5-fluorourasyl (5-FU)), antagonists of folic acid metabolism (e.g., the dihydropteroic acid synthase inhibitors sulfadiazine and sulfamethoxazole, and the dihydrofolic acid reductase inhibitors (DHFR inhibitors) methotrexate, trimethoprim and pyrimethamine), inhibitors of pyrimidine metabolism (e.g., the thymidylic acid synthase inhibitors 5-FU and flucytosine (5-FC)), inhibitors of purine metabolism (e.g., the IMPDH inhibitors 6-mercaptopurine and the prodrug thereof azathioprine), adenosine deaminase (ADA) inhibitors (e.g., pentostatin), ribonucleotide reductase inhibitors (the ribonucleotide reductase inhibitor hydroxyurea), nucleotide analogs (the purine analogs thioguanine, fludarabine phosphate and cladribine; the pyrimidine analogs cytarabine and gemcitabine), L-asparaginase, alkylating agents (e.g., the nitrogen mustards cyclophosphamide, melphalan and thiotepa; the platinum agents cisplatin, carboplatin and oxaliplatin; and the nitrosoureas dacarbazine, procarbazine and ranimustine), antitumor antibiotics (sarkomycin, mitomycin C, doxorubicin, epirubicin, daunorubicin), topoisomerase inhibitors (irinotecan, nogitecan, doxorubicin, etoposide, levofloxacin, ciprofloxacin), microtubule inhibitors (vinblastin, Vincristine, vindesine), colhicine, microtubule inhibitors (paclitaxel, docetaxel), molecularly targeted drugs (trastuzumab, rituximab, imatinib, gefitinib, bortezomib, erlotinib), steroids (e.g., dexamethasone), fmasteride, aromatase inhibitors, tamoxifen, and combinations thereof.

To determine the tumor cell growth inhibiting effects, two systems may be prepared, either in the form of a culture system wherein the same number of cells are seeded in culture tissue at the same cell concentration and cultured under the same conditions, or in the form of a transplantation animal model of the same line wherein the same number of cells are transplanted in the same cell concentration. The candidate substance is brought into contact with the cells of one system (the sample), but is not brought into contact with the cells of the other system (the control), and tumor cell growth in both systems is observed. The growth inhibiting effects can be ascertained by measuring and comparing the numbers of tumor cells present in the two systems after a given period of time has elapsed.

In cases where the tumor cells produced by the method of the invention are used for screening in the form of cultured cells, after the candidate substance has been brought into contact with the sample cells, the number of sample cells and the number of control cells are measured with a cell counter or the like, and the tumor growth effects can be determined by comparing the respective numbers of cells.

In cases where the tumor cells produced by the method of the invention are used for screening by implantation in an animal model, after the candidate substance has been administered to the animal model serving as the sample system, tumor tissue is removed from both sample animals and control animals, and the numbers of cells present in tumor tissue from the sample animals and from the control animals can be measured and compared. Alternatively, the tumor growth effects can be determined by removing the tumor tissue and comparing the volume of the tumor tissue from the sample animals and the control animals. The tumor volume can be determined using the following formula.

$$\text{Tumor volume} = ab^2/2 \qquad \text{(where a is the width, and b is the length)}$$

It is also possible, after administering the candidate substance to both sample and control animals, to determine the tumor growth effect by comparing the incidence of measurable tumor formation at the tumor cell implantation site in the sample animals and the control animals.

The animal model is the same as the animal model described above under "2. Tumor Cells." No particular limitation is imposed on the method of implantation in the animal model, although subcutaneous injection or intraperitoneal injection is preferred on account of the ease of re-extracting the implanted tumor cells.

In cases where the rate of increase in tumor cells within a sample system is smaller than the rate of increase in tumor cells within a control system, it may be concluded that the candidate substance has an antitumor effect. Alternatively, in cases where ample data on the rate of increase under given conditions already exists for the tumor cell produced by the method of the invention, such determinations may be made by comparison with a baseline derived from average values, standard deviations, etc. obtained by the statistical treatment of such data.

Average values, standard deviations, etc. for the tumor rate of increase can be obtained by various statistical methods. Specifically, in the case of cultured cells, by using as the parameters the initial number of cells and the cell density at the time of seeding, or, in the case of implanted cells, by using as the parameters the weight of the animal model at the time of implantation and the number of tumor cells implanted, these values can be determined by two-way ANOVA analysis using statistical analysis software such as IBM SSPS Statistics 18 (SSPS). It is possible to further enhance the accuracy of analysis by adding the rate of increase in tumor cells obtained from carrying out the method of the invention as new data to a population for statistical analysis and thus increasing the parameters.

Because the tumor cells produced by the method of the invention exhibit characteristics which are pathologically very similar to lung cancer cells isolated from patients, it is expected that cancer drugs confirmed by the screening method to have tumor cell growth-suppressing effects will also have antitumor effects when used in the treatment of actual cancer patients, particularly lung cancer patients.

The invention is illustrated more fully below by way of examples, although the invention is not limited to the modes thereof described in the examples.

Examples

The procedures of the experiments carried out in the examples are described below.

Retrovirus Vector and Retrovirus-Mediated Gene Transfer

Of the genes shown in Table 1, v-Src (SEQ ID NO:1) and $KRAS^{V12}$ (SEQ ID NO:5) were inserted into the pCX4pur vector (GenBank#: AB086386). c-Myc (SEQ ID NO:3), $PIK3CA^{H1047R}$ (SEQ ID NO: 11), Cyclin D1 (SEQ ID NO:13) and LKB1$^{D194A}$ (SEQ ID NO:15) were inserted into the pCX4bleo vector (GenBank#: ABO86388). BCL2 (SEQ ID NO:7) was inserted into the pCX4redEx vector (GenBank#: AB296084). The EGFR$^{T790M\ L858R}$ gene (SEQ ID NO: 17) was inserted into the pCX4pur vector (GenBank Accession No. AB086386). The TP63$^{\Delta N}$ gene (SEQ ID NO: 19) was inserted into the pCX4hyg vector (GenBank Accession No. AB086387). Of the genes shown in Table 2, hTERT (SEQ ID NO:21) was inserted into the pCX4neo vector (GenBank#: AB086385), CDK4 (SEQ ID NO:23) was inserted into the pCX4bsr vector (GenBank#: AB086384), and dominant-negative TP53 (SEQ ID NO:25) was inserted into the pCX4.1hisD vector (GenBank#: AB086389). In addition, for the inducible c-Myc gene, an inducible expression vector was prepared by inserting the c-Myc gene into a pT-REx inducible expression vector *(Invitrogen)* the expression of which can be controlled with tetracycline. Viruses were produced by transferring the above retroviral expression vectors, together with pGP and pE-eco plasmids (Takara Bio; Shiga, Japan), into 293T cells (ATCC; Manassas, USA). Next, HSAE cells that had been made to express Ecotropic receptor (Eco VR) were infected with a retrovirus. The cells infected with the virus vector were cultured for two weeks in the presence of blasticidin S, G418, puromycin, zeocine and L-Histidinol, thereby carrying out selection. Regardless of which of these drugs selection was carried out with, the cultured cells were selected from a polyclonal proliferative population of infected cells.

<u>Cell Culture</u>

Normal human small airway epithelial cells from a 19-year old Caucasian female were purchased from Lonza (Walkersville, MD, USA). These cells were placed on collagen-coated tissue and grown in a serum-free SAGM medium to which various growth factors supplied by Lonza had been added (SAGM Bullet Kit; Lonza). The cells were maintained in a low-oxygen environment (3% $O_2$ and 5% $CO_2$) within a wet incubator at37°C.

<u>Xeno graft Growth Experiment</u>

A xenograft growth experiment was carried out on mice. Single-cell suspensions containing $1\times10^6$ HSAE/4T53 cells or HSAE cells transduced with various cancer-associated genes were suspended in 50% MATRIGEL™ (BD Bioscience; San Jose, CA, USA), and subcutaneously injected into the flanks of 6- or 7-week female athymic nude mice (BALB/c nu/nu; Japan SLC; Hamamatsu, Japan) or NOD-SCID mice (Charles River). The dimensions of the tumors were measured with a vernier caliper and the tumor volume was calculated from the following equation, based on which the tumorigenicity was estimated.

$$\text{Tumor volume} = ab^2/2$$

(where a is the width, and b is the length)

Alternatively, the ratios in which measurable tumor masses formed at the sites to which the various cancer-associated gene-transduced HSAE/4T53 cells or HSAE cells had been implanted were calculated as the tumor incidence, based on which the tumorigenicity was estimated.

<u>Immunological Analysis and Immunohistochemical Staining</u>

The xenograft was formalin-fixed and paraffin-embedded, after which it was sectioned and hematoxylin eosin (H&E) staining was carried out according to the normal protocol. Immunohistochemical staining was carried out in accordance with the above literature (Sasai, K., et al., Am. J Surg. Pathol. 32, 1220-1227 (2008)) using the following antibodies: multi-cytokeratins (AE1/AE3; Dako; Glostrup, Denmark), p63 (4A4; Dako), TTF-1 (8G7G3/1; Dako), p53 (D07; Novocastra), and VIMENTIN (V9; Nichirei; Tokyo, Japan). The procedure used to carry out immunohistochemical staining was as follow. Tissue sections having a thickness of 4 μm were deparaffinized with xylene, then dehydrated with ethanol. The antigen was activated by 2 minutes of heating in 10 mM citrate buffer (pH 6.0) within a pressure cooker. The tissue slices were rehydrated with 0.01% Tween 20-containing phosphate-buffered saline (PBST) and incubated with 0.3% hydrogen peroxide, thereby deactivating the endogenous peroxidase. The tissue sections were incubated overnight at 4°C in primary antibody at a suitable dilution concentration, then washed with PBST, following which they were incubated for 30 minutes at room temperature in Envision Dual Link solution (Dako; Glostrup, Denmark). Next, the sections were treated with diaminobenzidine (Dako), thereby visualizing the antigen-antibody reaction sites, and nucleus staining was carried out by 90 seconds of hematoxylin treatment. Slides of the tissue sections were mounted using Entellan Neu reagent (Merck; Whitehouse Station, NJ), then sealed with a cover glass and furnished for observation.

Alcian Blue staining was carried out by formalin-fixing, paraffin-encapsulating and sectioning the xenograft, then staining the sections as described in the above literature (Steedman, H.F., Quarterly Journal of Microscopic Science, Vol. 91, p477-479 (1950)).

Immunoblotting Technique

Protein measurement, SDS-PAGE and immunoblotting were carried out as described in the above literature (Akagi, T., et al., Mol. Cell Biol. 22, 7015-7023 (2002)). Immunopositive protein signals were visualized by chemiluminescence using SuperSignal WestFemto reagent (Pierce, Rockford; IL, USA). Anti-c-Myc monoclonal antibodies were purchased from *Invitrogen* (Carlsbad, CA, USA).

Cloning by Limiting Dilution Technique

The preparation of cell clones from single cells was carried out as described in the above literature (Quintana, E., et al., Nature 456 (7222): 593-8 (Dec. 4, 2008)).

Experimental Results

[1] Production of Cancer Cells Transduced with v-Src Gene and c-Myc Gene

4T53MS cells produced by transferring the c-Myc gene and the v-Src gene to HSAE/4T53 cells were subcutaneously transplanted ($1\times10^6$ cells/animal) in athymic nude mice (BALB/c nu/nu; Japan SLC; Hamamatsu, Japan). The xenograft growth experiment, histological analysis and immunohistochemical staining were carried out on the tumor tissue that formed. Tumor mass formation was observed in the mice into which 4T53MS cells were transplanted (FIG. 1A). The 4T53MS cells had a very high tumorigenicity. Even compared with NCI-H460 lung cancer cells, which are known to have a high tumorigenicity, the 4T53MS cells had a tumorigenicity that was about twice as high (FIG. 1B). This tumor mass was removed, tissue sections were prepared and hematoxylin-eosin (H&E) staining was carried out, whereupon cancer tissue images having a very low degree of differentiation and exhibiting no differentiated morphology were observed. The immunostaining results were negative for staining with p63, a lung epithelium marker, and were negative as well for staining with cytokeratin (antibody clone name, AE1/AE3), which is an epithelial cell marker. On the other hand, the tumor cells were strongly positive for the mesenchymal cell marker VIMENTIN. It was concluded from the above that tumors from 4T53MS cells exhibit the nature of undifferentiated cancer (FIG. 1C). In cells obtained by the gene transfer of v-Src alone or c-Myc alone to HSAE/4T53 cells (the resulting cells are referred to here as, respectively, "4T53S" and "4T53M" cells), tumorigenicity was not observed.

The 4T53MS cells had a very strong tumorigenicity. Even when subcutaneously transplanted in amounts of $1\times10^6$, $1\times10^4$, $1\times10^3$ or $1\times10^2$ cells, tumor formation in NOD-SCID mice was observed at 100% probability (FIG. 2A). Even with the implantation of only ten cells, tumor formation at a probability of 50% was found to be possible (FIGS. 2A, 2B). Therefore, because 4T53MS cells are characterized by a very high tumorigenicity and differentiate into more than one type of cells, this demonstrated that 4T53MS cells have the characteristics of cancer stem cells.

4T53mS cells produced by transferring the inducible c-Myc gene and the v-Src gene to HSAE/4T53 cells were doxycycline (DOX) treated, thereby inducing c-Myc expression in an *in vitro* experimental system (FIG. 3A). 4T53mS cells cultured in the presence of DOX were transplanted subcutaneously in nude mice and DOX administration was continued. When tumor formation was observed, DOX administration was stopped (FIG. 3B). The tumor tissue obtained was stained with cytokeratin, thereby clearly distinguishing between strongly positive tumor cells and negative stromal cells. However, the characteristic images seen in, for example, adenocarcinoma or squamous cell cancer, were not observed, indicating that this was a poorly differentiated large-cell cancer-like tumor (FIG. 3C).

[2] Production of Cancer Cells by Transfer of Human Genes

4T53RM cells obtained by the transfer of both the c-Myc gene and the $KRAS^{V12}$ gene were subcutaneously implanted in athymic nude mice (BALB/c nu/nu; Japan SLC; Hamamatsu, Japan), and the xenograft growth experiment, histological analysis and immunohistochemical staining were carried out on the tumor tissue that formed. The 4T53RM cells had tumorigenicity. However, the tumor incidence was 38% (FIG. 4). 4T53RMB cells obtained by transferring the BCL2 gene into these 4T53RM cells were subcutaneously implanted in athymic nude mice (BALB/c nu/nu; Japan SLC; Hamamatsu, Japan), and the xenograft growth experiment, histological analysis and immunohistochemical staining were carried out on the tumor tissue that formed. The 4T53RMB cells formed tumors in nude mice at a high incidence of 80%.

In immunological examination, histologies that do not exhibit the characteristics of squamous cell cancer or adenocarcinoma were observed in both tumors from 4T53RM cells and tumors from 4T53RMB cells. These tumors were immunostained, as a result of which only a weakly positive reaction to cytokeratin was exhibited (AE1/AE3: FIG. 5). However, because a strongly positive reaction to VIMENTIN was exhibited, this was judged to be undifferentiated cancer.

4T53R cells were produced by transferring the $KRAS^{V12}$ gene to HSAE/4T53 cells. One of the genes $PIK3CA^{H1047R}$, Cyclin-D1 or $LKB1^{D194A}$ was transferred to the 4T53R cells, thereby establishing, respectively, 4T53RP cells, 4T53RD cells and 4T53RL cells. The 4T53R, 4T53RP, 4T53RD and 4T53RL cells were subcutaneously implanted in athymic nude mice (BALB/c nu/nu; Japan SLC; Hamamatsu, Japan), and the xenograft growth experiment, histological analysis and immunohistochemical staining were carried out on the tumor tissue that formed. Although tumorigenicity was not seen in the 4T53R cells, high tumor incidences were observed in the 4T53RP cells, 4T53RD cells and 4T53RL cells (these were respectively 100%, 100%, 83%; FIG. 4).

Histological analyses were carried out on tumors (xenografts) from the 4T53RP cells, 4T53RD cells or 4T53RL cells, whereupon these were found to exhibit histologies similar to actual human lung cancer tissue. In tumors formed with 4T53RP cells, histologies containing both adenocarcinoma-like regions and squamous cell cancer-like regions were observed (FIG. 6; H&E). In tumors (xenografts) formed with 4T53RD cells and 4T53RL cells, because glandular structure formation was observed and tumor stroma were abundantly present, this was regarded to have a morphology similar to that of human lung adenocarcinoma (FIG. 6; H&E). These tumors were composed of cells that are strongly positive for the epithelial cell marker cytokeratin (FIG. 6; AE1/AE3), and are also positive for lung epithelial cell marker p63 staining (FIG. 6; p63). The expression of these markers indicates that the tumors from the 4T53RP, 4T53RD and 4T53RL cells are differentiated tumors resembling human lung adenocarcinoma. Because tumors from 4T53RP cells also included squamous cell cancer-like regions, this was concluded to be a mixture of human adenocarcinoma and squamous cell carcinoma (adenosquamous cell carcinoma).

[3] <u>Production of Cells Having the Nature of Cancer Stem Cells for Lung Adenocarcinoma</u>

Cancer tissue from 4T53RD cells produced by transferring both the $KRAS^{V12}$ and Cyclin D1 genes to HSAE/4T53 cells form a glandular structure composed of a heterogeneous population of cells resembling lung adenocarcinoma. This suggests the possibility that 4T53RD cells have the nature of stem cells with the ability to differentiate into numerous types of cells.

In order to confirm this possibility, 4T53RD cells were cloned by the limiting dilution method, giving several cell clones originated from a single cell. These cell clones were subcutaneously implanted in NOD-SCID mice, and the xenograft growth experiment, histological analysis and immunohistochemical staining were carried out on the tumor tissue that formed. The 4T53RD cell clones from single cells formed tumor masses and, like the parent strain of 4T53RD cells, formed tumors morphologically similar to human lung adenocarcinoma (FIG. 8A).

The mouse airway epithelium, as shown in FIG. 8B, is composed of several cell populations, including basal cells which express p63, clara cells which express TTF1, goblet cells which produce mucin, and ciliated cells.

The tumor tissue that formed upon the implantation of single cell derived 4T53RD cell clones were immunohistochemically stained, as a result of which p63 positive basal cells were present in border areas in contact with the mouse substrate. However, the portion which forms a glandular structure on the inside thereof was p63 negative, and cells positive for the TTF1 which is expressed in clara cells, and mucin-producing cells that are stained by Alcian Blue were present (FIG. 8A). Taking into consideration the fact that, according to Rock, J.R., et al. Proc. Natl. Acad. Sci. USA 106 (31), 12771-5 (Aug. 4, 2009), p63 positive basal cells have recently been shown to function as stem cells in the bronchial tubes, these results suggest that, in tumors obtained by single cell derived 4T53RD cell clones, as p63 positive cells proliferate toward the inside, they differentiate into clara cells and mucin-secreting goblet cells, thereby forming cancer tissue composed of a heterogeneous population of cells.

Also, 4T53RD cell clones have a strong tumorigenicity. When $1\times10^4$, $1\times10^3$, $1\times10^2$, and $1\times10^1$ of these cells were subcutaneously implanted, tumor formation in NOD-SCID mice at probabilities of, respectively, 100%, 80%, 60% and 13% was observed (FIG. 9).

From the above results, 4T53RD cells were shown to possess the following two properties of cancer stem cells: (1) they have the ability to differentiate from a single type of cell into a plurality of types of cells in the course of forming a tumor *in vivo;* and (2) even a small number of cells are capable of forming a tumor.

[4] <u>Production of Cells Having the Characteristic of Lung Squamous Cell Cancer</u>

4T53RDΔNp63 cells produced by transferring the $KRAS^{V12}$, Cyclin D1 and $TP63^{\Delta N}$ genes to HSAE/4T53 cells were subcutaneously implanted in athymic nude mice (BALB/c nu/nu; Japan SLC; Hamamatsu, Japan), and histological analysis and immunohistochemical staining were carried out on the tumor tissue that formed.

4T53RD cells produced by transferring both the $KRAS^{V12}$ gene and the Cyclin D1 gene to HSAE/4T53 cells formed tumors having a morphology similar to that of human lung adenocarcinoma (FIG. 10A), whereas cells produced by transferring not only the $KRAS^{V12}$ and Cyclin D 1 genes but also the $TP63^{\Delta N}$ gene formed, according to histological analysis, squamous cell carcinoma-like tumor tissue (FIG. 10B).

[5] Production of Differentiated Lung Cancer Cells Using EGFR Mutated Gene and Cyclin D1

4T53 ED cells produced by transferring both the EGFR$^{T790M\ L858R}$ gene and the Cyclin D1 gene into HSAE/4T53 cells were subcutaneously implanted in athymic nude mice (BALB/c nu/nu; Japan SLC; Hamamatsu, Japan), and the xenograft growth experiment, histological analysis and immunohistochemical staining were carried out on the tumor tissue that formed.

The 4T53 ED cells exhibited 100% tumor incidence (FIG. 4), and thus were found to have a high tumorigenicity. Also, tumor tissue from 4T53 ED cells formed a particular type of differentiated tumor tissue having a morphology composed primarily of squamous epithelial-like cells among which were interspersed adenocarcinoma-like cells (FIGS. 11A to 11C).

As demonstrated by the above results, by transferring cancer-associated genes into immortalized human small airway epithelial cells (HSAE/4T53 cells), it is possible to regulate the degree of tumor cell differentiation. That is, as shown in FIG. 7, it is possible to produce models of undifferentiated cancer by gene transfer involving the combination of a c-Myc gene and a v-Src gene (4T53MS cells) or the combination of a c-Myc gene, a KRAS mutated gene and a BCL2 gene (4T53RMB cells). Also, by transferring genes in the combination of an inducible c-Myc gene and a v-Src gene (4T53mS cells), it is possible to establish a poorly differentiated lung cancer model resembling human lung large-cell cancer upon c-Myc induction. In addition, by gene transfer in any one of the following combinations, that is, the combination of a KRAS mutated gene and a PIK3CA mutated gene (4T53RP cells), the combination of a KRAS mutated gene and a Cyclin D1 gene (4T53RD cells), the combination of a KRAS mutated gene and a LKB1 mutated gene (4T53RL cells), the combination of a KRAS mutated gene, a Cyclin D1 gene and a TP63 gene (4T53RDΔNp63 cells), or the combination of a EGFR mutated gene and a Cyclin D1 gene (4T53ED cells), it is possible to produce human lung cancer models resembling adenocarcinoma or squamous cell carcinoma (or a mixture thereof).

INDUSTRIAL APPLICABILITY

The present invention provides a method for producing tumor cells which exhibit pathological characteristics similar to those of human lung cancer cells. Such tumor cells are useful in research on the biological mechanisms of lung cancer, the identification of target molecules for lung cancer treatment, and the screening and testing of cancer drugs. In particular, tumor cells produced by the transfer of only cancer-associated human genes faithfully recapitulate the properties of the cell, and are expected to be highly useful in the development of cancer drugs.

## Claims

1. A method for producing tumor cells by transferring a combination of cancer-associated genes into immortalized primate small airway epithelial cells, wherein:

   - the immortalized primate small airway epithelilal cells are produced by subjecting normal primate small airway epithelial cells to following treatments (1) to (3):

     (1) forced expression of telomere reverse transcriptase gene;
     (2) forced expression of cyclin-dependent kinase 4 gene; and
     (3) induction of p53 loss of function, and

   - the combination of cancer-associated genes is:

     (a) c-Myc gene and v-Src gene,
     (b) c-Myc gene, KRAS mutated gene and BCL2 gene,
     (c) KRAS mutated gene and Cyclin D1 gene,
     (d) KRAS mutated gene, Cyclin D1 gene and TP63 gene,
     (e) KRAS mutated gene and PIK3CA mutated gene,
     (f) KRAS mutated gene and LKB1 mutated gene, or
     (g) EGFR mutated gene and Cyclin D1 gene.

2. The method according claim 1, wherein the immortalized primate small airway epithelial cells are human small airway epithelial cells.

3. The method according claim 1 or 2, wherein the combination of cancer-associated genes is c-Myc gene and v-Src gene or c-Myc gene, KRAS mutated gene and BCL2 gene.

4. The method according to claim 3, wherein the tumor cells are undifferentiated cancer cells.

5. The method according to claim 4, wherein the tumor cells are cancer stem cells and wherein the combination of cancer-associated genes is c-Myc gene and v-Src gene.

6. The method according to claim 2, wherein the combination of cancer-associated genes is c-Myc gene and v-Src gene, wherein the c-Myc gene is inductively expressed in the immortalized human small airway epithelial cells.

7. The method according to claim 6, wherein the tumor cells are poorly differentiated lung cancer cells.

8. The method according to claim 1 or 2, wherein the combination of cancer-associated genes is KRAS mutated gene and Cyclin D1 gene.

9. The method according to claim 1 or 2, wherein the combination of cancer-associated genes is KRAS mutated gene, Cyclin D1 gene and TP63 gene; KRAS mutated gene and PIK3CA mutated gene; KRAS mutated gene and LKB1 mutated gene or EGFR mutated gene and Cyclin D1 gene.

10. The method according to claim 8 or 9, wherein the tumor cells are differentiated lung cancer cells.

**11.** The method according to claim 10, wherein the tumor cells are cancer stem cells of differentiated lung cancers.

**12.** The method of claim 10, wherein the combination of cancer-associated genes is (a) KRAS mutated gene and Cyclin D1 gene, (b) EGFR mutated gene and Cyclin D1 gene or (c) KRAS mutated gene and PIK3CA mutated gene.

**Patentansprüche**

**1.** Verfahren zur Herstellung von Tumorzellen durch Transferieren einer Kombination Krebs-assoziierter Gene in immortalisierte kleine Primaten-Atemwegsepithelzellen, wobei:

- die immortalisierten kleinen Primaten-Atemwegsepithel-zellen hergestellt werden, indem normale kleine Primaten-Atemwegsepithelzellen den folgenden Behandlungen (1) bis (3) unterzogen werden:

(1) erzwungene Expression des Telomer-Reverse-Transkriptase-Gens;
(2) erzwungene Expression des Cyclin-abhängige-Kinase-4-Gens und
(3) Induktion von p53-Funktionsverlust und

- die Kombination Krebs-assoziierter Gene ist:

(a) c-Myc-Gen und v-Src-Gen,
(b) c-Myc-Gen, mutiertes KRAS-Gen und BCL2-Gen,
(c) mutiertes KRAS-Gen und Cyclin-D1-Gen,
(d) mutiertes KRAS-Gen, Cyclin-D1-Gen und TP63-Gen,
(e) mutiertes KRAS-Gen und mutiertes PIK3CA-Gen,
(f) mutiertes KRAS-Gen und mutiertes LKB1-Gen oder
(g) mutiertes EGFR-Gen und Cyclin-D1-Gen.

**2.** Verfahren gemäß Anspruch 1, wobei die immortalisierten kleinen Primaten-Atemwegsepithelzellen humane kleine Atemwegsepithelzellen sind.

**3.** Verfahren gemäß Anspruch 1 oder 2, wobei die Kombination Krebs-assoziierter Gene c-Myc-Gen und v-Src-Gen oder c-Myc-Gen, mutiertes KRAS-Gen und BCL2-Gen ist.

**4.** Verfahren gemäß Anspruch 3, wobei die Tumorzellen undifferenzierte Krebszellen sind.

**5.** Verfahren gemäß Anspruch 4, wobei die Tumorzellen Krebsstammzellen sind, und wobei die Kombination Krebsassoziierter Gene c-Myc-Gen und v-Src-Gen ist.

**6.** Verfahren gemäß Anspruch 2, wobei die Kombination Krebsassoziierter Gene c-Myc-Gen und v-Src-Gen ist, wobei das c-Myc-Gen in den immortalisierten humanen kleinen Atemwegsepithelzellen induktiv exprimiert wird.

**7.** Verfahren gemäß Anspruch 6, wobei die Tumorzellen schwach differenzierte Lungenkrebszellen sind.

**8.** Verfahren gemäß Anspruch 1 oder 2, wobei die Kombination Krebs-assoziierter Gene mutiertes KRAS-Gen und Cyclin-D1-Gen ist.

**9.** Verfahren gemäß Anspruch 1 oder 2, wobei die Kombination Krebs-assoziierter Gene mutiertes KRAS-Gen, Cyclin-D1-Gen und TP63-Gen; mutiertes KRAS-Gen und mutiertes PIK3CA-Gen; mutiertes KRAS-Gen und mutiertes LKB1-Gen oder mutiertes EGFR-Gen und Cyclin-D1-Gen ist.

**10.** Verfahren gemäß Anspruch 8 oder 9, wobei die Tumorzellen differenzierte Lungenkrebszellen sind.

**11.** Verfahren gemäß Anspruch 10, wobei die Tumorzellen Krebsstammzellen aus differenziertem Lungenkrebs sind.

**12.** Verfahren gemäß Anspruch 10, wobei die Kombination Krebsassoziierter Gene (a) mutiertes KRAS-Gen und Cyclin-D1-Gen, (b) mutiertes EGFR-Gen und Cyclin-D1-Gen oder (c) mutiertes KRAS-Gen und mutiertes PIK3CA-Gen ist.

## Revendications

**1.** Procédé de production de cellules tumorales par transfert d'une combinaison de gènes associés au cancer dans des cellules épithéliales de petites voies aériennes de primate immortalisées, dans lequel

- on produit les cellules épithéliales de petites voies aériennes de primate immortalisées en soumettant des cellules épithéliales de petites voies aériennes de primate normales aux traitements (1) à (3) suivants :

(1) expression forcée d'un gène de transcriptase inverse télomérase,
(2) expression forcée d'un gène de kinase cycline-dépendante 4,
(3) induction de la perte de fonction de p53,

- et la combinaison de gènes associés au cancer est l'une des suivantes :

a) gène c-Myc et gène v-Src,
b) gène c-Myc, gène KRAS muté et gène BCL2
c) gène KRAS muté et gène de cycline D1
d) gène KRAS muté, gène de cycline D1 et gène TP63
e) gène KRAS muté et gène PIK3CA muté
f) gène KRAS muté et gène LKB1 muté
g) gène EGFR muté et gène de cycline D1.

**2.** Procédé conforme à la revendication 1, dans lequel les cellules épithéliales de petites voies aériennes de primate immortalisées sont des cellules épithéliales de petites voies aériennes humaines.

**3.** Procédé conforme à la revendication 1 ou 2, dans lequel la combinaison de gènes associés au cancer est celle du gène c-Myc et du gène v-Src ou celle du gène c-Myc, du gène KRAS muté et du gène BCL2.

**4.** Procédé conforme à la revendication 3, dans lequel les cellules tumorales sont des cellules de cancer indifférencié.

**5.** Procédé conforme à la revendication 4, dans lequel les cellules tumorales sont des cellules souches cancéreuses et la combinaison de gènes associés au cancer est celle du gène c-Myc et du gène v-Src.

**6.** Procédé conforme à la revendication 2, dans lequel la combinaison de gènes associés au cancer est celle du gène c-Myc et du gène v-Src, et le gène c-Myc est exprimé en mode inductif dans les cellules épithéliales de petites voies aériennes humaines immortalisées.

**7.** Procédé conforme à la revendication 6, dans lequel les cellules tumorales sont des cellules de cancer peu différencié du poumon.

**8.** Procédé conforme à la revendication 1 ou 2, dans lequel la combinaison de gènes associés au cancer est celle du gène KRAS muté et du gène de cycline D1.

**9.** Procédé conforme à la revendication 1 ou 2, dans lequel la combinaison de gènes associés au cancer est celle du gène KRAS muté, du gène de cycline D1 et du gène TP63, celle du gène KRAS muté et du gène PIK3CA muté, celle du gène KRAS muté et du gène LKB1 muté, ou celle du gène EGFR muté et du gène de cycline D1.

**10.** Procédé conforme à la revendication 8 ou 9, dans lequel les cellules tumorales sont des cellules de cancer différencié du poumon.

**11.** Procédé conforme à la revendication 10, dans lequel les cellules tumorales sont des cellules souches cancéreuses de cancers différenciés du poumon.

**12.** Procédé conforme à la revendication 10, dans lequel la combinaison de gènes associés au cancer est

a) celle du gène KRAS muté et du gène de cycline D1,
b) celle du gène EGFR muté et du gène de cycline D1,
c) ou celle du gène KRAS muté et du gène PIK3CA muté.

# Figure 1

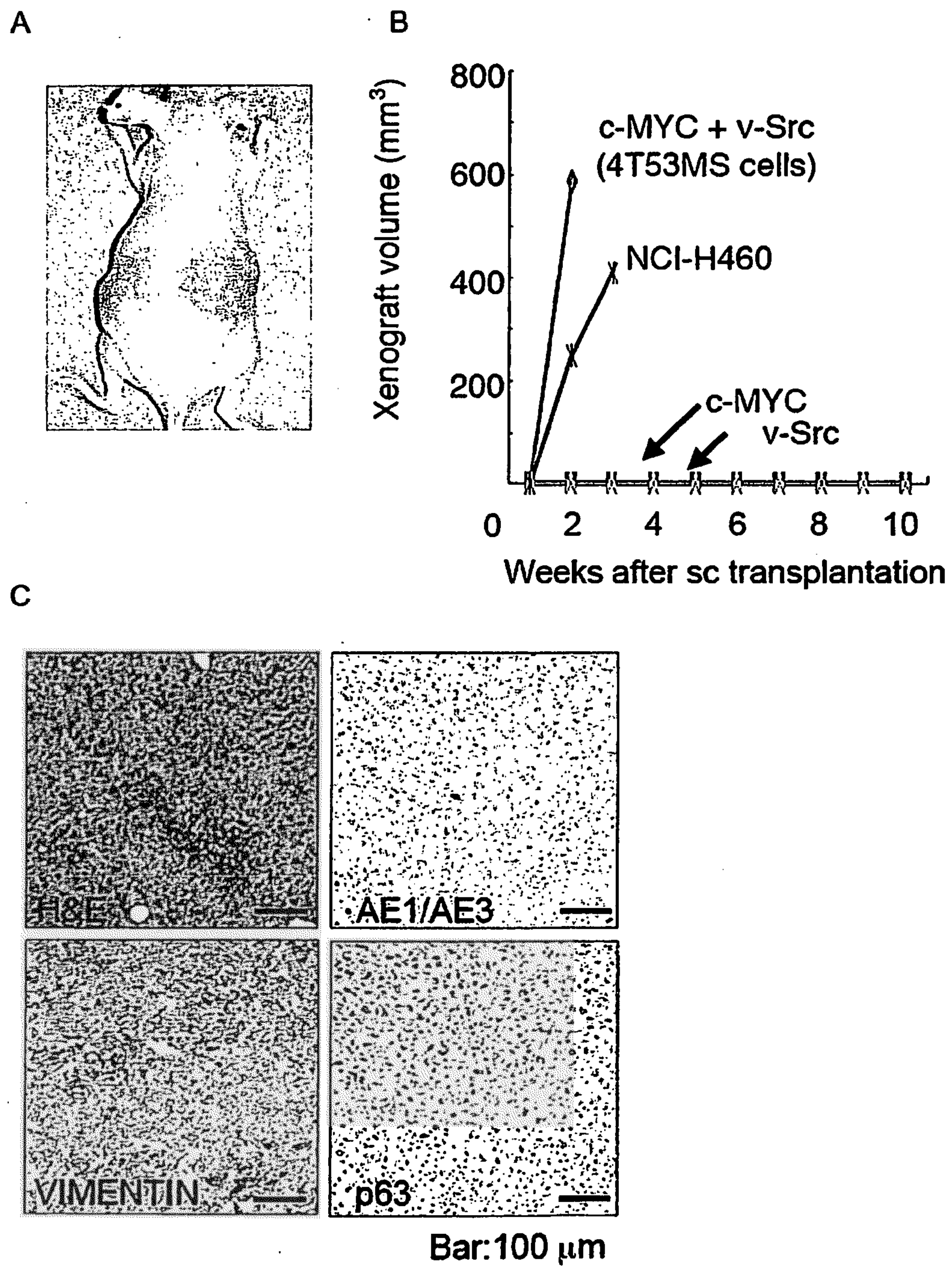
A
B
800
600
400
200
0
2
4
6
8
10
Xenograft volume (mm3)
Weeks after sc transplantation
c-MYC + v-Src
(4T53MS cells)
NCI-H460
c-MYC
v-Src
C
H&E
AE1/AE3
VIMENTIN
p63
Bar:100 µm

**Figure 2**

A

| # of cells injected | $10^6$ | $10^4$ | $10^3$ | $10^2$ | $10^1$ |
|---|---|---|---|---|---|
| Incidence (Latency*) | 10/10 (<2wks) | 4/4 (< 3wks) | 2/2 (<4wks) | 2/2 (5-6 wks) | 2/4 (~7wks) |

* Median time (weeks) for sc-xenograft to reach 1000 $mm^3$

B

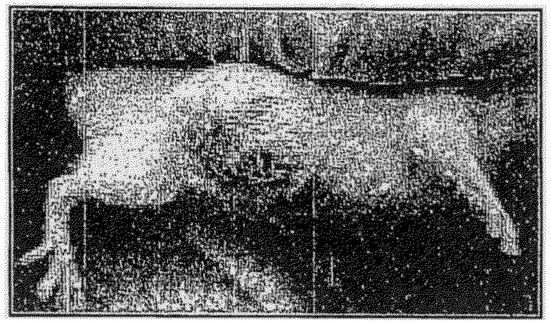

42 days after sc-injection (10 cells)

# Figure 3

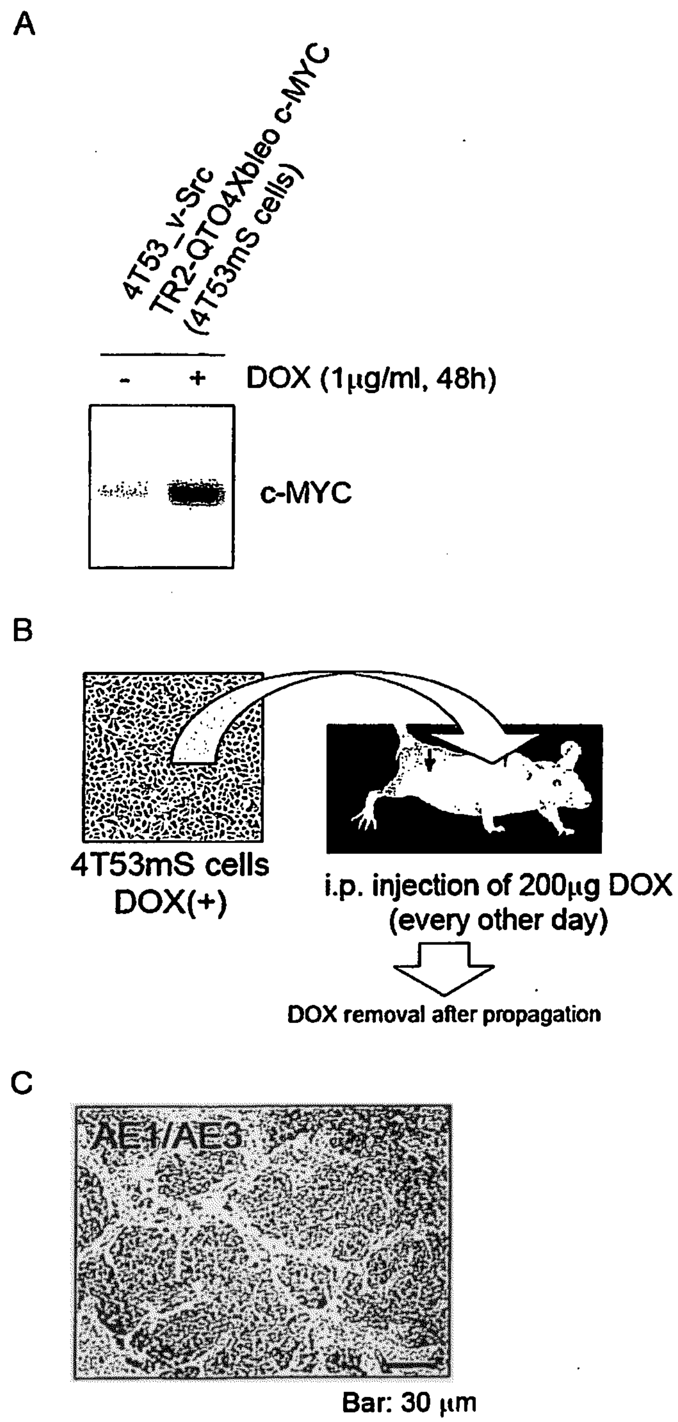
A
4T53_v-Src
TR2-QTO4Xbleo c-MYC
(4T53mS cells)
- + DOX (1μg/ml, 48h)
c-MYC
B
4T53mS cells
DOX(+)
i.p. injection of 200μg DOX
(every other day)
DOX removal after propagation
C
AE1/AE3
Bar: 30 μm

**Figure 4**

| *Cell types* | *Incidence* |
|---|---|
| 4T53 | 0/2 |
| 4T53 M | 0/4 |
| 4T53 R | 0/6 |
| 4T53 RM | 3/8 |
| 4T53 RMB | 8/10 |
| 4T53 RP | 6/6 |
| 4T53 RD | 6/6 |
| 4T53 RL | 5/6 |
| 4T53 ED | 4/4 |

**Figure 5**

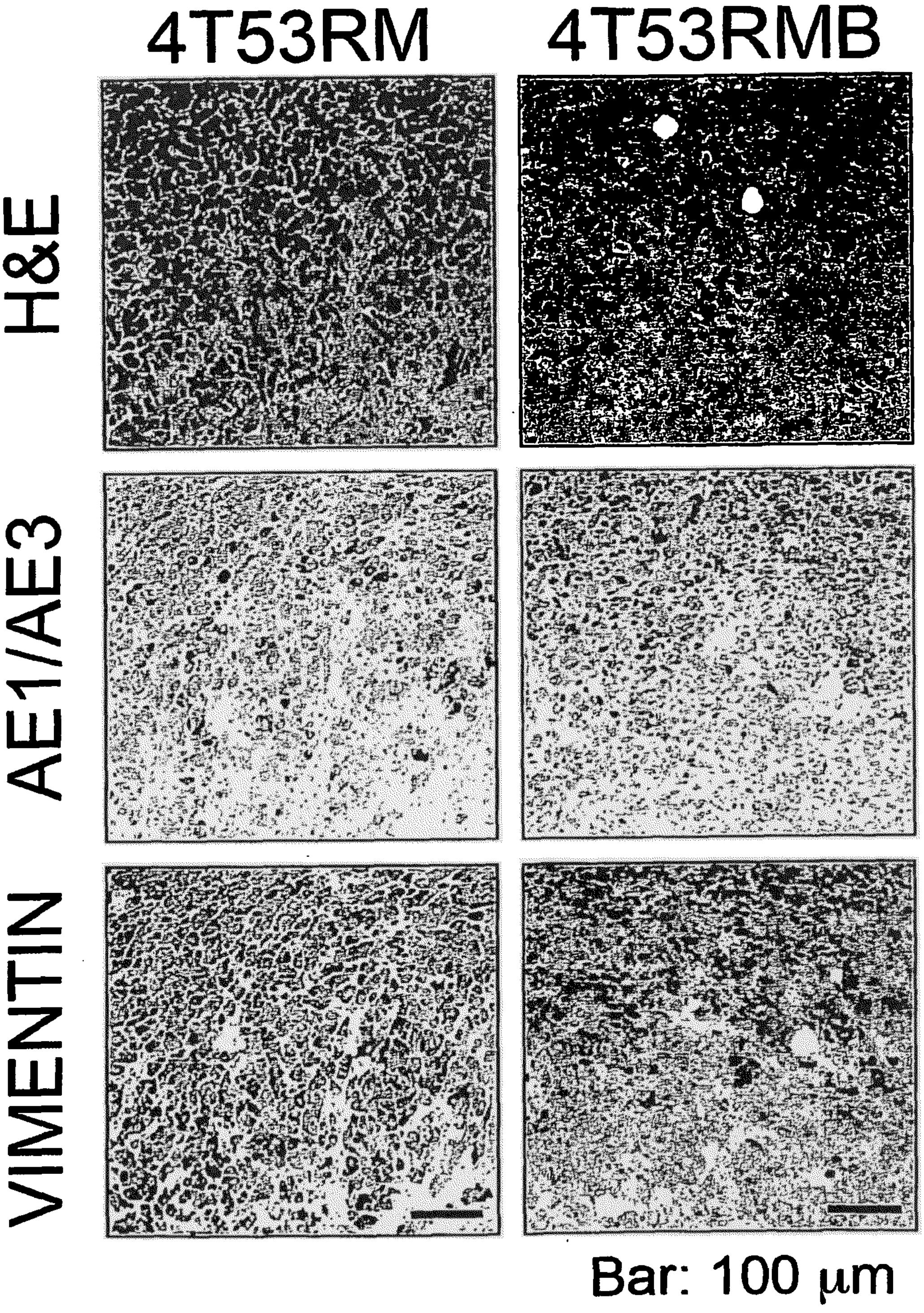

**Figure 6**

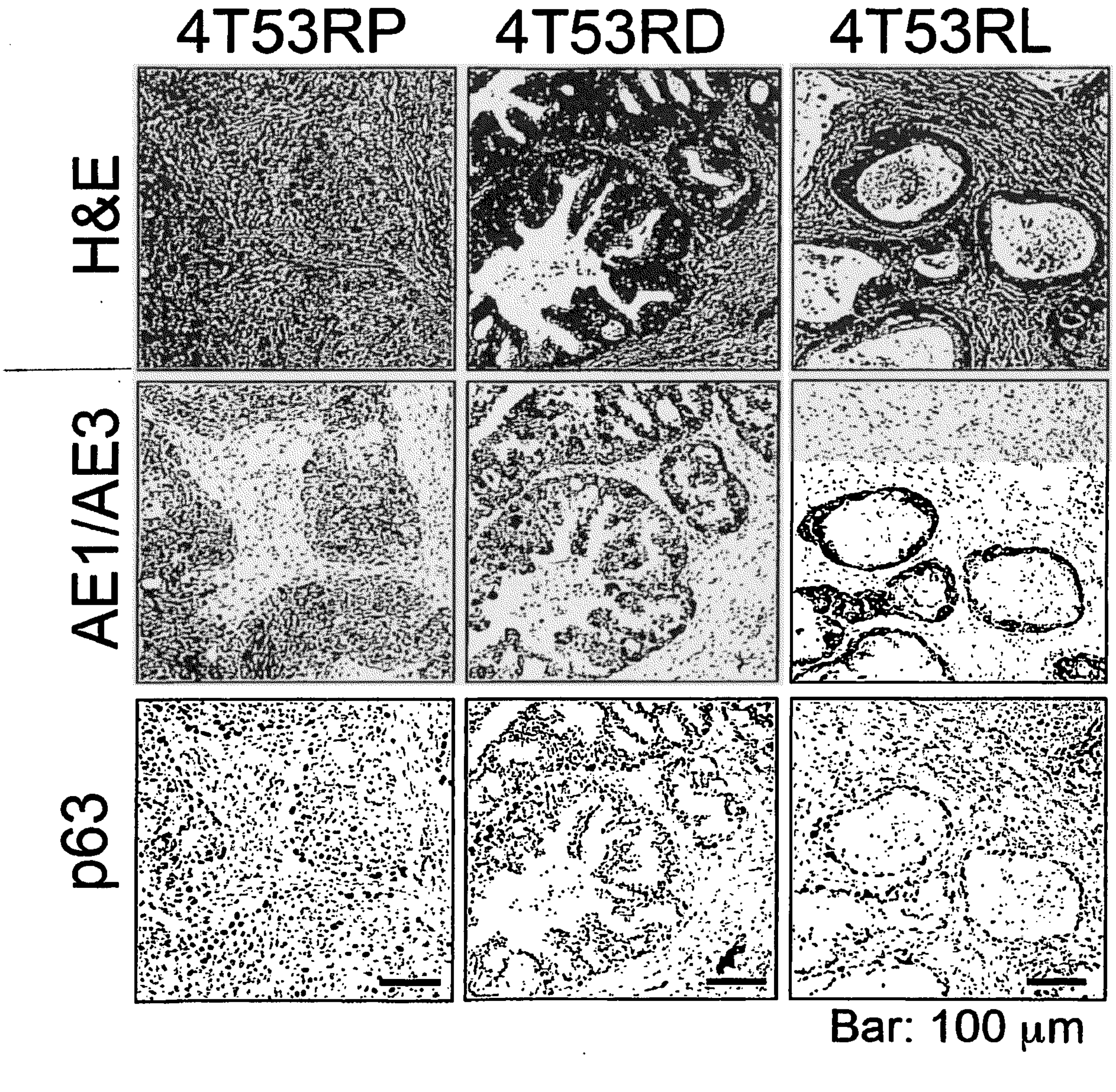

Figure 7

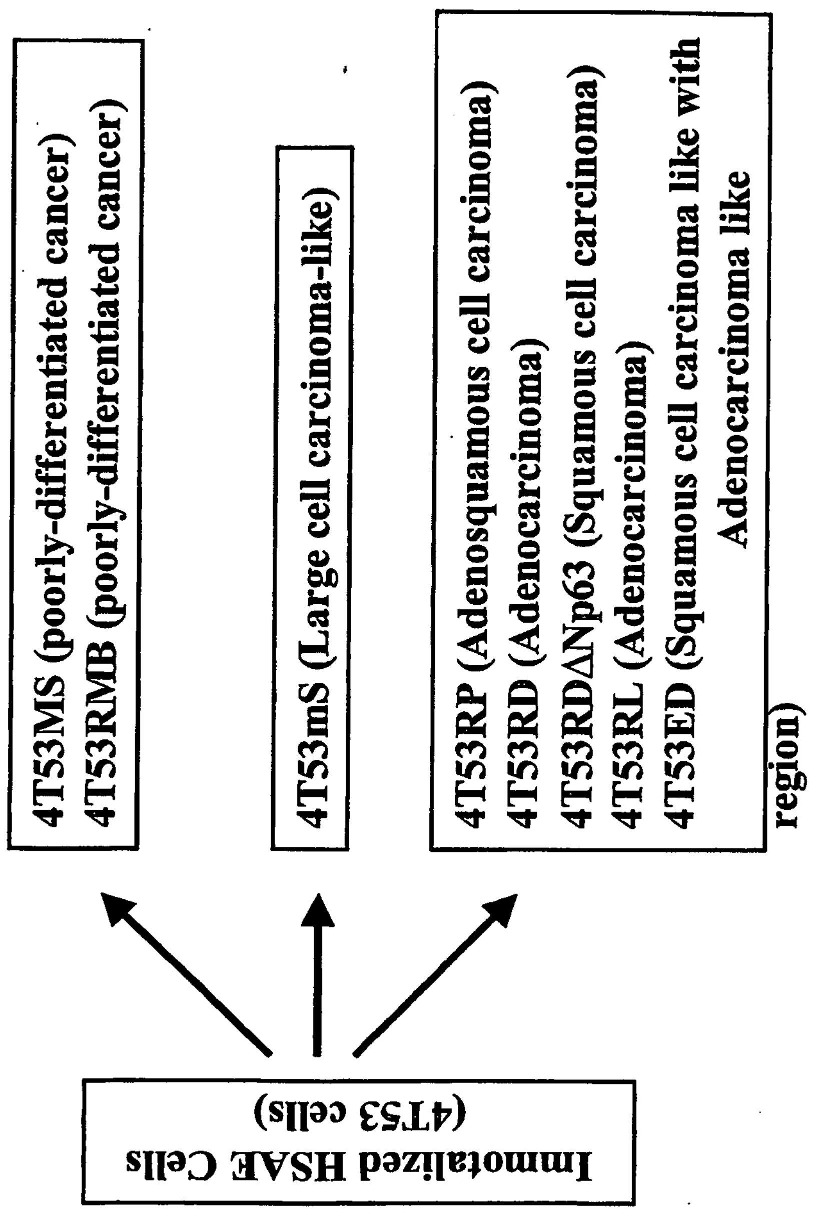
Immortalized HSAE Cells
(4T53 cells)
4T53MS (poorly-differentiated cancer)
4T53RMB (poorly-differentiated cancer)
4T53mS (Large cell carcinoma-like)
4T53RP (Adenosquamous cell carcinoma)
4T53RD (Adenocarcinoma)
4T53RDΔNp63 (Squamous cell carcinoma)
4T53RL (Adenocarcinoma)
4T53ED (Squamous cell carcinoma like with Adenocarcinoma like region)

# Figure 8

(A)

**4T53RD clone**

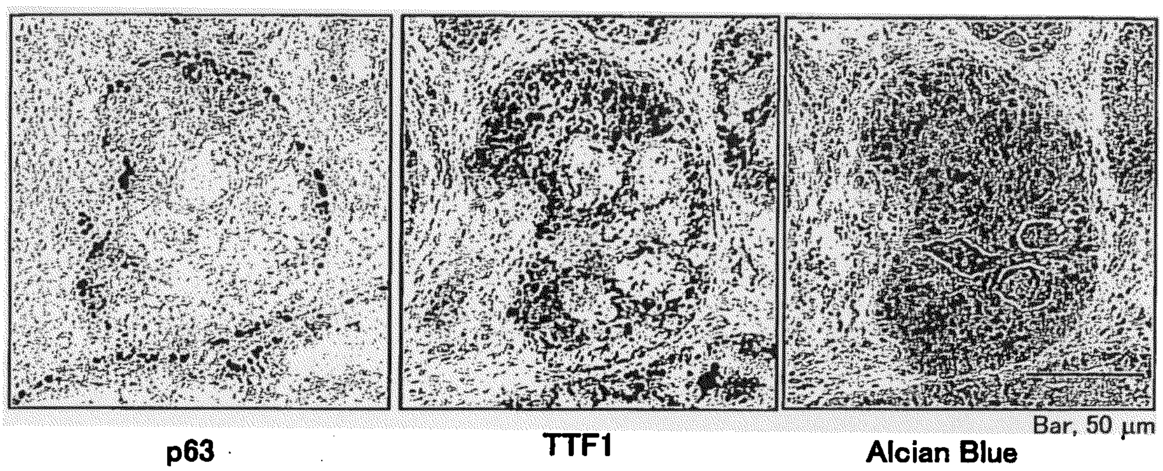


p63 TTF1 Alcian Blue

(B)

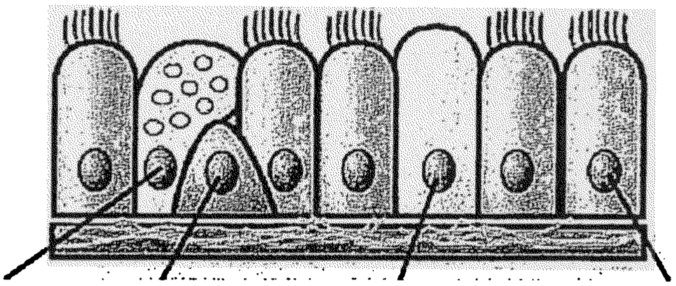


Scheme for adult mouse trachea

Maeda et al, Physiol Rev 2007;87:219-44

# Figure 9

| | | | | |
|---|---|---|---|---|
| Number of Cells Subcutaneously Injected to NOD-SCID | $10^4$ | $10^3$ | $10^2$ | $10^1$ |
| Rate of Tumorigenesis | 2/2 | 4/5 | 3/5 | 1/8 |

**Figure 10**

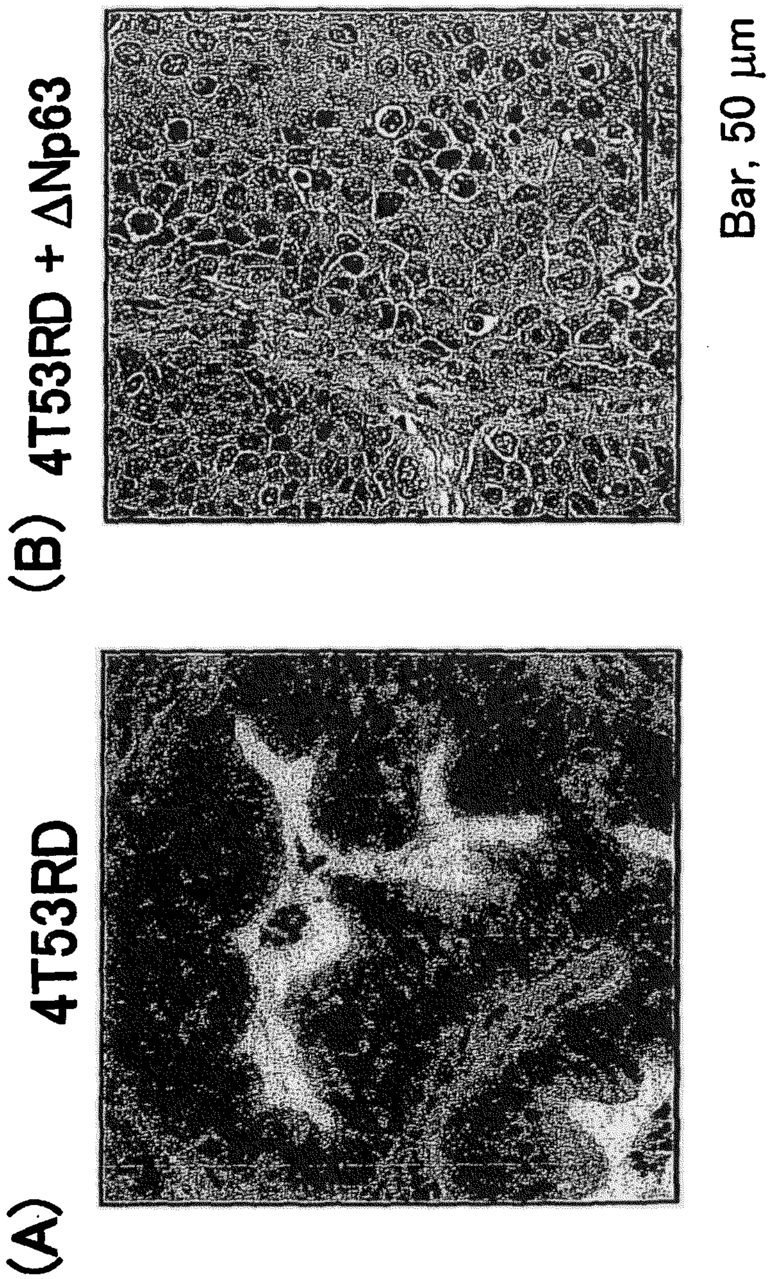

Figure 11

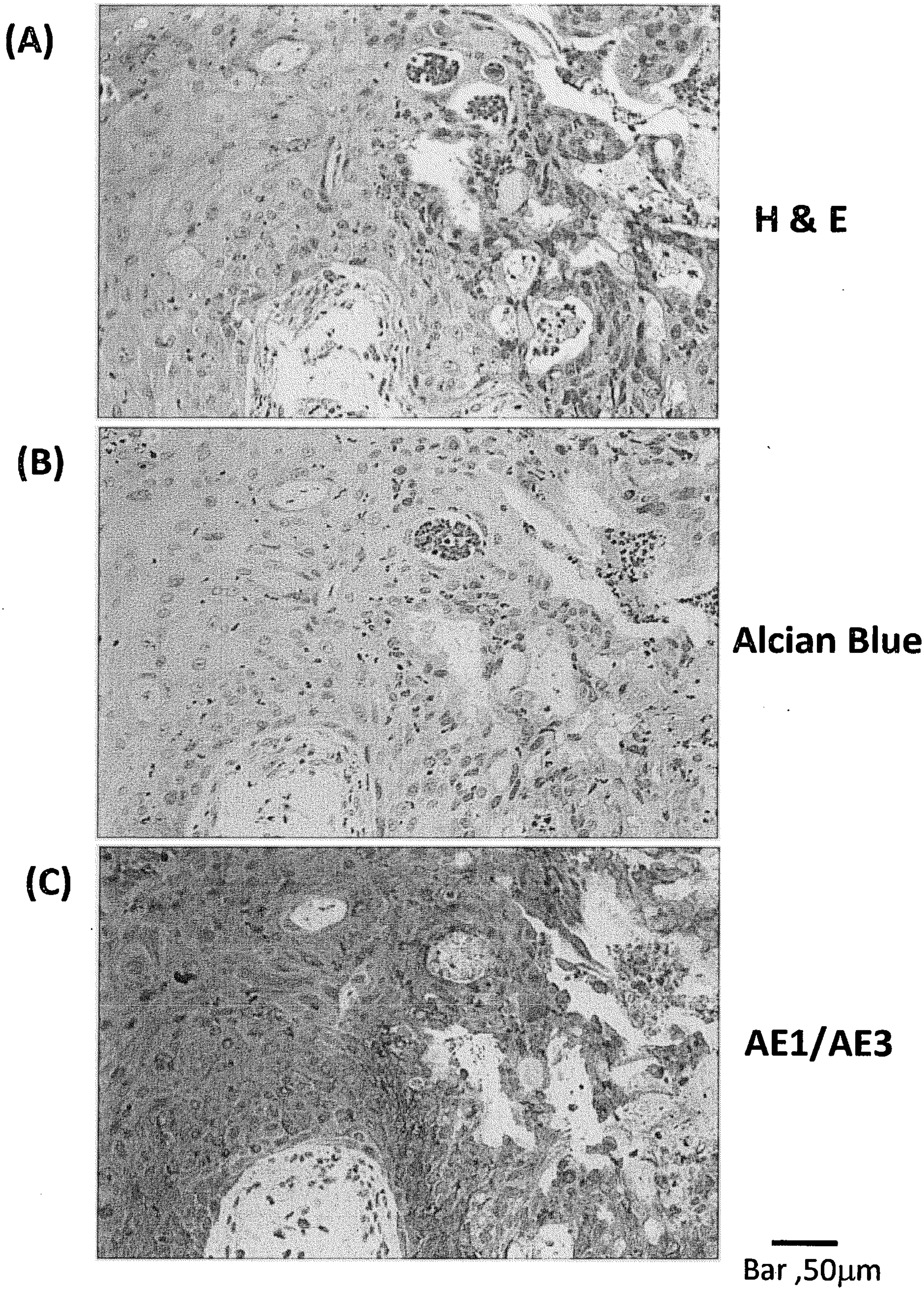

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010125256 A


### Non-patent literature cited in the description


- **MEUWISSEN, R. et al.** *Genes Dev.,* 2005, vol. 19, 643-664
- **HERBST, R.S. et al.** Lung cancer. *N. Engl. J. Med.,* 2008, vol. 359, 1367-1380
- **AKAGI, T.** *Trends Mol. Med.,* 2004, vol. 10, 542-548
- **ZHAO, J.J. et al.** *Trends Mol. Med.,* 2004, vol. 10, 344-350
- **LUNDBERG, A.S. et al.** *Oncogen,* 2002, vol. 21, 4577-4586
- **RAMIREZ, R.D. et al.** *Cancer Res.,* 2004, vol. 64, 9027-9034
- **SATO, M. et al.** *Cancer Res.,* 2006, vol. 66, 2116-2128
- **SEKIDO, Y. et al.** *Annu. Rev. Med.,* 2003, vol. 54, 73-87
- **DOMINGUEZ-SOLA, D. et al.** *Nature,* 2007, vol. 448, 445-451
- **MAYER B.J. et al.** *J. Virol.,* 1986, vol. 60 (3), 858-67
- **GOODSELL, D.S.** *Oncologist,* 1999, vol. 4 (3), 263-264
- **AVIEL-RONENV, S. et al.** *Clin. Lung Cancer,* 2006, vol. 1, 30-38
- **SATO, M. et al.** *Cancer Res.,* 2006, vol. 66 (4), 2116-2128
- **CHAO, D.T ; KORSMEYER, S.J.** *Annu. Rev. Immunol.,* 1998, vol. 16, 395-419
- *Nature Reviews Cancer,* 2005, vol. 5, 921-929
- *Sci. Transl. Med.,* 2010, vol. 2, 26-25
- **MA, Y.Y. et al.** *Oncogene,* 2000, vol. 19 (23), 2739-2744
- **MORGAN, D.O.** *Cell,* 2008, vol. 135, 764-794
- *Lung Cancer,* 2007, vol. 55, 1-14
- **KATAJISTO, P. et al.** *Biochem. Biophys. Acta,* 2007, vol. 1775 (1), 63-75
- **SHARMA, S.V et al.** *Nat. Rev. Cancer,* 2007, vol. 7 (3), 169-81
- **DEYOUNG, M.P. et al.** *Oncogene,* 2007, vol. 26, 5169-5183
- **MCCOY, M.S. et al.** *Mol. Cell. Biol.,* 1984, vol. 4, 1577-1582
- **SAMUELS, Y. et al.** *Science,* 2004, vol. 304, 554
- **SCOTT, K.D. et al.** *Cancer Res.,* 2007, vol. 67, 5622-5627
- **ZUO, L. et al.** *Nature Genet,* 1996, vol. 12, 97-99
- **SHAULIAN, E. et al.** *Mol. Cell. Biol,* 1992, vol. 12, 5581
- **SAMBROOK ; RUSSELL.** Molecular Cloning: A Laboratory Manual. Cold Spring Harbor, Laboratory Press, 2001, vol. 3
- **TANAKA, S. et al.** *Proc. Natl. Acad. Sci. USA,* 1997, vol. 94, 2356-2361
- **NISHIMURA, K. et al.** *Nature Methods,* December 2009, vol. 6 (12
- Genetic induction oftumorigenesis in swine. *Oncogen,* 11 September 2006, vol. 26, 1038-1045
- **SASAI, K. et al.** *Am. J Surg. Pathol.,* 2008, vol. 32, 1220-1227
- **STEEDMAN, H.F.** *Quarterly Journal of Microscopic Science,* 1950, vol. 91, 477-479
- **AKAGI, T. et al.** *Mol. Cell Biol.,* 2002, vol. 22, 7015-7023
- **QUINTANA, E. et al.** *Nature,* 04 December 2008, vol. 456 (7222), 593-8
- **ROCK, J.R. et al.** *Proc. Natl. Acad. Sci. USA,* 04 August 2009, vol. 106 (31), 12771-5